(19) Europäisches Patentamt
European Patent Office
Office européen des brevets

(11) EP 3 971 190 A1

(12) **EUROPEAN PATENT APPLICATION**
published in accordance with Art. 153(4) EPC

(43) Date of publication:
**23.03.2022 Bulletin 2022/12**

(21) Application number: **20810762.3**

(22) Date of filing: **18.05.2020**

(51) International Patent Classification (IPC):
***C07D 495/04*** *(2006.01)* ***C07D 487/04*** *(2006.01)*
***A61K 31/519*** *(2006.01)* ***A61P 35/00*** *(2006.01)*
***A61P 35/02*** *(2006.01)*

(52) Cooperative Patent Classification (CPC):
**A23L 29/00; A23L 33/10; A61K 31/519; A61P 35/00; A61P 35/02; C07D 487/04; C07D 495/04**

(86) International application number:
**PCT/KR2020/006496**

(87) International publication number:
**WO 2020/235902 (26.11.2020 Gazette 2020/48)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **17.05.2019 KR 20190057712**

(71) Applicants:
- **Voronoi Inc.**
  **Incheon 21984 (KR)**
- **Voronoibio Inc.**
  **Incheon 21984 (KR)**

(72) Inventors:
- **JO, Seo Hyun**
  **Seoul 03376 (KR)**
- **LI, Hua**
  **Incheon 21569 (KR)**
- **DO, Woo Mi**
  **Incheon 21982 (KR)**
- **RYU, Hee Sun**
  **Incheon 22231 (KR)**
- **KIM, Hwan**
  **Seoul 03958 (KR)**
- **SEOK, Ji Yoon**
  **Incheon 21982 (KR)**
- **LEE, Sun Hwa**
  **Incheon 21982 (KR)**
- **SON, Jung Beom**
  **Incheon 21986 (KR)**
- **KIM, Nam Doo**
  **Incheon 22008 (KR)**

(74) Representative: **Plasseraud IP**
**66, rue de la Chaussée d'Antin**
**75440 Paris Cedex 09 (FR)**

(54) **HETEROCYCLE-FUSED PYRIMIDINE DERIVATIVE AND USE THEREOF**

(57) The present invention relates to a heterocycle-fused pyrimidine derivative compound or a pharmaceutically acceptable salt thereof, a pharmaceutical composition for preventing or treating diseases related to one or more protein kinases of FLT3, CDK4, CDK6 and HPK1, comprising the same as an active ingredient, and a method for preventing or treating diseases related to one or more protein kinases of FLT3, CDK4, CDK6 and using the same.

EP 3 971 190 A1

## Description

### Technical Field

The present invention relates to a heterocycle-fused pyrimidine derivative compound or a pharmaceutically acceptable salt thereof, a pharmaceutical composition for preventing or treating FLT3, CDK4, CDK6 and HPK1-related diseases comprising, as an active ingredient, the same, and a method for preventing or treating FLT3, CDK4, CDK6 and HPK1-related diseases using the pharmaceutical composition.

### Background Art

Protein kinases are receptor-type or non-receptor-type proteins, which transfer the terminal phosphate of ATP to amino acid residues such as tyrosine, threonine and serine residues in the proteins, thereby activating or inactivating a signal transduction pathway. When these proteins are ruptured, they are known to be involved in many cytoplasmic mechanisms leading to diseases such as inflammation, as well as abnormal cell proliferation and migration.

Receptor tyrosine kinases (RTKs) are cell surface receptors having high affinity with many polypeptide growth factors, cytokines and hormones. These are not only major regulators of normal cellular processes, but also play an important role in the development and progression of various types of cancer diseases. Mutations in these receptor tyrosine kinases lead to activation of a series of signaling cascades that have many effects on protein expression.

Therefore, these receptor tyrosine kinases are targeted for drug therapy due to their potential against various cellular abnormalities such as cancer, degenerative diseases and cardiovascular diseases. Several anticancer drugs induced by the activated RTK have been approved by the United States Food and Drug Administration (FDA). These drugs have been developed so as to inhibit ligand binding and receptor oligomerization by targeting extracellular domains or catalytic domains.

The role of FLT3 among these receptor tyrosine kinases is of particular interest. In fact, 60 to 80% of acute myeloid leukemia (AML) fibrosis express the receptor FLT3, which is a receptor for the FLT3 ligand, and express it in a high percentage of acute lymphocytic leukemia (ALL). Both ligands and receptors are identified in U119 either directly or in concert (Hannum et al., Nature 368, pp. 643-648, 1994; Rosnet et al., Genomics 9, pp. 380-385, 1991). The FLT3 mediates differentiation and proliferation of normal hematopoietic stem cells and mediates proliferation and survival signals in AML fibrosis. Although the FLT3 is most commonly expressed in its wild-type form, 30 to 35% of leukemia clones (Nakao et al., Leukemia 12, pp. 1911-1918, 1996) in patients with AML express a mutated form (FLT3 ITD) of FLT3 including internal tandem duplication (ITD) of parallel membrane domain coding sequences. Such a mutation leads to spontaneous cytokine-independent growth and constitutive activation of the receptor. A group of AML patients (~7%) also contains a mutation in the activation loop (FLT3D835) of FLT3 near an amino acid position Asp835. Also, FLT3 mutations appear with an expression frequency of 15% in secondary AML, which may be associated with disease progression or recurrence of AML. Furthermore, patients with FLT3 mutations tend to have a poor diagnosis with reduced remission times and disease-free survival. Thus, inhibitors of congenital and/or variant FLT3 kinases may be attractive targets for the treatment of diseases of hematopoietic cells and adverse effects of hematology.

The regulation of the cell cycle is mainly influenced by a series of serine/threonine kinases, also called cyclin dependent kinases (CDKs), where they bind to subunit cyclins corresponding to each other to achieve the purpose of regulating the cell cycle. So far, at least 10 cyclin-dependent kinases (CDKs) and 15 cyclins have been identified. Cyclin-CDK complexes may be formed by pairing CDK1 with cyclin A or B; pairing CDK2 with cyclin A or E; pairing CDK3 with a sort of unknown cyclins; pairing CDK4 with cyclin D(1-3); pairing CDK5 with cyclin D or p35Nck5A; pairing CDK6 with cyclin D; pairing CDK7 with cyclin H; pairing CDK8 with cyclin C; and pairing CDK9 with cyclin T.

Abnormal proliferation of cancer cells and normal cell cycle disorders are common features of all types of cancer. On this account, inhibitors of key regulating factors of the cell cycle have already become attractive new anti-tumor targets. At the beginning of the G1 phase of the cell cycle, the complex formed with CDK4/6 and cyclin D is activated by an extracellular growth factor, and the complex thus activated can phosphorylate retinoblastoma protein (RB). Therefore, while the tightly bound transcription factor E2F is released in the unphosphorylated state and the E2F is activated to further promote transcription, the cell cycle crosses the R point and proceeds from the G1 phase to the S phase. The complex formed by the CDK4/6 and cyclin D is a key "master switch" in cell cycle regulation, and inhibits CDK4/6 to block the formation of the Cyclin D-CDK4/6 complex, thereby preventing the progression of the cell cycle from the G1 phase to the S phase to be capable of reaching the goal of inhibiting tumor proliferation, so that the CDK4/6 has been an important anti-cancer target. Currently, many studies have been conducted on CDK4/6 inhibitors for breast cancer, and Palbociclib has been used as a drug for treating breast cancer.

Hematopoietic progenitor kinase 1 (HPK1) is an enzyme expressed in a hematopoietic stem cell line, which is known as one of intracellular negative regulators in T cells, B cells and dendritic cells. It is known that the HPK1 phosphorylates SLP76 (Di Bartolo et al. (2007) JEM 204:681-691]) at Ser376 and Gads at Thr254 in T cells, thereby causing

mobilization of a 14-3-3 protein that binds to the phosphorylated SLP76 and Gads, and releases complexation of SLP76-Gads-14-3-3 from LAT-containing micro-clusters, thereby reducing the persistence of signaling micro-clusters to negatively regulate T cell activation (Lasserre et al. (2011) J Cell Biol 195(5):839-853]). It is known that the HPK1 plays a role in regulating the functions of various immune cells, and accordingly, is involved in autoimmune diseases and antitumor immunity (Shui, JW, et al., Nat Immunol, 2007. 8(1): p. 84-91; Wang, X., et al., J Biol Chem, 2012. 287(14): p. 11037-48).

## Disclosure

### Technical Problem

The present inventors have intensively studied to develop novel small molecule compounds capable of inhibiting or regulating FLT3, CDK4, CDK6, and HPK1 activity, and as a result, they have confirmed that a series of heterocycle-fused pyrimidine derivatives can inhibit the activity of FLT3, CDK4, CDK6 and HPK1, and can inhibit the proliferation of cells expressing FLT3, CDK4, CDK6, and HPK1 mutant genes, such as acute myeloid leukemia cells and breast cancer cells, and completed the present invention.

### Technical Solution

As one aspect for achieving the above object, the present invention provides a compound represented by the following formula 1, an isomer thereof, a solvate thereof, a hydrate thereof, or a pharmaceutically acceptable salt thereof.

[Formula 1]

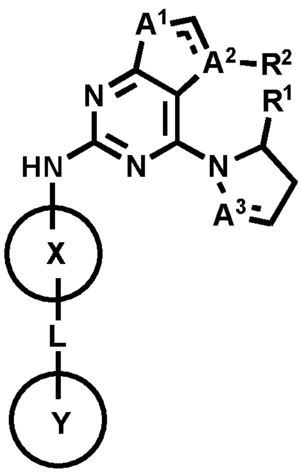

In Formula 1 above,

$A^1$ may be CH or NH;

$A^2$ may be CH or S;

$A^3$ may be N or O;

 is a single bond or a double bond, which is determined according to the valence of atoms located at both ends thereof;

$R^1$ is phenyl, thiophenyl, pyridinyl, $(C_{3-7})$cycloalkyl, naphthalenyl or isoquinolinyl, wherein the phenyl may be substituted with $(C_{1-3})$alkoxy or halogen;

$R^2$ may be absent, hydrogen, haloalkyl, cyano, or halogen;

ring X is benzene, pyridine, pyrazole, isoindoline, tetrahydroisoquinoline, dihydroisoquinoline, or isobenzofuran,

wherein the benzene, pyridine, pyrazole, isoindoline, tetrahydroisoquinoline, dihydroisoquinoline, or isobenzofuran may be substituted with one or more non-hydrogen substituents selected from the group consisting of $(C_{1-5})$alkyl, $(C_{1-5})$alkoxy, (di$(C_{1-3})$alkylamino$(C_{1-5})$alkyl)$(C_{1-3})$alkylamino, di$(C_{1-3})$alkylamino$(C_{1-5})$alkyl, (di$(C_{1-3})$alkylamino)$(C_{1-5})$alkoxy, di$(C_{1-3})$alkylaminocarbonyl$(C_{1-3})$alkyl, $(C_{3-7})$cycloalkyl, oxo('=O'), $(C_{1-3})$alkylsulfonyl, haloalkyl, $(C_{1-3})$alkylcarbonyl, hydroxy$(C_{1-5})$alkyl, and halogen;

L and ring Y are absent, or

when L and ring Y are present, L is a direct bond, -$(C_{1-5})$alkylene-, -O-, -(C=O)-, -O-$(C_{1-5})$alkylene-, -(C=O)-NH-, or piperidine, wherein ring Y is a heterocyclic group selected from the group consisting of piperazine, pyrrolidine, morpholino, piperidine, imidazole, diazabicyclo[2,2,1]heptane, and pyrazole, where the heterocyclic group may be substituted with one or more non-hydrogen substituents selected from the group consisting of $(C_{1-5})$alkyl, di$(C_{1-3})$alkylamino, hydroxy$(C_{0-5})$alkyl, and $(C_{3-7})$cycloalkyl;

the case where both of L and ring Y are piperidine may be excluded; and

the case where both of $A^1$ and $A^2$ are CH or are not CH may be excluded.

Another aspect of the present invention provides a pharmaceutical composition for preventing or treating diseases related to one or more protein kinases of FLT3, CDK4, CDK6, and HPK1 comprising, as an active ingredient, the compound, a stereoisomer thereof, a hydrate thereof or a pharmaceutically acceptable salt thereof.

Another aspect of the present invention provides a pharmaceutical composition for preventing or treating: hematologic malignancies including acute myeloid leukemia, myelodysplastic syndrome, acute lymphocytic leukemia, and chronic myeloid leukemia; proliferative diseases including cancer diseases; autoimmune diseases including multiple sclerosis, psoriasis, inflammatory small intestine disease, ulcerative colitis, Crohn's disease, rheumatoid arthritis and polyarthritis, local and systemic scleroderma, systemic lupus erythematosus, discoid lupus erythematosus, cutaneous lupus, dermatomyositis, polymyositis, Sjogren's syndrome, nodular panarteritis, autoimmune enteritis, atopic dermatitis and proliferative glomerulonephritis; allergic diseases including asthma, allergic rhinitis, allergic rhinosinusitis, anaphylaxis, urticaria, angioedema, atopic dermatitis, allergic contact dermatitis, erythema nodosum, erythema multiforme, cortical phlebitis and insect bite dermatitis or blood-sucking parasite infection; or neurological diseases including Huntington's disease, schizophrenia, Parkinson's disease, and Alzheimer's disease, comprising, as an active ingredient, the compound, a stereoisomer thereof, a hydrate thereof or a pharmaceutically acceptable salt thereof.

Another aspect of the present invention provides a method for treating the above diseases, comprising administering the compound, a stereoisomer thereof, a hydrate thereof or a pharmaceutically acceptable salt thereof to an individual or subject in need thereof.

Another aspect of the present invention provides the compound, a stereoisomer thereof, a hydrate thereof or a pharmaceutically acceptable salt thereof for use in the prevention or treatment of the above diseases.

Another aspect of the present invention provides a use of the compound, a stereoisomer thereof, a hydrate thereof or a pharmaceutically acceptable salt thereof for use in the preparation of a medicament for the prevention or treatment of the above diseases.

**Advantageous Effects**

The heterocycle-fused pyrimidine derivative of the present invention can inhibit the activity of FLT3, CDK4, CDK6 and HPK1, and can inhibit the proliferation of cells overexpressing mutants thereof, so that it can be usefully used for the treatment or prevention of diseases caused by abnormal expression or excessive activity of FLT3, CDK4, CDK6 and HPK1.

**Best Mode**

Hereinafter, the present invention will be described in detail.

Meanwhile, embodiments of the present invention may be modified in various other forms, and the scope of the present invention is not limited to the embodiments described below. In addition, the embodiments of the present invention are provided in order to more completely explain the present invention to those having average knowledge in the relevant technical field. Furthermore, the matter that "comprises" a certain element throughout the specification means that other components may be further included, rather than excluding other components, unless there is a particular contrary description.

In this specification, the "halogen" may be F, Cl, Br, or I.

In this specification, the "haloalkyl" may mean linear or branched alkyl (hydrocarbon) having a carbon atom substituted with one or more halogen atoms as defined herein. An example of the haloalkyl includes methyl, ethyl, propyl, isopropyl, isobutyl and N-butyl independently substituted with one or more halogen atoms, for example, F, Cl, Br, or I, but is not limited thereto.

In this specification, the "alkyl" may mean linear or branched acyclic saturated hydrocarbon consisting of carbon atoms. The representative -($C_{1\sim8}$ alkyl) may include - methyl, -ethyl, -N-propyl, -N-butyl, -N-pentyl, -N-hexyl, -N-heptyl and -N-octyl; and the branched saturated alkyl may include -isopropyl, -secondary (sec)-butyl, -isobutyl, -tertiary (tert)-butyl, -isopentyl, 2-methylpentyl, 3-methylpentyl, 4-methylpentyl, 2,3-dimethylbutyl, and the like. The -($C_{1\sim8}$ alkyl) may also be substituted or unsubstituted. For example, a $C_{1\sim8}$ alkyl group may be substituted with phenyl to form a benzyl group.

In this specification, the "cycloalkyl" may mean a non-aromatic saturated or unsaturated carbon ring. The representative cycloalkyl includes cyclopropyl, cyclobutyl, cyclopentyl, cyclopentadienyl, cyclohexyl, cyclohexenyl, 1,3-cyclohexadienyl, 1,4-cyclohexadienyl, cycloheptyl, 1,3-cycloheptadienyl, 1,3,5-cycloheptatrienyl, cyclooctyl and cyclooctadienyl, but is not limited thereto. The cycloalkyl group may also be substituted or unsubstituted. In one embodiment, this cycloalkyl group may be a $C_{3\sim8}$ cycloalkyl group. The $C_7$ or higher cycloalkyl group may have two or more ring structures, where a specific example thereof may be a bicycloalkyl group, and more specifically, bicycloheptane may be used in the present invention.

In this specification, the "aryl" may mean any functional group or substituent derived by removing one hydrogen from an aromatic hydrocarbon ring. The aryl group may be a monocyclic aryl group or a polycyclic aryl group. The number of ring-forming carbon atoms in the aryl group may be 5 or more and 30 or less, 5 or more and 20 or less, or 5 or more and 15 or less. An example of the aryl group may be exemplified by a phenyl group, a naphthyl group, a fluorenyl group, an anthracenyl group, a phenanthryl group, a biphenyl group, a terphenyl group, a quarterphenyl group, a quinquephenyl group, a sexphenyl group, a triphenylene group, a pyrenyl group, a benzofluoranthenyl group, a chrysenyl group, and the like, but is not limited thereto.

In this specification, the "heteroaryl" may be an aryl ring group containing one or more of O, N, P, Si, and S as the heterogeneous element. The number of ring-forming carbon atoms in the heteroaryl group may be 2 or more and 30 or less, or 2 or more and 20 or less. The heteroaryl may be monocyclic heteroaryl or polycyclic heteroaryl. The polycyclic heteroaryl may be, for example, one having a bicyclic or tricyclic structure. An example of heteroaryl includes thienyl, thiophene, furyl, pyrrolyl, pyrazolyl, imidazolyl, thiazolyl, oxazolyl, isothiazolyl, oxadiazolyl, triazolyl, pyridinyl, bipyridyl, triazinyl, triazolyl, an acridyl group, pyridazinyl, pyrazinyl, quinolinyl, quinazolinyl, quinoxalinyl, phenoxazil, phthalazinyl, pyrimidinyl, pyridopyrimidinyl, pyridopyrazinyl, pyrazinopyrazinyl, isoquinolinyl, indolyl, carbazolyl, imidazopyridazinyl, imidazopyridinyl, imidazopyrimidinyl, pyrazolopyrimidinyl, imidazopyrazinyl or pyrazolopyridinyl, N-arylcarbazolyl, N-heteroarylcarbazolyl, N-alkylcarbazolyl, benzooxazolyl, benzoimidazolyl, benzothiazolyl, benzocarbazolyl, benzothiophenyl, dibenzothiophenyl, thienothiophene, benzofuranyl, phenanthroline, isooxazolyl, oxadiazolyl, thiadiazolyl, benzothiazolyl, tetrazolyl, phenothiazinyl, dibenzosilol and dibenzofuranyl, and the like, but is not limited thereto. In one embodiment of the present invention, the heteroaryl may also include bicyclic heterocyclo-aryl including an aryl ring fused to a heterocycloalkyl ring or heteroaryl fused to a cycloalkyl ring.

One or more of the above-mentioned homogeneous or heterogeneous substituents may be substituted at the same position or at different positions, and may also be substituted sequentially. The term "sequentially" means that one substituent is substituted in the formula and then another substituent is successively substituted in the substituent, and for example, when an alkyl group is substituted, and then a cycloalkyl group is substituted in the alkyl group and a carbonyl group is sequentially substituted in the cycloalkyl group, it can be indicated that they have been sequentially substituted by naming it carbonylcycloalkylalkyl.

In addition, the above-listed linking radicals do not specify the bonding direction, and the bonding direction is arbitrary. For example, the radical L linked in

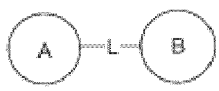

is -M-W-, where the -M-W- can link Ring A and Ring B in the same direction as in the reading order from left to right to form

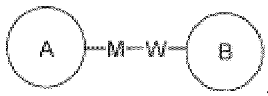,

and can link Ring A and Ring B in the opposite direction to the reading order from left to right to form

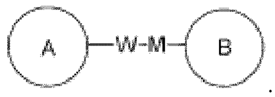

One aspect of the present invention provides a compound represented by the following formula 1, a stereoisomer thereof, a hydrate thereof, or a pharmaceutically acceptable salt thereof.

[Formula 1]

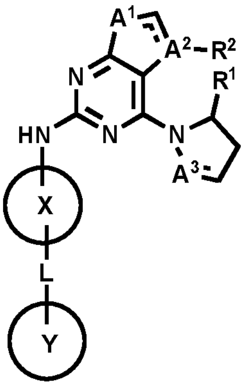

In Formula 1 above,

$A^1$ is CH or NH;

$A^2$ is CH or S;

$A^3$ is N or O;

------ is a single bond or a double bond, which is determined according to the valence of atoms located at both ends thereof;

$R^1$ is phenyl, thiophenyl, pyridinyl, $(C_{3-7})$cycloalkyl, naphthalenyl, or isoquinolinyl, wherein the phenyl may be substituted with $(C_{1-3})$alkoxy or halogen;

$R^2$ may be absent, hydrogen, haloalkyl, cyano, or halogen;

ring X is benzene, pyridine, pyrazole, isoindoline, tetrahydroisoquinoline, dihydroisoquinoline, or isobenzofuran, wherein the benzene, pyridine, pyrazole, isoindoline, tetrahydroisoquinoline, dihydroisoquinoline, or isobenzofuran may be substituted with one or more non-hydrogen substituents selected from the group consisting of $(C_{1-5})$alkyl, $(C_{1-5})$alkoxy, (di$(C_{1-3})$alkylamino$(C_{1-5})$alkyl)$(C_{1-3})$alkylamino, di$(C_{1-3})$alkylamino$(C_{1-5})$alkyl, (di$(C_{1-3})$alkylamino)$(C_{1-5})$alkoxy, di$(C_{1-3})$alkylaminocarbonyl$(C_{1-3})$alkyl, $(C_{3-7})$cycloalkyl, oxo('=O'), $(C_{1-3})$alkylsulfonyl, haloalkyl, $(C_{1-3})$alkylcarbonyl, hydroxy$(C_{1-5})$alkyl, and halogen;

L and ring Y are absent, or

when L and ring Y are present, L is a direct bond, -$(C_{1-5})$alkylene-, -O-, -(C=O)-, -O-$(C_{1-5})$alkylene-, -(C=O)-NH-, or piperidine, wherein ring Y is a heterocyclic group selected from the group consisting of piperazine, pyrrolidine, morpholino, piperidine, imidazole, diazabicyclo[2,2,1]heptane, and pyrazole, where the heterocyclic group may be substituted with one or more non-hydrogen substituents selected from the group consisting of $(C_{1-5})$alkyl, di$(C_{1-3})$alkylamino, hydroxy$(C_{0-5})$alkyl, and $(C_{3-7})$cycloalkyl; the case where both of L and ring Y are piperidine may be excluded; and

the case where both of $A^1$ and $A^2$ are CH or are not CH may be excluded.

In another aspect,

in the compound represented by Formula 1 above, when $A^1$ is CH, $A^2$ is S, and $A^3$ is N,

$R^1$ is phenyl, pyridinyl, $(C_{3-7})$cycloalkyl, naphthalenyl, or isoquinolinyl, wherein the phenyl may be substituted with halogen;

$R^2$ is absent;

ring X is benzene, pyridine, pyrazole, or isoindoline, wherein the benzene, pyridine, pyrazole, or isoindoline may be substituted with one or more non-hydrogen substituents selected from the group consisting of $(C_{1-5})$alkyl, $(C_{1-5})$alkoxy, $(di(C_{1-3})$alkylamino$(C_{1-5})$alkyl$)(C_{1-3})$alkylamino, $di(C_{1-3})$alkylamino$(C_{1-5})$alkyl, $(di(C_{1-3})$alkylamino$)(C_{1-5})$alkoxy, $(C_{3-7})$cycloalkyl, oxo ('=O'), haloalkyl, and halogen;

both of L and ring Y are absent; or

when L and ring Y are present, L is a direct bond, $-(C_{1-5})$alkylene-, -O-, -(C=O)-, $-O-(C_{1-5})$alkylene-, -(C=O)-NH-, or piperidine, wherein ring Y is a heterocyclic group selected from the group consisting of piperazine, pyrrolidine, morpholino, piperidine, imidazole, and pyrazole; where the heterocyclic group may be substituted with one or more non-hydrogen substituents selected from the group consisting of $(C_{1-5})$alkyl, $di(C_{1-3})$alkylamino, and hydroxy$(C_{0-5})$alkyl, and the case where both of L and ring Y are piperidine may be excluded.

In one specific embodiment,

$R^1$ is phenyl, pyridinyl, cyclohexyl, naphthalenyl, or isoquinolinyl, wherein the phenyl may be substituted with fluoro;
$R^2$ is absent;
ring X is benzene, pyridine, pyrazole, or isoindoline, wherein the benzene, pyridine, pyrazole, or isoindoline may be substituted with one or more non-hydrogen substituents selected from the group consisting of methyl, ethyl, methoxy, ethoxy, (dimethylaminoethyl)methylamino, (dimethylamino)ethyl, (dimethylamino)propyl, (diethylamino)ethoxy, (diethylamino)propoxy, cyclopropyl, oxo ('=O'), trifluoromethyl, and fluoro;
both of L and ring Y are absent; or
when L and ring Y are present, L is a direct bond, $-CH_2-$, -O-, -(C=O)-, $-O-CH_2CH_2-$, $-O-CH_2CH_2CH_2-$, -(C=O)-NH-, or piperidine, wherein the ring Y is a heterocyclic group selected from the group consisting of piperazine, pyrrolidine, morpholino, piperidine, imidazole, and pyrazole, where the heterocyclic ring group may be substituted with one or more non-hydrogen substituents selected from the group consisting of methyl, ethyl, dimethylamino, and hydroxyethyl, and the case where both of L and Y are piperidine may be excluded.

In a more specific embodiment,
when L and ring Y are present,

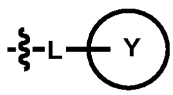

may be

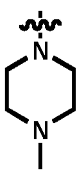, 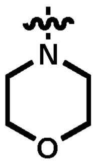, 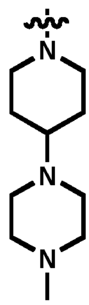, 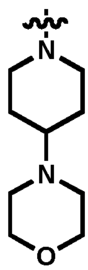, 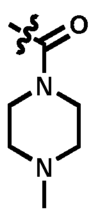, 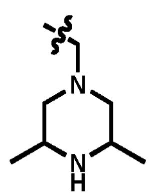, 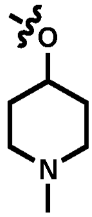,

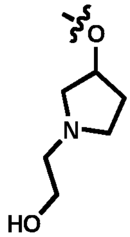, 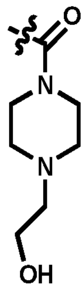, 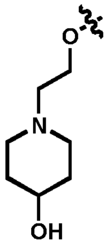, 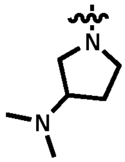, 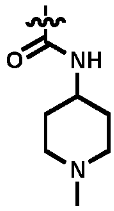, 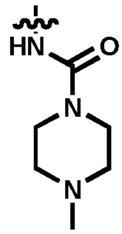, 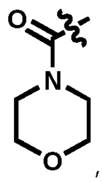, 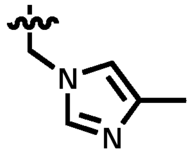, 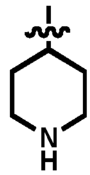,

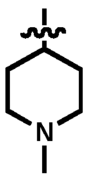, 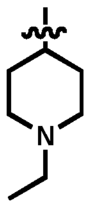, 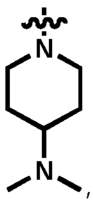, 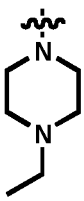, 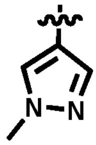, 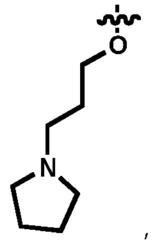, 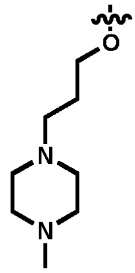, or 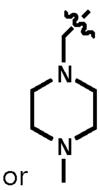.

In another aspect,

in the compound represented by Formula 1 above, when $A^1$ is NH, $A^2$ is CH, and $A^3$ is N,

$R^1$ is phenyl, thiophenyl, or pyridinyl, wherein the phenyl may be substituted with $(C_{1-3})$alkoxy or halogen;

$R^2$ is hydrogen, haloalkyl, cyano, or halogen;

ring X is benzene, pyrazole, tetrahydroisoquinoline, dihydroisoquinoline, or isobenzofuran, wherein the benzene, pyrazole, tetrahydroisoquinoline, dihydroisoquinoline, or isobenzofuran may be substituted with one or more non-hydrogen substituents selected from the group consisting of $(C_{1-5})$alkyl, $(C_{1-5})$alkoxy, $(di(C_{1-3})$alkylamino)$(C_{1-5})$alkoxy, $di(C_{1-3})$alkylaminocarbonyl$(C_{1-3})$alkyl, $(C_{3-7})$cycloalkyl, oxo('=O'), $(C_{1-3})$alkylsulfonyl, $(C_{1-3})$alkylcarbonyl, and hydroxy$(C_{1-5})$alkyl;

L and ring Y are absent; or

when L and ring Y are present, L is a direct bond, -O-, -(C=O)-, -O-$(C_{1-5})$alkylene-,-(C=O)-NH-, or piperidine, wherein ring Y is a heterocyclic group selected from the group consisting of piperazine, pyrrolidine, morpholino, piperidine, and diazabicyclo[2,2,1]heptane, wherein the heterocyclic group may be substituted with one or more non-hydrogen substituents selected from the group consisting of $(C_{1-5})$alkyl, $di(C_{1-3})$alkylamino, hydroxy$(C_{0-5})$alkyl, and $(C_{3-7})$cycloalkyl, and the case where both of L and ring Y are piperidine may be excluded.

In one specific embodiment,

$R^1$ is phenyl, thiophenyl, or pyridinyl, wherein the phenyl may be substituted with methoxy or fluoro;
$R^2$ is hydrogen, trifluoromethyl, cyano, or chloro;
ring X is benzene, pyrazole, tetrahydroisoquinoline, dihydroisoquinoline, or isobenzofuran, wherein the benzene, pyrazole, tetrahydroisoquinoline, dihydroisoquinoline, or isobenzofuran may be substituted with one or more non-hydrogen substituents selected from the group consisting of methyl, ethyl, methoxy, (diethylamino)propoxy, (dimethylamino)carbonylmethyl, cyclopropyl, oxo ('=O'), methylsulfonyl, methylcarbonyl, and branched hydroxybutyl;
L and ring Y are absent; or
when L and ring Y are present, L is a direct bond, -O-, -(C=O)-, $-OCH_2CH_2CH_2-$, - (C=O)-NH-, or piperidine, and ring Y is a heterocyclic group selected from the group consisting of piperazine, pyrrolidine, morpholino, piperidine, and diazabicyclo[2,2,1]heptane, wherein the heterocyclic group may be substituted with one or more non-hydrogen substituents selected from the group consisting of methyl, ethyl, dimethylamino, hydroxyethyl, and cyclopropyl, and the case where both of L and ring Y are piperidine may be excluded.

In a more specific embodiment,
when L and ring Y are present,

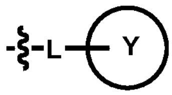

may be

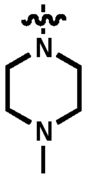, 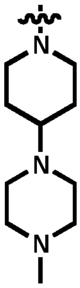, 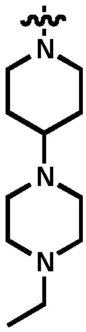, 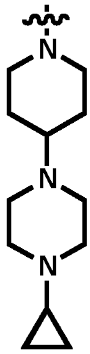, 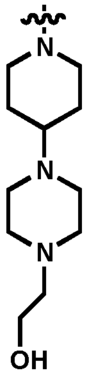, 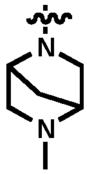, 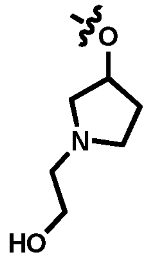,

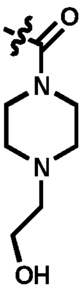, 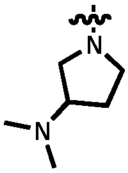, 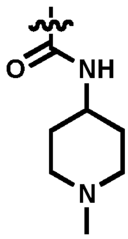, 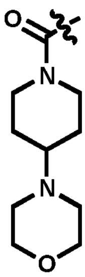, 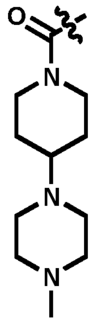, 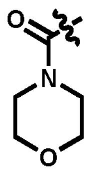, 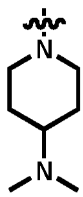, 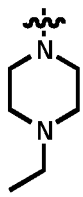, 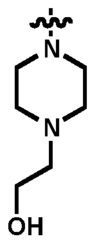,

or

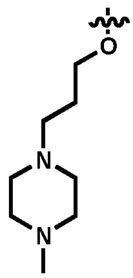.

In another aspect,

in the compound represented by Formula 1, when $A^1$ is CH, $A^2$ is S and $A^3$ is O, or

when $A^1$ is NH, $A^2$ is CH and $A^3$ is N,

$R^1$ is phenyl;

$R^2$ is absent;

ring X is benzene, wherein the benzene may be substituted with $(C_{1-3})$alkoxy or $(C_{3-7})$cycloalkyl;

L is a direct bond, -O-, -(C=O)-, or piperidine; and

ring Y is piperazine or pyrrolidinyl, wherein the piperazine or pyrrolidine may be substituted with $(C_{1-3})$alkyl, hydroxy$(C_{1-3})$alkyl, or di$(C_{1-3})$alkylamino.

In one specific embodiment,

ring X is benzene, wherein the benzene may be substituted with methoxy or cyclopropyl;

L is a direct bond, -O-, -(C=O)-, or piperidine; and

ring Y is piperazine or pyrrolidine, wherein the piperazine or pyrrolidine may be substituted with methyl, hydroxyethyl, or dimethylamino.

In a more specific embodiment,

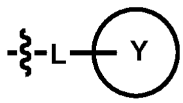

may be

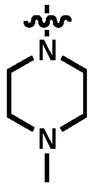 , 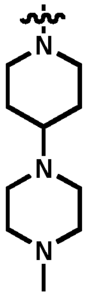 , 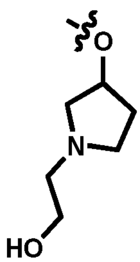 , 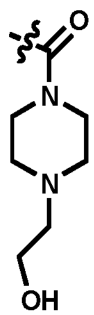 , or 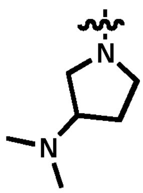 .

In another aspect,

An example of the compound represented by Formula 1 or a pharmaceutically acceptable salt thereof according to the present invention may include the compounds of Examples 1 to 129 listed in [Table 1] below, or pharmaceutically acceptable salts thereof.

The compound of the present invention represented by Formula 1 above may be used in the form of a pharmaceutically acceptable salt, and as the salt, an acid addition salt formed by a pharmaceutically acceptable free acid is useful. The acid addition salt is obtained from inorganic acids such as hydrochloric acid, nitric acid, phosphoric acid, sulfuric acid, hydrobromic acid, hydriodic acid, nitrous acid, and phosphorous acid; nontoxic organic acids such as aliphatic mono- and dicarboxylates, phenyl-substituted alkanoates, hydroxy alkanoates and alkandioates, aromatic acids, and aliphatic and aromatic sulfonic acids; or organic acids such as trifluoroacetic acid, acetate, benzoic acid, citric acid, lactic acid, maleic acid, gluconic acid, methanesulfonic acid, 4-toluenesulfonic acid, tartaric acid, and fumaric acid. Types of such a pharmaceutically non-toxic salt include sulfates, pyrosulfates, bisulfates, sulfites, bisulfites, nitrates, phosphates, monohydrogen phosphates, dihydrogen phosphates, methaphosphates, pyrophosphate chlorides, bromides, iodides, fluorides, acetates, propionates, decanoates, caprylates, acrylates, formates, isobutyrates, caprates, heptanoates, propiolates, oxalates, malonates, succinates, suberates, sebacates, fumarates, maleates, butyne-1,4-dioates, hexane-1,6-dioates, benzoates, chlorobenzoates, methylbenzoates, dinitro benzoates, hydroxybenzoates, methoxybenzoates, phthalates, terephthalates, benzenesulfonates, toluenesulfonates, chlorobenzene sulfonates, xylenesulfonates, phenylacetates, phenylpropionates, phenylbutyrates, citrates, lactates, β-hydroxybutyrates, glycolates, maleates, tartrates, methanesulfonates, propanesulfonates, naphthalene-1-sulfonates, naphthalene-2-sulfonates, and mandelates, and the like.

The acid addition salt according to the present invention may be prepared using conventional methods, and,

for example, by dissolving a derivative of Formula 1 in an organic solvent such as methanol, ethanol, acetone, methylenechloride, or acetonitrile and filtering and drying a precipitate generated by adding an organic acid or an inorganic acid thereto, or may be prepared by distilling a solvent and excess acid under reduced pressure, followed by drying and crystallization under an organic solvent.

In addition, bases can be used to prepare pharmaceutically acceptable metal salts. The alkali metal or alkaline earth metal salt is obtained, for example, by dissolving the compound in an excess alkali metal hydroxide or alkaline earth metal hydroxide solution, filtering the undissolved compound salt, and evaporating and drying the filtrate. At this time, it is pharmaceutically suitable to prepare a sodium, potassium or calcium salt as the metal salt. The corresponding salt is also obtained by reacting an alkali metal or alkaline earth metal salt with a suitable silver salt (e.g., silver nitrate).

Furthermore, the present invention includes not only the compound represented by Formula 1 above and pharmaceutically acceptable salts thereof, but also solvates, optical isomers, hydrates, and the like, which can be prepared therefrom.

In the present specification, "hydrate" may refer to a compound of the present disclosure or salt thereof containing a stoichiometric or non-stoichiometric amount of water bonded by a non-covalent intermolecular force. A hydrate of the compound represented by Formula 1 may contain a stoichiometric or non-stoichiometric amount of water bonded by a non-covalent intermolecular force. The hydrate may contain 1 equivalent or more, preferably, about 1 equivalent to about 5 equivalents of water. Such a hydrate may be prepared by crystallizing the compound represented by Formula 1 of the present disclosure, an isomer thereof, or a pharmaceutically acceptable salt thereof from water or a solvent containing water.

In the present specification, "solvate" may refer to a compound of the present disclosure or salt thereof containing a stoichiometric or non-stoichiometric amount of a solvent bonded by a non-covalent intermolecular force. Suitable solvents therefor include volatile solvents, non-toxic solvents, and/or solvents suitable for administration to humans.

In the present specification, "isomer" may refer to a compound of the present disclosure or salt thereof having the same chemical formula or molecular formula, but being structurally or sterically different. Such isomers include structural isomers such as a tautomer, R or S isomers having an asymmetric carbon center, stereoisomers such as a geometric isomer (trans or cis), and optical isomers (enantiomers). All these isomers and mixtures thereof also fall within the scope of the present disclosure. Unless otherwise specified, a solid line bond (-) connected to an asymmetric carbon atom may include a wedge-shaped solid line bond ( )or a wedge-shaped dotted line bond ( ) indicating the absolute arrangement of stereocenters.

As another aspect of the present invention, as shown in Scheme A below,

a method for preparing the compound represented by Formula 1, an isomer thereof, a solvate thereof, a hydrate thereof or a pharmaceutical thereof, comprising: a step of reacting a compound represented by Formula 2 below with a compound represented by Formula 3 below to prepare a compound represented by Formula 4 below (STEP 1); and

a step of reacting the compound represented by Formula 4 obtained from the previous step with a compound represented by Formula 5 below (STEP 2), is provided:

[Scheme A]

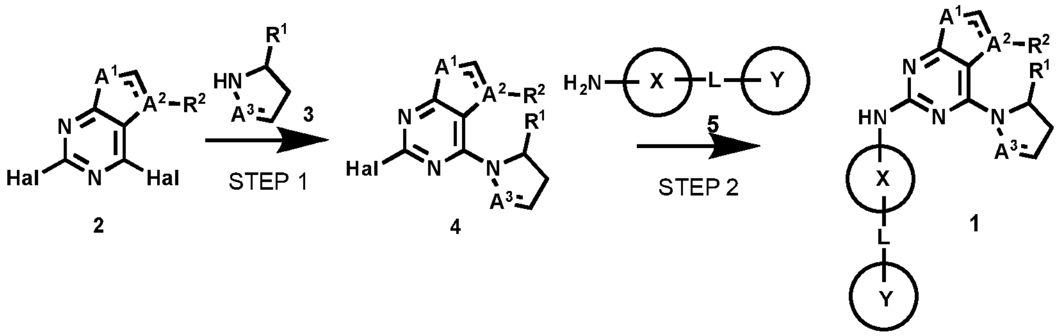


In Scheme A above,

Hal is halogen;

$A^1$, $A^2$, $A^3$, $R^1$, $R^2$, L, ring X, ring Y, and  are as defined above.

Hereinafter, a method for preparing the compound of the present invention represented by Formula 1 above will be described.

In the method for preparing the compound represented by Formula 1 above,
the STEP 1 may be performed by heating them on an organic solvent together with a tertiary amine such as DIPEA (N,N-diisopropylethylamine) or TEA (triethylamine) to 50°C to 70°C, but is not limited thereto. In some cases, in order to obtain a desired product in STEP 1, a reaction of attaching an SEM protecting group to the compound represented by Formula 2 may be additionally performed before the reaction with the compound represented by Formula 3. Here, an example of the SEM protecting group may include a 2-(trimethylsilyl)ethoxymethyl group, a trimethylsilyl group (TMS), a benzyl group or an acetyl group, and the like.

The STEP 2 may be performed by 1) mixing them with an excess of a base in an organic solvent, optionally removing gas from the reaction mixture, and then adding a palladium catalyst, and phosphine derived from biphenyl thereto to react them at 80°C to 120°C, or 2) reacting them with 0.7 to 1.3-fold moles of acid molecules at 80°C to 120°C, but is not limited thereto.

For example, in order to achieve a higher yield, one method for performing STEP 2 above may optionally comprise a step of sonicating the reaction solution before adding the catalyst to remove gas from the reaction mixture, but is not limited thereto. For example, in a specific example of the present invention, the desired compound could be obtained in a higher yield by sonicating it for several minutes before adding the catalyst. In the method, potassium carbonate, cesium carbonate, or the like can be used as the base, $Pd(OAc)_2$ or $Pd_2(dba)_3$ can be used as the palladium catalyst, and Xphos, Xantphos, BINAP (2,2'-bis(diphenylphosphino)-1,1'-binaphthyl), or the like can be sued as the phosphine derived from biphenyl, without being limited thereto. In a specific example of the present invention, it has been confirmed that the desired compound can be obtained in high yield by using potassium carbonate as the base, $Pd_2(dba)_3$ as the palladium catalyst, and Xphos as the copper cocatalyst together, but the type of the base, and the type and combination of catalyst and/or cocatalyst are not limited thereto.

Alternatively, in another method for performing STEP 2 above, it may be performed by adding acid molecules thereto and then heating to maintain the temperature and simultaneously maintaining it for several hours, and as a result, a product in the form of a salt may be obtained. For example, trifluoroacetic acid may be used as the acid molecules, without being limited thereto.

Furthermore, the preparation method of the present invention may further comprise a step of separating optical isomers after STEP 2. For example, the preparation method of the present invention may use, as a reactant, a compound having a stereocenter, that is, a specific optical isomer, or a mixture thereof, or produce a compound having one or more stereocenters in the molecule as a product. As a result, the preparation method of the present invention may also provide a product mainly comprising a specific isomer, such as an (R)-form or (S)-form compound, or may also provide it in a mixed form thereof, such as a racemic mixture mixed in a ratio of 1:1. Therefore, in order to obtain a specific isomer with high purity from such a mixture, the step of separating optical isomers may be further performed. The separation may be performed through supercritical fluid chromatography, but is not limited thereto, and methods known in the art may be used without limitation.

In addition, the preparation method of the present invention may optionally further comprise one or more steps of extraction, drying, concentration and purification between STEP 1 and STEP 2, after STEP 2 or both, but is not limited thereto. The series of steps may be performed using methods known in the art without limitation.

In another aspect of the present invention,
the present invention provides a pharmaceutical composition for preventing or treating diseases related to one or more protein kinases of FLT3, CDK4, CDK6, and HPK1, comprising as an active ingredient, the compound represented by Formula 1 above, a stereoisomer thereof, a hydrate thereof or a pharmaceutically acceptable salt thereof.

The pharmaceutical composition of the present invention may be usefully used to prevent or treat diseases related to one or more protein kinases of FLT3, CDK4, CDK6 and HPK1, such as hematologic malignancies, cancer diseases, metabolic diseases, autoimmune diseases, allergic diseases, degenerative diseases, neurological diseases, hematopoietic cell diseases, or adverse effects on blood production.

Specifically, the protein kinase-related diseases that can be prevented or treated using the pharmaceutical composition of the present invention may be hematologic malignancies including acute myeloid leukemia, myelodysplastic syndrome, acute lymphocytic leukemia, and chronic myeloid leukemia; proliferative diseases including cancer diseases; autoimmune diseases including multiple sclerosis, psoriasis, inflammatory small intestine disease, ulcerative colitis, Crohn's disease, rheumatoid arthritis and polyarthritis, local and systemic scleroderma, systemic lupus erythematosus, discoid lupus erythematosus, cutaneous lupus, dermatomyositis, polymyositis, Sjogren's syndrome, nodular panarteritis, autoimmune enteritis, atopic dermatitis and proliferative glomerulonephritis; allergic diseases including asthma, allergic rhinitis, allergic rhinosinusitis, anaphylaxis, urticaria, angioedema, atopic dermatitis, allergic contact dermatitis, erythema nodosum, erythema multiforme, cortical phlebitis and insect bite dermatitis or blood-sucking parasite infection; or neurological diseases including Huntington's disease, schizophrenia, Parkinson's disease, and Alzheimer's disease, but is not limited thereto.

The term "prevention" of the present invention may mean any action that inhibits or delays the occurrence, spread, and recurrence of the protein kinase-related disease by administration of the composition of the present invention,

and the "treatment" may mean any action that improves or beneficially changes the symptoms of the disease by administration of the composition of the present invention.

The composition of the present invention can inhibit the activity of the protein kinase and inhibit the proliferation of cells expressing the mutation thereof, so that it can be usefully used for the prophylaxis or treatment of diseases caused by abnormality of the protein kinase activity or the expression of the mutation thereof.

The composition of the present invention may further comprise a pharmaceutically acceptable carrier, diluent or excipient; may be formulated in various forms, such as oral dosage forms of powders, granules, tablets, capsules, suspensions, emulsions, syrups, aerosols, or the like and injections of sterile injection solutions, or the like, according to a conventional method for each purpose of use, and used; and may be administered orally or administered through various routes including intravenous, intraperitoneal, subcutaneous, rectal, topical administration, and the like. An example of the suitable carrier, excipient or diluent that can be included in such a composition may include lactose, dextrose, sucrose, sorbitol, mannitol, xylitol, erythritol, maltitol, starch, gum acacia, alginate, gelatin, calcium phosphate, calcium silicate, cellulose, methylcellulose, microcrystalline cellulose, polyvinylpyrrolidone, water, methylhydroxybenzoate, propylhydroxybenzoate, talc, magnesium stearate and mineral oil, and the like. In addition, the composition of the present invention may further comprise a filler, an anti-aggregating agent, a lubricant, a wetting agent, a fragrance, an emulsifier, a preservative, and the like.

Solid formulations for oral administration may include tablets, pills, powders, granules and capsules, etc., and such solid formulations may be formulated by mixing, with at least one compound, at least one excipient, for example, starch, calcium carbonate, sucrose, lactose or gelatin, etc. Further, in addition to simple excipients, lubricants such as magnesium stearate or talc may be used in formulation.

Liquid formulations for oral administration include a suspension, a solution, an emulsion and a syrup, etc. In addition to water commonly used as a simple diluent and liquid paraffin, various excipients, for example, wetting agents, sweetening agents, flavors, preservatives, etc. may be included.

Formulations for parenteral administration include sterile aqueous solutions, non-aqueous solvents, suspensions, emulsions, lyophilized formulations, and suppositories. As the non-aqueous solvents and suspensions, propylene glycol, polyethylene glycol, vegetable oils such as olive oil, injectable esters such as ethyl oleate, and the like may be used. As the base of the suppositories, Witepsol, Macrogol, Tween 61, cacao butter, laurin fat, glycerogelatin, and the like may be used. Meanwhile, injections may comprise conventional additives such as solubilizers, isotonic agents, suspending agents, emulsifiers, stabilizers and preservatives.

The composition of the present invention is administered in a pharmaceutically effective amount. The term "pharmaceutically effective amount" of the present invention means an amount sufficient to treat a disease at a reasonable benefit/risk ratio applicable to medical treatment and causing no side effect, where the effective dose level may be determined depending on factors including the patient's health condition, disease type, severity, drug activity, sensitivity to drug, administration method, administration time, administration route and excretion rate, treatment period and the formulated or concurrently used drugs, and other factors well known in the medical field. The composition of the present invention may be administered as an individual therapeutic agent or may be administered in combination with other therapeutic agents, may be administered sequentially or simultaneously with conventional therapeutic agents, and may be administered singly or multiple times. In consideration of all of the above-described factors, it is important to administer an amount that can obtain the maximum effect with a minimum amount without side effects, which can be easily determined by those skilled in the art.

In another aspect of the present invention,

the present invention provides a method for preventing or treating the protein kinase-related diseases, comprising a step of administering the compound represented by Formula 1 above, a stereoisomer thereof, a hydrate thereof or a pharmaceutically acceptable salt thereof, or a pharmaceutical composition comprising the same as an active ingredient to an individual in need thereof in a therapeutically effective amount.

The term "therapeutically effective amount" used in combination with an active ingredient in the present invention means an amount of a heterocycle-fused pyrimidine derivative compound, a stereoisomer thereof, a hydrate thereof or a pharmaceutically acceptable salt thereof which is effective for preventing or treating a target disease.

Another aspect of the present invention provides the compound, a stereoisomer thereof, a hydrate thereof or a pharmaceutically acceptable salt thereof for use in the treatment of the protein kinase-related disease.

Another aspect of the present invention provides a use of the compound, a stereoisomer thereof, a hydrate thereof or a pharmaceutically acceptable salt thereof for use in the preparation of a medicament for the protein kinase-related treatment.

Since the protein kinase and its related diseases are the same as described above, detailed descriptions are omitted to avoid overlapping descriptions.

Hereinafter, the constitutions and effects of the present invention will be described in more detail through examples. These examples are only for illustrating the present invention, and the scope of the present invention is not limited by these examples.

**<Medium pressure liquid chromatography (MPLC) for purification>**

In medium pressure liquid chromatography, TELEEDYNE ISCO's CombiFlash Rf +UV was used.

**<Analytical LC-MS (ACQUITY UPLC H-Class Core System)>**

A device, in which a QDA Detector manufactured by Waters was mounted on a UPLC system (ACQUITY UPLC PDA Detector) manufactured by Waters, was used. The column used was Waters' ACQUITY UPLC®BEH C18 (1.7μm, 2.1X50mm), and the column temperature proceeded at 30°C.

Mobile phase A was water containing 0.1% formic acid, and mobile phase B was acetonitrile containing 0.1% formic acid.
Gradient condition (10-100% B for 3 minutes, movement speed = 0.6ml/min)

**Prep-150LC System for purification (Preparative-Liquid chromatography UV spectrometry)**

A Prep 150 LC system (2545 Quaternary gradient module, 2998 Photodiode Array Detector, Fraction collector III) manufactured by Waters was used. The column used was the Waters XTERRA®Prep RP18 OBD™ (10μm, 30X300mm), and the column was carried out at room temperature.
Gradient condition (120 minutes with 3-100% B, speed = 40ml/min)

**<SFC conditions for chiral compound separation>**

An 80Q Preparative SFC system manufactured by Waters was used. The column used was DAICEL's CHIRALPAK® AS (10μm, 250X30mm I.D.), and the column temperature proceeded at room temperature. $CO_2$ as a mobile phase was flowed for 130 minutes using methanol, to which 0.1% aqueous ammonia was added, as an auxiliary solvent

**< NMR Analysis >**

NMR analysis was carried out using the AVANCE III 400 or AVANCE III 400 HD manufactured by Bruker, and data was presented in ppm (parts per million(δ)).

The commercially available reagent used was used without additional purification. In the present invention, the room temperature means a temperature of about 20 to 25°C or so. For concentration under pressure and removal of solvent through distillation, a rotary evaporator was used.

**<Preparation Example 1> Preparation of 5-phenyl-4,5-dihydro-1H-pyrazole**

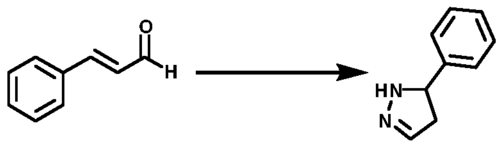

Hydrazine monohydrate (865.98 g, 26.48mol, 977.40 mL) was dissolved in t-BuOH (500 mL), and then heated to 110°C. (E)-cinnamaldehyde (500 g, 3.78 mol, 476.19 mL) was added to the reaction solution and stirred for 20 hours. After completion of the reaction, the temperature was lowered to room temperature, and the reaction mixture was diluted with water (1.5 L) and extracted with dichloromethane (500 mL×4). After the collected organic layers were combined, extracted with brine (500 mL), dried over sodium sulfate and concentrated, the desired compound (500 g) was obtained in a liquid state, which was used in the next reaction without further purification.

MS (m/z): 147.1 $[M+1]^+$

$^1H$ NMR (400 MHz, $CDCl_3$) δ 7.43 - 7.30 (m, 5H), 4.74 (m, 1H), 3.16 (m, 1H), 2.73 (m, 1H).

**<Preparation Example 2> Preparation of (S)-3-phenylisoxazolidine**

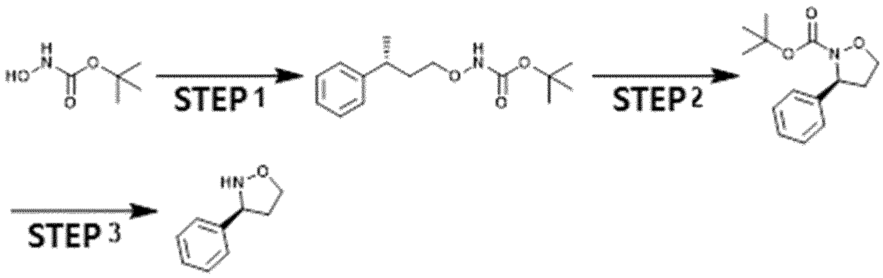


STEP 1: Preparation of tert-butyl (R)-(3-hydroxy-3-phenylpropoxy)carbamate

tert-butyl hydroxycarbamate (7.8 g, 58.6 mmol) was dissolved in dimethylformamide (140 mL), and then sodium hydride (2.58 g, 64.5 mmol) was added at 0°C, and the mixture was reacted for 30 minutes. Then, (R)-3-chloro-1-phenylpropan-1-ol (5 g, 29.3 mmol) dissolved in dimethylformamide (10 mL) was slowly added dropwise at 0°C for 10 minutes, and stirred at room temperature for 72 hours. The reaction was terminated by adding an aqueous ammonium chloride solution to the reaction mixture, which was extracted with ethyl acetate and brine to combine the organic layers. After drying the organic layer over sodium sulfate, it was depressurized and concentrated, and then purified by medium pressure liquid chromatography (ethyl acetate/n-hexane) to obtain the desired compound tert-butyl (R)-(3-hydroxy-3-phenylpropoxy)carbamate (2.8 g, 68%).

MS (m/z): 150.17 $[M+1]^+$

$^1H$ NMR (400 MHz, $CDCl_3$) $\delta$ = 7.43 - 7.39 (m, 2H), 7.38 - 7.32 (m, 2H), 7.27 - 7.24 (m, 1H), 5.05 - 4.97 (m, 1H), 4.15 - 4.08 (m, 1H), 4.07 - 4.00 (m, 1H), 2.10 - 1.93 (m, 2H), 1.54 - 1.48 (m, 9H)

STEP 2: Preparation of *tert*-butyl (S)-3-phenylisoxazolidine-2-carboxylate

The compound *tert*-butyl (R)-(3-hydroxy-3-phenylpropoxy)carbamate (2.55 g, 9.54 mmol) obtained in STEP 1 of Preparation Example 2 and triethylamine (3.13 ml, 22.44 mmol) were dissolved in dichloromethane (250 ml) and then cooled to 0°C. Then, methanesulfonyl chloride (1 ml, 13 mmol) was added dropwise and reacted at 0°C for 2 hours. The reaction mixture was extracted with brine and dichloromethane to combine the organic layers. After drying the organic layer over sodium sulfate, it was depressurized and concentrated to obtain the desired compound *tert*-butyl 3-phenylisooxazolidine-2-carboxylate, which was used in the next reaction without purification.

MS (m/z): 194.13 $[M+1]^+$

$^1H$ NMR (400 MHz, $CDCl_3$) $\delta$ = 7.29 - 7.23 (m, 4H), 7.18 - 7.17 (m, 1H), 5.12 - 5.11 (m, 1H), 4.10 - 4.03 (m, 1H), 3.82- 3.80 (m, 1H), 2.75 - 2.65 (m, 1H), 2.29 - 2.15 (m, 1H), 1.37 (s, 9H)

**STEP 3**: Preparation of (S)-3-phenylisoxazolidine

The compound 3-phenylisoxazolidine-2-carboxylate (2.3 g) obtained in STEP 2 of Preparation Example 2 was dissolved in dichloromethane (90 mL), and then trifluoroacetic acid (14 mL) was added thereto and reacted at room temperature for 1 hour. The reaction mixture was neutralized with aqueous sodium bicarbonate solution, and then the organic layers were combined. After drying the organic layer over sodium sulfate, it was concentrated under reduced pressure, and purified by medium pressure liquid chromatography (tetrahydrofuran/n-hexane) to obtain the desired compound 3-phenylisooxazolidine (1.3 g, 94%).

MS (m/z): 150.08 $[M+1]^+$

$^1H$ NMR (400 MHz, DMSO-$d_6$) $\delta$ = 7.59 - 7.52 (m, 2H), 7.50 - 7.39 (m, 3H), 5.01 - 4.93 (m, 1H), 2.93 - 2.82 (m, 1H), 2.62 - 2.53 (m, 2H)

**<Preparation Example 3> Preparation of (R)-3-phenylisooxazolidine**

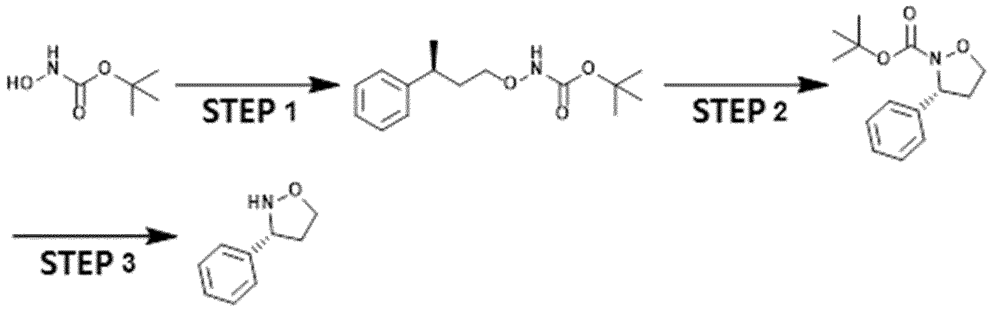


The desired compound was synthesized in a similar manner to Preparation Example 2 and obtained, except for using (S)-3-chloro-1-phenylpropan-1-ol instead of (R)-3-chloro-1-phenylpropan-1-ol in Step 1.

MS (m/z): 150.0 $[M+1]^+$;

$^1H$ NMR (400 MHz, DMSO-$d_6$) $\delta$ = 7.59 - 7.53 (m, 2H), 7.51 - 7.41 (m, 3H), 5.02 - 4.93 (m, 1H), 4.52 - 4.43 (m, 1H), 4.33 - 4.23 (m, 1H), 2.93 - 2.83 (m, 1H), 2.62 - 2.55 (m, 1H)

**<Preparation Example 4> (S)-3-(3-fluorophenyl)isoxazolidine**

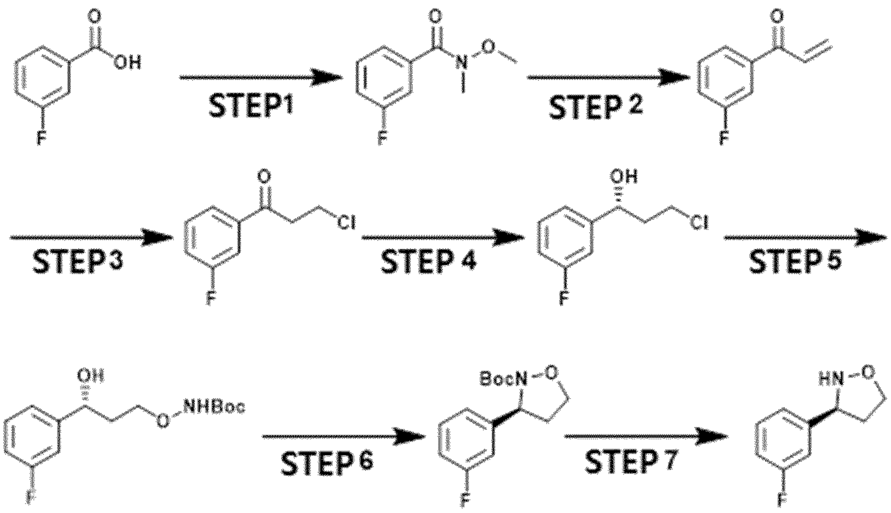


STEP 1: Preparation of 3-fluoro-N-methoxy-N-methylbenzamide

3-fluorobenzoic acid (90 g, 642.35 mmol, 1 eq) is dissolved in pyridine (150 mL), and then N, *O*-dimethylhydroxylamine hydrochloride (75.19 g, 770.81 mmol, 1.2 eq) is added. Then, EDCI (147.77 g, 770.81 mmol, 1.2 eq) at 15°C was added. The reaction mixture is stirred at 50°C for 30 minutes. As a result of TLC analysis (Hex: EA = 3:1), all starting materials disappeared, and new spots with low polarity were detected. The pyridine solvent was removed by concentration under reduced pressure, and the organic layer was extracted using DCM (500 mL), HCl (500 mL, 2N), and brine (200 mL). The organic layer was dried over sodium sulfate and concentrated under reduced pressure to obtain the desired compound 3-fluoro-N-methoxy-N-methylbenzamide (110 g, 600.50 mmol, 93.49% yield) as a yellow oil.

STEP 2: Preparation of 1-(3-fluorophenyl)prop-2-en-1-one

3-fluoro-N-methoxy-N-methyl-benzamide (110 g, 600.50 mmol, 1 eq) obtained in STEP 1 of Preparation Example 4 was dissolved in THF (1 L), and then bromo(vinyl)magnesium (1 M, 630.53 mL, 1.05 eq) at 0°C was added dropwise at -78°C. Then, the reaction mixture was stirred at 0°C for 30 minutes. As a result of TLC analysis (Hex: EA = 4:1), all starting materials disappeared, and new spots with low polarity were detected. After the reaction was terminated by adding HCl (4N, 500 mL), the organic layer was extracted using MTBE (2000 mL) and brine (500 mL). The organic layer was dried over sodium sulfate, and then concentrated under reduced pressure. The concentrated compound was purified using medium pressure liquid chromatography (Hex: EA = 30:1) to obtain the desired compound 1-(3-fluorophenyl)prop-2-en-1-one (80 g, 532.80 mmol, 88.73% yield) in a yellow oil state.

STEP 3: Preparation of 3-chloro-1-(3-fluorophenyl)propan-1-one

1-(3-fluorophenyl) prop-2-en-1-one (71 g, 472.86 mmol, 1.0 eq) obtained in STEP 2 of Preparation Example 4 was dissolved in DCM (71 mL), and then HCl/dioxane (4M, 295.54 mL, 2.5 eq) was added at 0°C. Thereafter, the reaction mixture was stirred at 15°C for 1.5 hours. As a result of TLC analysis (Hex: EA = 10:1), all starting materials disappeared, and the desired compound was detected. The reaction mixture was concentrated under reduced pressure, and the organic layer was extracted by adding DCM (450 mL) and water (200 mL * 5), dried over sodium sulfate, and then concentrated under reduced pressure to obtain the desired compound 3-chloro-1-(3)-fluorophenyl) propan-1-one (73 g, 391.19 mmol, 82.73% yield) as a yellow solid.

$^1$H NMR (400 MHz, $CDCl_3$) $\delta$ = 7.78 - 7.72 (m, 1H), 7.69 - 7.60 (m, 1H), 7.53 - 7.44 (m, 1H), 7.37 - 7.24 (m, 1H), 3.93 (t, *J* = 6.8 Hz, 2H), 3.46 (t, *J =6.8* Hz, 2H)

STEP 4: Preparation of (R)-3-chloro-1-(3-fluorophenyl)propan-1-ol

(S)-1-methyl-3,3-diphenyltetrahydro-1H,3H-pyrrolo[1,2-c][1,3,2]oxazaborol (8.61 g, 31.08 mmol, 9.27 mL, 0.1 eq) was dissolved in THF (380 mL), and then $BH_3$.THF (1 M, 186.48 mL, 0.6 eq) was added dropwise at 0°C under a nitrogen stream. The reaction mixture was stirred at 0°C for 30 minutes. Thereafter, 3-chloro-1-(3-fluorophenyl)propan-1-one (70 g, 375.11 mmol, 1 eq) obtained in STEP 3 of Preparation Example 4, which was diluted in THF (390 mL), was added dropwise to the reaction mixture at 0°C. The reaction mixture was stirred at 0°C for 30 minutes. As a result of TLC analysis (Hex: EA = 5:1), all starting materials disappeared, and the desired compound spots were detected. After the reaction was terminated by adding MeOH (100 mL) at 0°C, the solvent was blown off under reduced pressure. The organic layer was extracted from the concentrated compound using DCM (100 mL * 3) and $NH_4Cl$ solution (300 mL). The organic layer was dried over sodium sulfate and then concentrated under reduced pressure. The concentrated compound was purified by using medium pressure liquid chromatography (Hex: EA = 50:1 to 5:1) to obtain (R)-3-chloro-1-(3-fluorophenyl)propan-1-ol (55 g, 286.33 mmol, 92.13% yield, 98.2% purity, 54.1% e.e.) as a colorless oil.

MS (m/z): 135.2 $[M-56+H]^+$

$^1$H NMR (400 MHz, $CDCl_3$) $\delta$ = 7.35 - 7.28 (m, 1H), 7.19 - 7.04 (m, 2H), 7.01 - 6.91 (m, 1H), 5.00 - 4.88 (m, 1H), 3.76 - 3.70 (m, 1H), 3.60 - 3.51 (m, 1H), 2.24 - 2.13 (m, 1H), 2.12 - 1.98 (m, 2H)

STEP 5: Preparation of *tert*-butyl (R)-(3-(3-fluorophenyl)-3-hydroxypropoxy) carbamate

tert-butyl hydroxycarbamate (40.76 g, 306.16 mmol, 1.05 eq) was dissolved in DMF (400 mL), and then NaH (12.83 g, 320.74 mmol, 60% purity, 1.1 eq) was added at 0°C under a nitrogen stream. The reaction mixture was stirred at 10°C for 1 hour, and (1R)-3-chloro-1-(3-fluorophenyl)propane-1-ol (55 g, 291.58 mmol, 1 eq) obtained in STEP 4 of Preparation Example 4, which was diluted in DMF (150 mL), was added dropwise at 0°C and stirred at 10°C for 16 hours. As a result of TLC analysis (Hex: EA = 2:1), all starting materials disappeared, and the desired compound was detected. After the reaction was terminated by adding water (800 mL), the resulting solid was filtered. The organic layer was extracted from the recovered solid using ethyl acetate (800 mL), water (100 mL) and brine (100 mL). The organic layer was dried over sodium sulfate and then concentrated under reduced pressure to obtain tert-butyl (R)-(3-(3-fluorophenyl)-3-hydroxypropoxy)carbamate (72 g, 189.57 mmol, 65.01% yield, 75.12% purity) as a light yellow solid.

MS (m/z): 167.9 $[M-118]^+$

$^1$H NMR (400 MHz, $CDCl_3$) $\delta$ = 7.17 - 7.06 (m, 1H), 7.01 - 6.93 (m, 2H), 6.80 - 6.73 (m, 1H), 4.89 - 4.79 (m, 1H), 3.95 - 3.89 (m, 1H), 3.88 - 3.82 (m, 1H), 1.89 - 1.81 (m, 1H), 1.80 - 1.69 (m, 1H), 1.32 (s, 9H)

STEP 6: Preparation of *tert*-butyl (S)-3-(3-fluorophenyl)isoxazolidine-2-carboxylate

tert-butyl (R)-(3-(3-fluorophenyl)-3-hydroxypropoxy)carbamate (72 g, 252.36 mmol, 1 eq) obtained in STEP 5 of Preparation Example 4 and $Et_3N$ (76.61g, 757.07mmol, 105.38mL, 3eq) were dissolved in DCM (700 mL), and then methanesulfonic anhydride (65.94 g, 378.53 mmol, 1.5 eq) was slowly added at 0°C. The reaction mixture was stirred at 20°C for 12 hours. As a result of TLC analysis (PE: EA = 3:1), all starting materials disappeared, and new spots were detected. After the reaction was terminated by adding water (1000 mL), the organic layer was extracted using DCM (200 mL * 3). The organic layer was dried over sodium sulfate and then concentrated under reduced pressure. The concentrated compound was purified by medium pressure liquid chromatography (PE: EA = 50:1 to 5:1) to extract 35 g of the desired compound having an e.e value of 57.7%. The desired compound was purified through SFC to obtain tert-butyl (S)-3-(3-fluorophenyl)isoxazolidine-2-carboxylate (25 g, 92.00 mmol, 36.17% yield, 98.37% purity, 100% e.e.) and tert-butyl (R)-3-(3-fluorophenyl)isoxazolidine-2-carboxylate (6.5 g, 22.16 mmol, 8.71% yield, 91.13% purity, 100% e.e.) as a light

yellow solid.

MS (m/z): 212.2 [M-56+H]$^+$

$^1$H NMR (400 MHz, $CDCl_3$) δ = 7.34 - 7.27 (m, 1H), 7.17 - 7.05 (m, 2H), 6.99 - 6.91 (m, 1H), 5.20 (dd, *J* = 5.6, 8.8 Hz, 1H), 4.22 - 4.14 (m, 1H), 3.94 - 3.84 (m, 1H), 2.84 - 2.73 (m, 1H), 2.34 - 2.22 (m, 1H), 1.47 (s, 9H)

STEP 7: Preparation of (S)-3-(3-fluorophenyl)isoxazolidine

*tert-butyl* (3S)-3-(3-fluorophenyl)isoxazolidine-2-carboxylate (25 g, 93.53 mmol, 1 eq) obtained in STEP 6 of Preparation Example 4 was dissolved in EA (50 mL) and then 4M HCl/EA (250 mL) was added at 0°C. Thereafter, the reaction mixture was stirred at 10°C for 1 hour. As a result of LC-MS analysis, all starting materials disappeared and 96.97% of the desired compound was detected. In order to obtain a solid, it was concentrated under reduced pressure to obtain (S)-3-(3-fluorophenyl)isoxazolidine (19.12 g, 86.71 mmol, 92.71% yield, 92.35% purity, HCl, 99.91% e.e.) as a white solid.

MS (m/z): 168.3 [M+H]$^+$

$^1$H NMR (400 MHz, DMSO-$d_6$) δ = 12.51 (br s, 1H), 7.55 - 7.44 (m, 2H), 7.44 - 7.37 (m, 1H), 7.32 - 7.22 (m, 1H), 5.03 (t, *J* =8.0 Hz, 1H), 4.54 - 4.43 (m, 1H), 4.34 - 4.24 (m, 1H), 2.95 - 2.83 (m, 1H), 2.64 - 2.52 (m, 1H)

Preparation Examples 5 to 28 were prepared in a similar manner to Preparation Examples 1, 2, 3 and 4, and the compound names, chemical structures, UPLC and NMR analysis results of Preparation Examples 5 to 28 were shown below and were used upon the preparation of the following examples.

**<Preparation Example 5> 5-(4-chlorophenyl)-4,5-dihydro-1H-pyrazole**

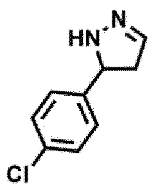

MS (m/z): 181.1 [M+1]$^+$

<Preparation Example 6> **5-(3,5-difluorophenyl)-4,5-dihydro-1H-pyrazole**

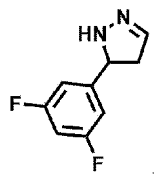

MS (m/z): 183.1 [M+1]$^+$

**<Preparation Example 7> 5-(4-(trifluoromethyl)phenyl)-4,5-dihydro-1H-**pyrazole

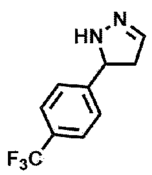

MS (m/z): 215.1 [M+1]$^+$

**<Preparation Example 8> 5-(3-fluorophenyl)-4,5-dihydro-1H-pyrazole**

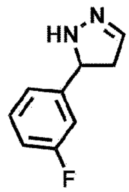

MS (m/z): 165.1 [M+1]+

**<Preparation Example 9> 5-(4-fluorophenyl)-4,5-dihydro-1H-pyrazole**

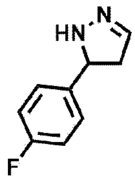

MS (m/z): 165.1 [M+1]+

**<Preparation Example 10> 3-(4,5-dihydro-1H-pyrazol-5-yl)pyridine**

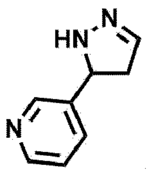

MS (m/z): 148.1 [M+1]+

**<Preparation Example 11> 2-(4,5-dihydro-1H-pyrazol-5-yl)pyridine**

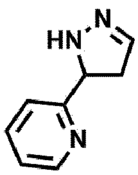

MS (m/z): 148.1 [M+1]+

**<Preparation Example 12> 4-(4,5-dihydro-1H-pyrazol-5-yl)pyridine**

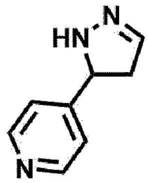

MS (m/z): 148.1 $[M+1]^+$

**<Preparation Example 13> 5-(4-methoxyphenyl)-4,5-dihydro-1H-pyrazole**

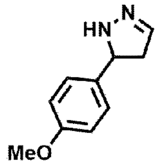

MS (m/z): 177.2 $[M+1]^+$

<Preparation Example 14> (R)-3-(3-fluorophenyl)isoxazolidine

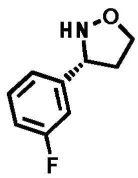

$^1$H NMR (400 MHz, DMSO-$d_6$) δ = 12.31 (br s, 1H), 7.55 - 7.43 (m, 2H), 7.43 - 7.37 (m, 1H), 7.32 - 7.22 (m, 1H), 5.04 - 5.00 (t, $J$ = 7.6 Hz, 1H), 4.51 - 4.43 (m, 1H), 4.32 - 4.23 (m, 1H), 2.95 - 2.82 (m, 1H), 2.63 - 2.51 (m, 1H)

**<Preparation Example 15> (S)-3-(3,5-difluorophenyl)isoxazolidine**

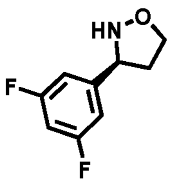

$^1$H NMR (400 MHz, DMSO-$d_6$) δ = 7.36 - 7.28 (m, 3H), 5.04 - 4.98 (t, $J$ = 7.6 Hz, 1H), 4.47 - 4.38 (m, 1H), 4.25 - 4.19 (dd, $J$ = 7.6, 15.2 Hz, 1H), 2.89 - 2.81 (m, 1H), 2.58 - 2.51 (m, 1H)

**<Preparation Example 16> (R)-3-(3,5-difluorophenyl)isoxazolidine**

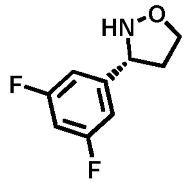

$^1$H NMR (400 MHz, DMSO-$d_6$) δ = 7.36 - 7.27 (m, 3H), 5.04 - 4.98 (t, *J* = 7.6 Hz, 1H), 4.46 - 4.36 (m, 1H), 4.25 - 4.19 (dd, *J* = 7.6, 15.2 Hz, 1H), 2.90 - 2.78 (m, 1H), 2.56 - 2.51 (m, 1H)

<Preparation Example 17> (S)-3-(4-chlorophenyl)isoxazolidine

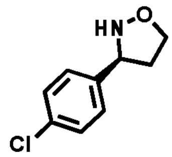

$^1$H NMR (400 MHz, MeOD) δ = 7.58 - 7.52 (m, 4H), 5.19 - 5.15 (t, *J* = 7.6 Hz, 1H), 4.71 - 4.66 (m, 1H), 4.48 - 4.42 (m, 1H), 3.04 - 3.0 (m, 1H), 2.81 - 2.75 (m, 1H)

**<Preparation Example 18> (S)-3-(4-bromophenyl)isoxazolidine**

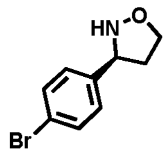

$^1$H NMR (400 MHz, MeOD) δ = 7.69 - 7.65 (d, *J* = 8.6 Hz, 2H), 7.48 - 7.46 (d, *J* = 8.6 Hz, 2H), 5.16 - 5.12 (m, 1H), 4.67 - 4.63 (m, 1H), 4.46 - 4.40 (m, 1H), 3.02 - 2.98 (m, 1H), 2.78 - 2.72 (m, 1H)

**<Preparation Example 19> (S)-3-(3,4-dichloro-2-fluorophenyl)isoxazolidine**

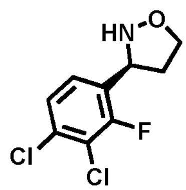

$^1$H NMR (400 MHz, DMSO-$d_6$) δ = 7.64 - 7.58 (m, 2H), 5.16 - 5.06 (dd, *J* = 6.4, 8.0 Hz, 1H), 4.44 - 4.32 (m, 1H), 4.20 - 4.10 (dd, *J* = 7.6, 15.2 Hz, 1H), 2.91 - 2.76 (m, 1H), 2.57 -2.51 (m, 1H)

**<Preparation Example 20> (S)-3-(4-chloro-3-fluorophenyl)isoxazolidine**

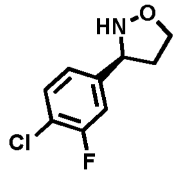

$^1H$ NMR (400MHz, DMSO-$d_6$) δ = 7.78 - 7.63 (m, 2H), 7.43 - 7.40 (dd, *J* = 1.6, 8.0 Hz, 1H), 5.03 - 4.99 (t, *J* = 7.6 Hz, 1H), 4.47 - 4.40 (m, 1H), 4.26 - 4.20 (q, *J* = 7.6 Hz, 1H), 2.90 - 2.82 (m, 1H), 2.58 - 2.52 (m, 1H)

**<Preparation Example 21> (S)-3-(3-chloro-4-fluorophenyl)isoxazolidine**

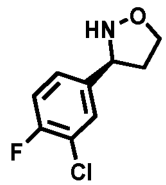

$^1H$ NMR (400 MHz, DMSO-$d_6$) δ = 7.80 - 7.87 (dd, *J* = 2.0,7.2 Hz, 1H), 7.55 - 7.50 (m, 2H), 4.98 - 4.94 (t, *J* = 8.0 Hz, 1H), 4.43 - 4.39 (m, 1H), 4.22 - 4.18 (m, 1H), 2.85 - 2.81(m, 1H), 2.54 - 2.53 (m, 1H)

**<Preparation Example 22> (S)-3-(2,5-difluorophenyl)isoxazolidine**

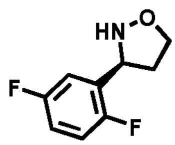

$^1H$ NMR (400MHz, DMSO-$d_6$) δ = 7.55 - 7.49 (m, 1H), 7.39 - 7.30 (m, 2H), 5.13 - 5.10 (t, *J* = 7.2 Hz, 1H), 4.47 - 4.39 (m, 1H), 4.22 - 4.17 (q, *J* = 7.6 Hz, 1H), 2.93 - 2.82 (m, 1H), 2.59 - 2.52 (m, 1H)

**<Preparation Example 23> (S)-3-(3-chloro-2-fluorophenyl)isoxazolidine**

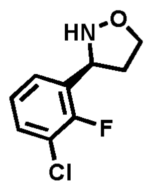

$^1H$ NMR (400 MHz, DMSO-$d_6$) δ = 7.49 - 7.43 (m, 2H), 7.20 - 7.16 (m, 1H), 6.56 (s, 1H), 4.65 (s, 1H), 3.96 - 3.91 (m, 1H), 3.67 - 3.65 (m, 1H), 2.66 - 2.61 (m, 1H), 2.08 - 2.01 (m, 1H)

**<Preparation Example 24> (S)-3-(3-methoxyphenyl)isoxazolidine**

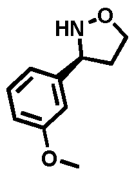

$^{1}$H NMR (400 MHz, DMSO-$d_6$) δ = 7.37 (t, *J* = 8.0 Hz, 1H), 7.23 - 7.20 (m, 1H), 7.13 (d, *J* = 7.7 Hz, 1H), 7.01 (dd, *J* = 8.3, 2.5 Hz, 1H), 4.96 (t, *J* = 8.1 Hz, 1H), 4.49 (td, *J* = 8.1, 4.0 Hz, 1H), 4.30 (q, *J* = 7.7 Hz, 1H), 3.79 (s, 3H), 2.88 (dtd, *J* = 11.5, 7.4, 4.0 Hz, 1H), 2.58 (dq, *J* = 12.5, 8.5 Hz, 1H)

**<Preparation Example 25> (S)-3-(6-methylpyridin-3-yl)isoxazolidine**

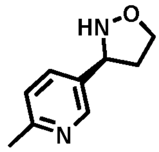

$^{1}$H NMR (400 MHz, MeOD) δ = 8.98 (d, *J* = 2.0 Hz, 1H), 8.71-8.68 (dd, *J* = 2.0, 8.4 Hz, 1H), 8.09 - 8.07 (d, *J* = 8.4 Hz, 1H), 5.53 - 5.49 (dd, *J* = 6.8, 8.0 Hz, 1H), 4.79-4.75 (m, 1H), 4.49 - 4.43 (m, 1H), 3.17 - 3.11 (m, 1H), 2.94 - 2.78 (m, 4H)

**<Preparation Example 26> (S)-3-(pyridin-3-yl)isoxazolidine**

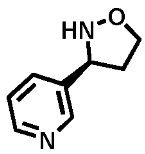

$^{1}$H NMR (400 MHz, DMSO-$d_6$) δ = 9.09 - 9.08 (d, *J* = 1.6 Hz, 1H), 8.96 - 8.95 (d, *J* = 5.2 Hz 1H), 8.76 - 8.74 (m, 1H), 8.15 - 8.12 (dd, *J* = 5.6 Hz, 8.0 Hz, 1H), 5.34 -5.30 (m, 1H), 4.52 - 4.48 (m, 1H), 4.29 - 4.25 (m, 1H), 2.94 - 2.91 (m, 1H), 2.69 - 2.66 (m, 1H)

**<Preparation Example 27> (S)-5-(isooxazolidin-3-yl)nicotinonitrile**

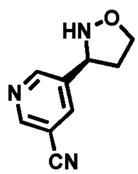

$^{1}$H NMR (400 MHz, $CDCl_3$) δ = 8.79 - 8.76 (dd, *J* = 2.0, 12.4 Hz, 2H), 8.09 (s, 1H), 5.58 (br s, 1H), 4.67 - 4.66 (m, 1H), 4.19 - 4.14 (m, 1H), 3.84 - 3.82 (m, 1H), 2.82 - 2.77 (m, 1H), 2.29 - 2.25 (m, 1H)

**<Preparation Example 28> (S)-3-(4-(trifluoromethyl)thiazol-2-yl)isoxazolidine**

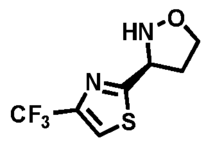

$^1H$ NMR (400 MHz, $CDCl_3$) δ = 7.71 (s, 1H), 5.76 (br d, *J* = 4.4 Hz, 1H), 4.91 - 4.88 (m, 1H), 4.20 - 4.15 (m, 1H), 3.80 (m, 1H), 2.81 - 2.76 (m, 2H)

**<Example 8> Preparation of N-(3-methoxy-4-(4-(4-methylpiperazin-1-yl)piperidin-1-yl)phenyl)-4-(5-phenyl-4,5-dihydro-1H-pyrazol-1-yl)thieno[3,2-d]pyrimidin-2-amine**

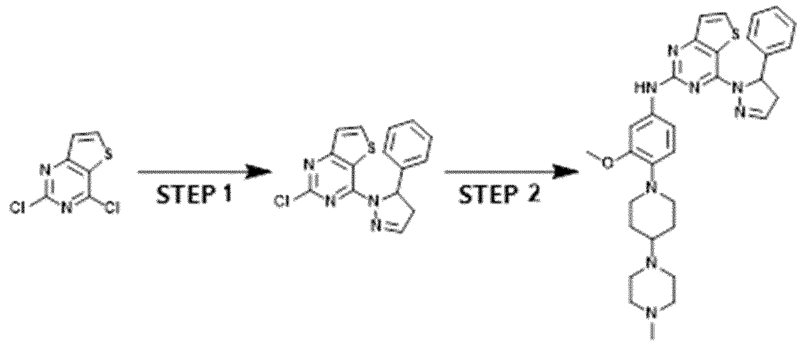


STEP 1: Preparation of 2-chloro-4-(5-phenyl-4,5-dihydro-1H-pyrazol-1-yl)thieno[3,2-d]pyrimidine

2,4-dichlorothieno[3,2-d]pyrimidine (1.53 g, 7.46 mmol), 5-phenyl-4,5-dihydro-1H-pyrazole (2 mL, 8.21 mmol) prepared according to Preparation Example 1 above and DIPEA (2.61 mL, 14.92 mmol) were dissolved in DMSO (19 mL), and then reacted at 60°C for 4 hours. After completion of the reaction, the temperature was lowered to room temperature, and the reaction mixture was extracted with ethyl acetate and water to recover the organic layer. The obtained organic layer was dried over sodium sulfate, and then concentrated under reduced pressure, and purified by medium pressure liquid chromatography (n-hexane/ethyl acetate) to obtain the desired compound (1 g, 43% yield).
MS (m/z): 315.2 $[M+1]^+$
$^1H$ NMR (400 MHz, DMSO-$d_6$) δ 8.31 (d, *J* = 5.5 Hz, 1H), 7.59 (s, 1H), 7.41 - 7.19 (m, 6H), 5.71 (dd, *J* = 11.7, 4.2 Hz, 1H), 3.70 (ddd, *J* = 18.8, 11.7, 1.0 Hz, 1H), 2.94 (ddd, *J*= 19.0, 4.0, 1.5 Hz, 1H)

STEP 2: Preparation of N-(3-methoxy-4-(4-(4-methylpiperazin-1-yl)piperidin-1-yl)phenyl)-4-(5-phenyl-4,5-dihydro-1H-pyrazol-1-yl)thieno[3,2-dlpyrimidin-2-amine

2-chloro-4-(5-phenyl-4,5-dihydro-1H-pyrazol-1-yl)thieno[3,2-d]pyrimidine (300 mg, 0.75 mmol) obtained in STEP 1 of Example 8, and 3-methoxy-4-(4-(4-methylpiperazin-1-yl)piperidin-1-yl)aniline (290 mg, 0.75 mmol) and $K_2CO_3$ (312 mg, 2.26 mmol) were dissolved in sec-BuOH (2.5 mL), and then sonicated under nitrogen for 5 minutes to remove gas. $Pd_2(dba)_3$ (69 mg, 0.075 mmol) and Xphos (71.8 mg, 0.151 mmol) were added to the reaction mixture, and then reacted at 100°C for 3 hours. After completion of the reaction, the temperature was lowered to room temperature, and the reactant was filtered through diatomaceous earth. The obtained filtrate was concentrated under reduced pressure, and then purified by medium pressure chromatography (MeOH/DCM 0~10%) to obtain the desired compound N-(3-methoxy-4-(4-(4-methylpiperazin-1-yl)piperidin-1-yl)phenyl)-4-(5-phenyl-4,5-dihydro-1H-pyrazol-1-yl)thieno[3,2-d]pyrimidin-2-amine (224 mg, 51% yield) in a solid state.
MS (m/z): 583.4 $[M+1]^+$
$^1H$ NMR (400 MHz, DMSO-$d_6$) δ 8.70 (s, 2H), 8.06 (d, *J*= 5.5 Hz, 2H), 7.40 (s, 2H), 7.36 - 7.17 (m, 11H), 7.12 (dd, *J* = 11.4, 5.8 Hz, 6H), 5.75 (dd, *J* = 11.7, 3.9 Hz, 2H), 3.74 - 3.65 (m, 9H), 3.31 (t, *J* = 10.2 Hz, 6H), 2.83 (dd, *J* = 18.6, 2.3 Hz, 3H), 2.61 (d, *J* = 51.9 Hz, 15H), 2.37 (s, 6H), 1.84 (d, *J* = 10.9 Hz, 4H), 1.56 (dd, *J* = 20.5, 11.0 Hz, 4H)

**<Example** 57> **Preparation of (*S*)-*N*-(3-methoxy-4-(4-(4-methylpiperazin-1-yl)piperidin-1-yl)phenyl)-4-(3-phenylisooxazolidin-2-yl)thieno[3,2-*d*]pyrimidin-2-amine**

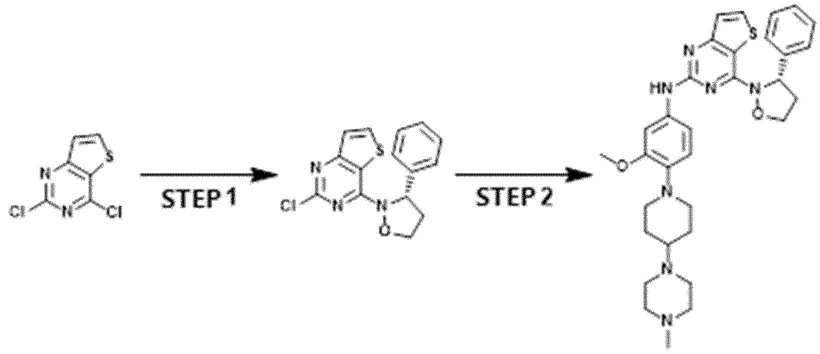


STEP 1: Preparation of (S)-2-(2-chlorothieno[3.2-d]pyrimidin-4-yl)-3-phenylisoxazolidine

2,4-dichlorothieno[3,2-d]pyrimidine (1 g, 5.18 mmol), (S)-3-phenylisoxazolidine (0.85 g, 5.70 mmol) prepared according to Preparation Example 2 and DIPEA (1.8 ml, 10.36 mmol) were dissolved in DMSO (10 ml) and stirred at 60°C for 4 hours. After completion of the reaction, the temperature was lowered to room temperature, and the reaction mixture was extracted with ethyl acetate and water to recover the organic layer. The obtained organic layer was dried over sodium sulfate, and then concentrated under reduced pressure, and purified by medium pressure liquid chromatography (EA/Hex 0~40%) to obtain the desired compound (S)-2-(2-chlorothieno[3,2-d]pyrimidin-4-yl)-3-phenylisooxazolidine (1.7 g, 85% yield).

MS (m/z): 318.1 $[M+1]^+$

STEP 2: Preparation of (S)-N-(3-methoxy-4-(4-(4-methylpiperazin-1-yl)piperidin-1-yl)phenyl)-4-(3-phenylisooxazolidin-2-yl)thieno[3,2-d]pyrimidin-2-amine

(S)-2-(2-chlorothieno[3,2-d]pyrimidin-4-yl)-3-phenylisoxazolidine (95 mg, 0.3 mmol) obtained in STEP 1 of Example 57, and 3-methoxy-4-(4-(4-methylpiperazin-1-yl)piperidin-1-yl)aniline (90 mg, 0.3 mmol) and $K_2CO_3$ (123 mg, 0.89 mmol) were dissolved in sec-BuOH (1 mL), and then sonicated under nitrogen for 5 minutes to remove gas. $Pd_2(dba)_3$ (27 mg, 0.03 mmol) and Xphos (28 mg, 0.06 mmol) were added to the reaction mixture, and then reacted at 100°C for 3 hours. After completion of the reaction, the temperature was lowered to room temperature, and the reactant was filtered through diatomaceous earth. The obtained filtrate was concentrated under reduced pressure, and then purified by medium pressure liquid chromatography (MeOH/DCM 0~15%) to obtain the desired compound (S)-N-(3-methoxy-4-(4-(4-methylpiperazin-1-yl)piperidin-1-yl)phenyl)-4-(3-phenylisooxazolidin-2-yl)thieno[3,2-d]pyrimidine-2-amine (94 mg, 54% yield).

MS (m/z): 586.4 $[M+1]^+$

$^1$H NMR (400 MHz, DMSO-$d_6$) δ 9.06 (s, 1H), 8.13 (d, *J* = 5.5 Hz, 1H), 7.50 - 7.45 (m, 2H), 7.42 - 7.34 (m, 3H), 7.33 - 7.27 (m, 1H), 7.21 (d, *J* = 5.5 Hz, 1H), 6.70 (d, *J* = 8.6 Hz, 1H), 5.89 (dd, *J* = 8.7, 5.3 Hz, 1H), 3.84 (q, *J*= 7.9 Hz, 1H), 3.62 (s, 3H), 3.31 - 3.25 (m, 2H), 2.97 - 2.87 (m, 1H), 2.54 (s, 2H), 2.46 - 2.22 (m, 9H), 2.19 (s, 3H), 1.79 (d, *J*= 12.0 Hz, 2H), 1.55 - 1.48 (m, 2H), 1.31 - 1.20 (m, 2H)

**<Example 69> Preparation of *N*-(3-methoxy-4-(4-(4-methylpiperazin-1-yl)piperidin-1-yl)phenyl)-4-(5-phenyl-4,5-dihydro-1*H*-pyrazol-1-yl)-7*H*-pyrrolo[2,3-*d*]pyrimidin-2-amine**

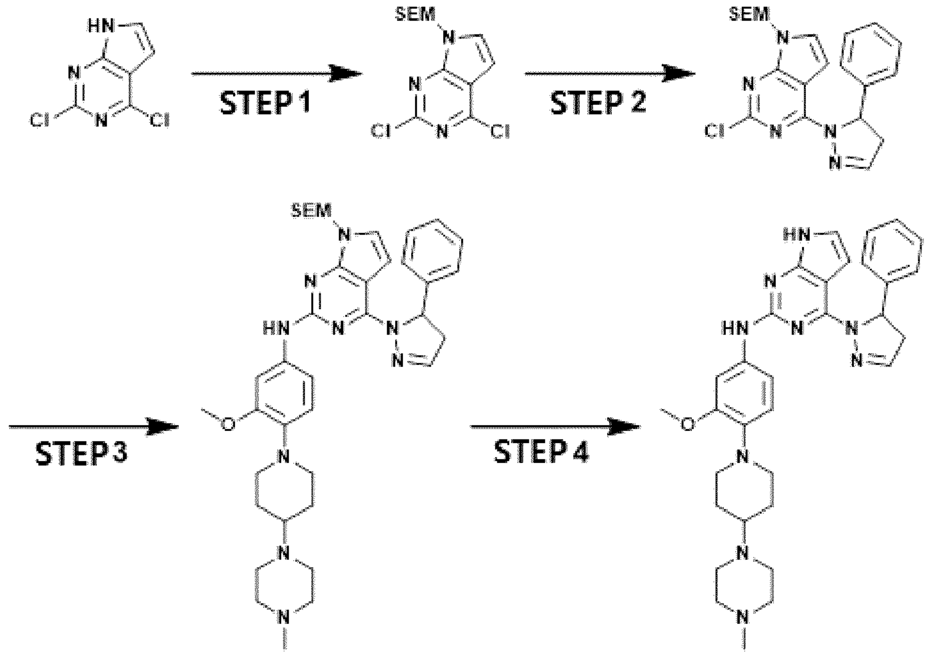

STEP 1: Preparation of 2,4-dichloro-7-((2-(trimethylsilyl)ethoxy)methyl)-7H-pyrrolo[2,3-d]pyrimidine

2,4-dichloro-7H-pyrrolo[2,3-d]pyrimidine (5 g, 26.6 mmol) was dissolved in DMF (26 ml), and then NaH (1.3 g) was slowly added thereto at 0°C. The reaction mixture was stirred at 0°C for 30 minutes, and then (2-(chloromethoxy)ethyl)trimethylsilane (7.08 ml, 39.9 mmol) was slowly added dropwise thereto. The reaction mixture was stirred at room temperature for 2 hours, and then added to excess water and the resulting solid was filtered to obtain the desired compound 2,4-dichloro-7-((2-(trimethylsilyl)ethoxy)methyl)-7H-pyrrolo[2,3-d]pyrimidine (8.2 g 97% yield).

MS (m/z): 318.1 $[M+1]^+$

STEP 2: Preparation of 2-chloro-4-(5-phenyl-4,5-dihydro-1H-pyrazol-1-yl)-7-((2-(trimethylsilyl)ethoxy)methyl)-7H-pyrrolo[2,3-d]pyrimidine

2,4-dichloro-7-((2-(trimethylsilyl)ethoxy)methyl)-7H-pyrrolo[2,3-d]pyrimidine (1 g, 3.93 mmol) obtained in STEP 1 of Example 69 above, 5-phenyl-4,5-dihydro-1H-pyrazole (2 mL, 8.21 mmol) prepared according to Preparation Example 1 above and DIPEA (2.2 mL, 12.6 mmol) were dissolved in DMSO (11 mL), and then reacted at 60°C for 4 hours. After completion of the reaction, the temperature was lowered to room temperature, and the reaction mixture was extracted with ethyl acetate and water to recover the organic layer. The obtained organic layer was dried over sodium sulfate, and then concentrated under reduced pressure, and purified by medium pressure liquid chromatography (n-hexane/ethyl acetate) to obtain the desired compound 2-chloro-4-(5-phenyl-4,5-dihydro-1H-pyrazol-1-yl)-7-((2-(trimethylsilyl)ethoxy)methyl)-7H-pyrrolo[2,3-d]pyrimidine (0.8 g, 60% yield).

MS (m/z): 428.2 $[M+1]^+$

STEP 3: Preparation of N-(3-methoxy-4-(4-(4-methylpiperazin-1-yl)piperidin-1-yl)phenyl)-4-(5-phenyl-4,5-dihydro-1H-pyrazol-1-yl)-7-((2-(trimethylsilyl)ethoxy)methyl)-7H-pyrrolo[2,3-d]pyrimidin-2-amine

2-chloro-4-(5-phenyl-4,5-dihydro-1H-pyrazol-1-yl)-7-((2-(trimethylsilyl)ethoxy)methyl)-7H-pyrrolo[2,3-d]pyrimidine (200 mg, 0.47 mmol) obtained in STEP 2 of Example 69 above, 3-methoxy-4-(4-(4-methylpiperazin-1-yl)piperidine-1-yl)aniline (142 mg, 0.47 mmol) and $K_2CO_3$ (194 mg, 1.4 mmol) were dissolved in sec-BuOH (1.5 mL), and then sonicated under nitrogen for 5 minutes to remove gas. $Pd_2(dba)_3$ (43 mg, 0.047 mmol) and Xphos (44 mg, 0.093 mmol) were added to the reaction mixture, and then reacted at 100°C for 3 hours. After completion of the reaction, the temperature was lowered to room temperature, and the reactant was filtered through diatomaceous earth. The obtained filtrate was concentrated under reduced pressure and then purified by medium pressure chromatography (MeOH/DCM 0~10%) to obtain the desired compound N-(3-methoxy-4-(4-(4-methylpiperazin-1-yl)piperidin-1-yl)phenyl)-4-(5-phenyl-4,5-dihydro-1H-pyrazol-1-yl)-7H-pyrrolo[2,3- d] pyrimidin-2-amine (220 mg, 67% yield) in a solid state.

MS (m/z): 696.5 $[M+1]^+$

STEP 4: Preparation of N-(3-Methoxy-4-(4-(4-methylpiperazin-1-yl)piperidin-1-yl)phenyl)-4-(5-phenyl-4,5-dihydro-1H-pyrazol-1-yl)-7H-pyrrolo[2,3-d]pyrimidin-2-amine

N-(3-methoxy-4-(4-(4-methylpiperazin-1-yl)piperidin-1-yl)phenyl)-4-(5-phenyl-4,5-dihydro-1H-pyrazol-1-yl)-7H-pyrrolo[2,3-d]pyrimidin-2-amine (220 mg, 0.32 mmol) obtained in STEP 3 of Example 69 above was dissolved in DCM (1.5 ml), and then TFA (73 μl, 0.95 mmol) was added thereto and stirred at room temperature for 6 hours. After the reaction mixture was concentrated under reduced pressure, and then dissolved in 1,4-dioxane (1.5 ml), aqueous ammonia (123 μl, 3.16 mmol) was slowly added thereto dropwise. The reaction mixture was stirred at 60° C for 1 hour, and then concentrated under reduced pressure, and purified by Prep-150 LC System to obtain the desired compound N-(3-methoxy-4-(4-(4-methylpiperazin-1-yl)piperidin-1-yl)phenyl)-4-(5-phenyl-4,5-dihydro-1H-pyrazol-1-yl)-7H-pyrrolo[2,3-d]pyrimidine-2-amine (110 mg, 50% yield) in a solid state.

MS (m/z): 566.4 $[M+1]^+$

$^1$H NMR (400 MHz, DMSO-$d_6$) δ 11.07 (s, 1H), 8.29 (s, 1H), 7.32 - 7.21 (m, 5H), 7.13 (d, *J* = 7.2 Hz, 2H), 6.86 (dd, *J* = 3.4, 2.2 Hz, 1H), 6.68 (dd, *J* = 3.4, 2.1 Hz, 1H), 6.62 (d, *J* = 9.0 Hz, 1H), 5.75 (dd, *J* = 11.9, 4.0 Hz, 1H), 3.72 (s, 3H), 3.63 - 3.52 (m, 1H), 3.32 (s, 8H), 3.26 (d, *J*= 11.6 Hz, 3H), 2.79 - 2.71 (m, 1H), 2.45 (d, *J*= 11.8 Hz, 3H), 2.16 (s, 4H), 1.80 (d, *J* = 12.0 Hz, 2H), 1.53 (q, *J* = 10.9 Hz, 3H)

**<Example 129> Preparation of (*R*)-*N*-(3-methoxy-4-(4-(4-methylpiperazin-1-yt)piperidin-1-yt)phenyl)-4-(3-phenylisooxazolidin-2-yl)-7*H*-pyrrolo[2,3-*d*]pyrimidin-2-amine**

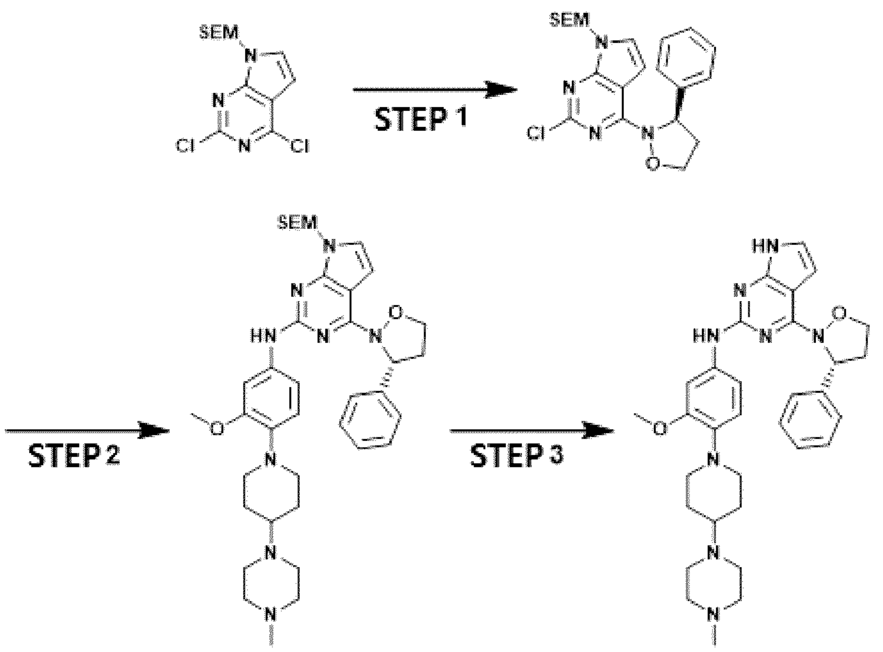


STEP 1: Preparation of (R)-2-(2-chloro-7-((2-(trimethylsilyl)ethoxy)methyl)-7H-pyrrolo[2,3-d]pyrimidin-4-yl)-3-phenylisooxazolidine

2,4-dichloro-7-((2-(trimethylsilyl)ethoxy)methyl)-7H-pyrrolo[2,3-d]pyrimidine (1 g, 3.14 mmol), (R)-3-phenylisoxazolidine (0.5 g, 3.46 mmol) prepared in Preparation Example 3 above and DIPEA (1.1 ml, 6.28 mmol) were dissolved in DMSO (2 ml) and then stirred at 60°C for 2 hours. After completion of the reaction, the temperature was lowered to room temperature, and the reaction mixture was extracted with ethyl acetate and water to recover the organic layer. The obtained organic layer was dried over sodium sulfate, and then concentrated under reduced pressure, and purified by medium pressure liquid chromatography (EA/Hex 0~50%) to obtain the desired compound (R)-2-(2-chloro-7-((2-(trimethylsilyl)ethoxy)methyl)-7H-pyrrolo[2,3-d]pyrimidin-4-yl)-3-phenylisoxazolidine (1.3 g, 90% yield).

MS (m/z): 431.16 $[M+1]^+$

STEP 2: Preparation of (R)-N-(3-methoxy-4-(4-(4-methylpiperazin-1-yl)piperidin-1-yl)phenyl)-4-(3-phenylisooxazolidin-2-yl)-7-((2-(trimethylsilyl)ethoxy)methyl)-7H-pyrrolo[2,3-d]pyrimidin-2-amine

(R)-2-(2-chloro-7-((2-(trimethylsilyl)ethoxy)methyl)-7H-pyrrolo[2,3-d]pyrimidine-4-yl)-3-phenylisoxazolidine (200 mg, 0.46 mmol) obtained in STEP 1 of Example 129 above, and 3-methoxy-4-(4-(4-methylpiperazin-1-yl)piperidin-1-yl)aniline (141 mg, 0.46 mmol) and $K_2CO_3$ (160 mg, 1.16 mmol) were dissolved in sec-BuOH (1.5 mL), and then sonicated under nitrogen for 5 minutes to remove gas. $Pd_2(dba)_3$ (42 mg, 0.046 mmol) and Xphos (44 mg, 0.093 mmol) were added to the reaction mixture, and then reacted at 100°C for 3 hours. After completion of the reaction, the temperature was lowered to room temperature, and the reactant was filtered through diatomaceous earth. The obtained filtrate was concentrated under reduced pressure, and then purified by medium pressure liquid chromatography (MeOH/DCM 0~10%) to obtain the desired compound (R)-N-(3-methoxy-4-(4-(4-methylpiperazin-1-yl)piperidin-1-yl)phenyl)-4-(3-phenylisooxazolidin-2-yl)-7-((2-(trimethylsilyl)ethoxy)methyl)-7H-pyrrolo[2,3-d]pyrimidin-2-amine (162 mg, 50% yield) in a solid state.

MS (m/z): 699.4 $[M+1]^+$

STEP 3: Preparation of (R)-N-(3-methoxy-4-(4-(4-methylpiperazin-1-yl)piperidin-1-yl)phenyl)-4-(3-phenylisooxazolidin-2-yl)-7H-pyrrolo[2,3-d]pyrimidin-2-amine

(R)-N-(3-methoxy-4-(4-(4-methylpiperazin-1-yl)piperidin-1-yl)phenyl)-4-(3-phenylisooxazolidin-2-yl)-7-((2-(trimethylsilyl)ethoxy)methyl)-7H-pyrrolo[2,3-d]pyrimidin-2-amine (162 mg, 0.32 mmol) obtained in STEP 2 of Example 129 above was dissolved in DCM (1.5 ml), and then TFA (89 μl, 1.16 mmol) was added thereto, and the mixture was stirred at room temperature for 6 hours. After the reaction mixture was concentrated under reduced pressure and then dissolved in 1,4-dioxane (1.5 ml), aqueous ammonia (90 μl, 2.32 mmol) was slowly added thereto dropwise. The reaction mixture was stirred at 60°C for 1 hour, and then concentrated under reduced pressure, and purified by Prep-150 LC System to obtain the desired compound (R)-N-(3-methoxy-4-(4-(4-methylpiperazin-1-yl)piperidin-1-yl)phenyl)-4-(3-phenylisoxazolidin-2-yl)-7H-pyrrolo[2,3-d]pyrimidin-2-amine (83 mg, 63% yield) in a solid state.

MS (m/z): 569.3 $[M+1]^+$

$^1H$ NMR (400 MHz, DMSO-$d_6$) δ 11.27 (d, *J*= 2.3 Hz, 1H), 8.69 (s, 1H), 7.51 - 7.47 (m, 2H), 7.43 - 7.36 (m, 3H), 7.31 - 7.26 (m, 1H), 7.21 (dd, *J*= 8.6, 2.3 Hz, 1H), 6.96 (dd, *J* = 3.5, 2.2 Hz, 1H), 6.54 (dd, *J*= 3.5, 2.0 Hz, 1H), 4.24 (td, *J*= 7.9, 3.6 Hz, 1H), 3.65 (s, 3H), 3.44 - 3.19 (m, 10H), 2.84 (qq, *J* = 8.2, 3.7 Hz, 1H), 2.48 - 2.39 (m, 4H), 2.30 (ddt, *J* = 11.9, 7.9, 4.3 Hz, 3H), 2.24 (s, 3H), 1.81 (d, *J* = 12.0 Hz, 2H), 1.54 (q, *J* = 11.6 Hz, 2H)

All the example compounds (Examples 1 to 129) of the present invention were prepared in a similar manner to Examples 8, 57, 69 and 129, and the chemical structures, compound names, and NMRs, LC-MS analysis results of the respective example compounds were summarized and shown in Table 1 below.

[Table 1]

| Example compounds | structure | Compound name | $^1H$ NMR | LC-MS (m/z) |
|---|---|---|---|---|
| **1** | 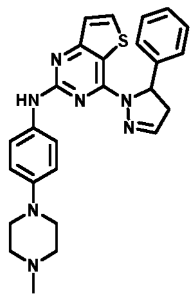 | *N*-(4-(4-methy lpiperazin-1-yl)phenyl)-4-(5-phenyl-4,5-dihydro-1*H*-pyrazol-1-yl) thieno[3,2-*d*]pyrimidin-2-amine | $^1H$ NMR (400 MHz, DMSO) δ 10.09 (s, 1H), 8.35 (d, *J* = 5.5 Hz, 1H), 7.76 (d, *J* = 1.8 Hz, 1H), 7.35 (dd, *J* = 8.2, 6.7 Hz, 2H), 7.30 - 7.24 (m, 2H), 7.18 - 7.09 (m, 4H), 6.92 - 6.87 (m, 2H), 5.71 (dd, *J* = 11.4, 3.9 Hz, 1H), 3.80 (d, *J* = 13.6 Hz, 2H), 3.76 - 3.69 (m, 1H), 3.57 (d, *J* = 12.0 Hz, 2H), 3.20 (s, 2H), 2.95 (dd, *J* = 4.0, 1.9 Hz, 2H), 2.90 (s, 3H) | 470.2 $[M+H]^+$ |

(continued)

| Example compounds | structure | Compound name | $^1H$ NMR | LC-MS (m/z) |
|---|---|---|---|---|
| **2** | 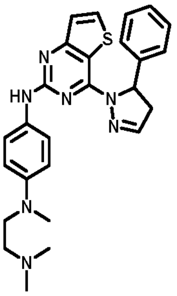 | *N*-(2-(dimethylamino)ethyl)-*N*-methyl-*N*-(4-(5-phenyl-4,5-dihydro-1*H*-pyrazol-1-yl)thieno[3,2-*d*]pyrimidin-2-yl)benzene-1,4-diamine | $^1H$ NMR (400 MHz, DMSO-$d_6$) δ 8.66 (s, 1H), 8.05 (d, *J* = 5.5 Hz, 1H), 7.35 (dd, *J* = 16.7, 8.8 Hz, 3H), 7.25 (dd, *J* = 13.2, 7.6 Hz, 5H), 7.09 (d, *J* = 5.5 Hz, 1H), 6.61 (d, *J* = 9.0 Hz, 2H), 5.73 (dd, *J*= 11.9, 4.2 Hz, 1H), 3.66 (ddd, *J* = 18.5, 11.9, 1.3 Hz, 1H), 3.49 (t, *J* = 7.1 Hz, 2H), 3.02 (t, *J* = 7.3 Hz, 2H), 2.84 (s, 3H), 2.79 (d, *J* = 1.6 Hz, 1H), 2.70 (s, 6H) | 472.3 [M+H]+ |
| **3** | 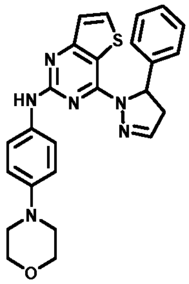 | *N*-(4-morpholinophenyl)-4-(5-phenyl-4,5-dihydro-1*H*-pyrazol-1-yl)thieno[3,2-*d*]pyrimidin-2-amine | $^1H$ NMR (400 MHz, DMSO-$d_6$) δ 8.75 (s, 1H), 8.05 (d, *J*= 5.5 Hz, 1H), 7.36 (dd, *J* = 14.3, 6.4 Hz, 3H), 7.29 - 7.20 (m, 5H), 7.11 (d, *J* = 5.5 Hz, 1H), 6.75 - 6.63 (m, 3H), 6.52 - 6.46 (m, 1H), 5.74 (dd, *J* = 11.9, 4.2 Hz, 1H), 3.78-3.71 (m, 4H), 3.71 - 3.67 (m, 4H), 3.66 (dd, *J* = 6.7, 1.6 Hz, 1H), 3.00 (t, *J* = 4.8 Hz, 4H), 2.89 - 2.85 (m, 4H), 2.80 (ddd, *J* = 18.6, 4.2, 1.7 Hz, 1H) | 457.2 [M+H]+ |
| **4** | 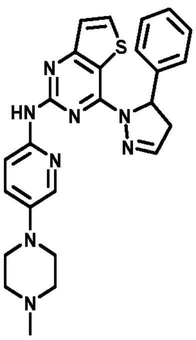 | *N*-(5-(4-methy lpiperazin-1-yl)pyridin-2-yl)-4-(5-phenyl-4,5-dihydro-1*H*-pyrazol-1-yl)thieno[3,2-*d*]pyrimidin-2-amine | $^1H$ NMR (400 MHz, DMSO-$d_6$) δ 8.71 (s, 1H), 8.10 (d, *J* = 5.5 Hz, 1H), 7.90 (d, *J* = 2.8 Hz, 1H), 7.62 (d, *J* = 9.1 Hz, 1H), 7.42 (s, 1H), 7.34 (d, *J* = 7.4 Hz, 1H), 7.27 (d, *J* = 7.4 Hz, 2H), 7.18 (d, *J* = 5.5 Hz, 1H), 5.76 (s, 1H), 3.67 (dd, *J* = 17.6, 12.2 Hz, 1H), 3.14 - 3.01 (m, 4H), 2.88 - 2.77 (m, 1H), 2.49 - 2.45 (m, 4H), 2.23 (s, 3H) | 471.4 [M+H]+ |
| **5** | 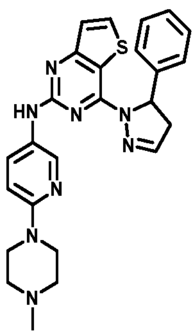 | *N*-(6-(*4*-methy lpiperazin-1-yl)pyridin-3-yl)-4-(5-phenyl-4,5-dihydro-1*H*-pyrazol-1-yl)thieno[3,2-*d*]pyrimidin-2-amine | $^1H$ NMR (400 MHz, DMSO-$d_6$) δ 8.71 (s, 1H), 8.20 (s, 1H), 8.05 (d, *J* = 5.5 Hz, 1H), 7.56 (d, *J* = 8.0 Hz, 1H), 7.39 (s, 1H), 7.30 (d, *J* = 7.6 Hz, 1H), 7.21 - 7.17 (m, 2H), 7.11 (d, *J*= 5.5 Hz, 1H), 6.62 (d, *J*= 9.1 Hz, 1H), 5.69 (dd, *J* = 11.9, 4.2 Hz, 1H), 3.65 (ddd, *J* = 18.6, 11.9, 1.4 Hz, 1H), 3.40-3.36 (m, 4H), 2.81 (ddd, *J* = 18.6, 4.2, 1.7 Hz, 1H), 2.45 - 2.37 (m, 4H), 2.22 (s, 3H) | 471.3 [M+H]+ |

(continued)

| Example compounds | structure | Compound name | $^1$H NMR | LC-MS (m/z) |
|---|---|---|---|---|
| **6** | 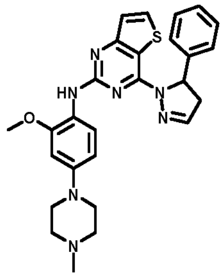 | *N*-(2-methoxy-4-(4-methylpiperazin-1-yl)phenyl)-4-(5-phenyl-4,5-dihydro-1*H*-pyrazol-1-yl)thieno[3,2-*d*]pyrimidin-2-amine | $^1$H NMR (400 MHz, DMSO-$d_6$) δ 8.05 (d, *J* = 5.5 Hz, 1H), 7.60 (d, *J*= 8.2 Hz, 1H), 7.39 (s, 1H), 7.32 (t, *J* = 7.4 Hz, 2H), 7.24 (dd, *J* = 7.8, 1.6 Hz, 1H), 7.20 (dd, *J*= 7.0, 5.8 Hz, 3H), 7.11 (d, *J* = 5.5 Hz, 1H), 6.57 (d, *J* = 2.5 Hz, 1H), 6.34 (dd, *J* = 8.8, 2.5 Hz, 1H), 5.68 (dd, *J* = 11.9, 4.5 Hz, 1H), 3.78 (s, 3H), 3.64 (ddd, *J* = 18.6, 12.0, 1.4 Hz, 1H), 3.11 - 3.04 (m, 4H), 2.82 (ddd, *J* = 18.7, 4.6, 1.7 Hz, 1H), 2.49 - 2.44 (m, 4H), 2.23 (s, 3H) | 500.3 [M+H] + |
| 7 | 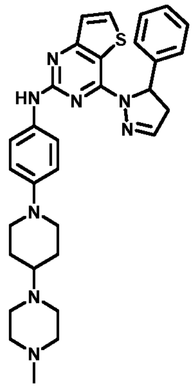 | *N*-(4-(4-(4-methylpiperazin-1-yl)piperidin-1-yl)phenyl)-4-(5-phenyl-4,5-dihydro-1*H*-pyrazol-1-yl)thieno[3,2-*d*]pyrimidin-2-amine | $^1$H NMR (400 MHz, DMSO-$d_6$) δ 10.15 (s, 1H), 8.32 (d, *J* = 5.5 Hz, 1H), 7.71 (s, 1H), 7.34 (d, *J*= 7.6 Hz, 2H), 7.24 (d, *J*= 5.5 Hz, 1H), 7.17 (d, *J* = 7.5 Hz, 2H), 6.94 (d, *J* = 8.5 Hz, 2H), 5.72 (dd, *J*= 11.5, 3.9 Hz, 1H), 3.73 (dd, *J* = 17.8, 11.8 Hz, 9H), 2.96 - 2.87 (m, 2H), 2.81 (s, 4H), 2.07 (d, *J* = 11.1 Hz, 2H), 1.71 (dd, *J* = 21.8, 11.0 Hz, 2H) | 553.4 [M+H] + |
| **8** | 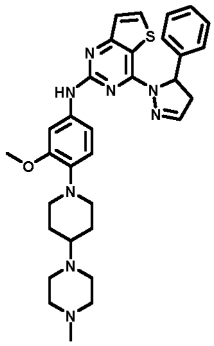 | *N*-(3-methoxy-4-(4-(4-methylpiperazin-1-yl) piperidin-1-yl)phenyl)-4-(5-phenyl-4,5-dihydro-1*H*-pyrazol-1-yl)thieno[3,2-d]pyrimidin-2-amine | $^1$H NMR (400 MHz, DMSO-$d_6$) δ 8.70 (s, 2H), 8.06 (d, *J* = 5.5 Hz, 2H), 7.40 (s, 2H), 7.36-7.17 (m, 11H), 7.12 (dd, *J* = 11.4, 5.8 Hz, 6H), 5.75 (dd, *J* = 11.7, 3.9 Hz, 2H), 3.74 - 3.65 (m, 9H), 3.31 (t, *J* = 10.2 Hz, 6H), 2.83 (dd, *J* = 18.6, 2.3 Hz, 3H), 2.61 (d, J= 51.9 Hz, 15H), 2.37 (s, 6H), 1.84 (d, *J* = 10.9 Hz, 4H), 1.56 (dd, *J*= 20.5, 11.0 Hz, 4H). | 583.4 [M+1] + |
| **9** | 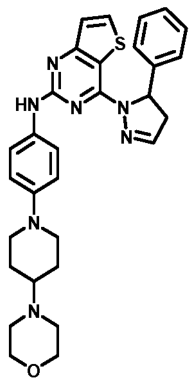 | *N*-(4-(4-morpholinopiperidin-1-yl)phenyl)-4-(5-phenyl-4,5-dihydro-1*H*-pyrazol-1-yl) thieno[3,2-*d*]pyrimidin-2-amine | $^1$H NMR (400 MHz, DMSO-$d_6$) δ 8.71 (s, 1H), 8.05 (d, *J* = 5.5 Hz, 1H), 7.36 (d, *J* = 17.3 Hz, 2H), 7.24 (s, 3H), 7.10 (d, *J* = *5.5* Hz, 1H), 6.72 (d, *J* = 8.9 Hz, 2H), 5.74 (dd, *J* = 11.9, 4.0 Hz, 1H), 3.66 (*dd, J* = 18.1, 12.6 Hz, 1H), 3.58 (s, 6H), 2.80 (dd, *J* = 18.6, 2.6 Hz, 1H), 2.56 (t, *J* = 12.0 Hz, 2H), 2.47 (d, *J* = 4.4 Hz, 5H), 2.23 (t, *J* = 11.1 Hz, 1H), 1.84 (t, *J* = 15.1 Hz, 3H), 1.56 - 1.43 (m, 3H) | 540.4 [M+H] + |

(continued)

| Example compounds | structure | Compound name | $^1$H NMR | LC-MS (m/z) |
|---|---|---|---|---|
| **10** | 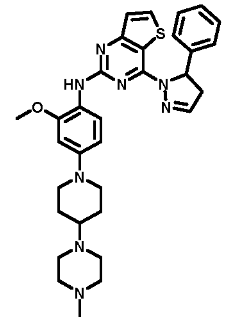 | *N*-(2-methoxy-4-(4-(4-methylpiperazin-1-yl) piperidin-1-yl)phenyl)-4-(5-phenyl-4,5-dihydro-1*H*-pyrazol-1-yl)thieno[3,2-*d*]pyrimidin-2-amine | $^1$H NMR (400 MHz, DMSO-$d_6$) δ 8.05 (d, *J* = 5.5 Hz, 1H), 7.60 (d, *J*= 8.0 Hz, 1H), 7.39 (s, 1H), 7.31 (d, *J*= 7.6 Hz, 1H), 7.18 (d, *J* = 7.6 Hz, 3H), 7.11 (d, *J* = 5.5 Hz, 1H), 6.56 (d, *J* = 2.4 Hz, 1H), 6.34 (dd, *J* = 8.8, 2.3 Hz, 1H), 5.67 (dd, *J* = 11.9, 4.5 Hz, 1H), 3.77 (s, 3H), 3.62 (dd, *J*= 12.6, 4.1 Hz, 2H), 2.85 - 2.76 (m, 1H), 2.60 (t, *J* = 11.8 Hz, 2H), 2.50 (s, 4H), 2.28 (dd, *J*= 11.4, 3.6 Hz, 4H), 2.13 (s, 3H), 1.83 (d, *J* = 11.9 Hz, 2H), 1.51 (dt, *J* = 11.6, 8.9 Hz, 2H) | 583.4 [M+H]+ |
| **11** | 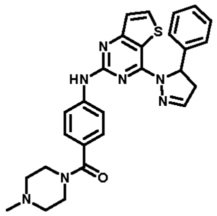 | (4-methylpiperazin-1-yl) (4-((4-(5-phenyl-4,5-dihydro-1*H*-pyrazol-1-yl) thieno[3,2-*d*]pyrimidin-2-yl) amino)phenyl)m ethanone | $^1$H NMR (400 MHz, DMSO-$d_6$) δ 9.32 (s, 1H), 8.13 (d, *J* = 5.5 Hz, 1H), 7.43 (d, *J* = 1.4 Hz, 2H), 7.34 (d, *J* = 7.1 Hz, 2H), 7.26 (d, *J*= 7.5 Hz, 3H), 7.18 (d, *J*= 5.5 Hz, 1H), 7.13 (d, *J* = 8.6 Hz, 2H), 5.82 (dd, *J* = 11.9, 4.2 Hz, 1H), 3.69 (ddd, *J* = 18.5, 11.9, 1.4 Hz, 1H), 3.48 (s, 4H), 2.82 (ddd, *J* = 18.6, 4.2, 1.7 Hz, 1H), 2.33 (s, 4H), 2.22 (s, 3H) | 498.3 [M+H]+ |
| **12** | 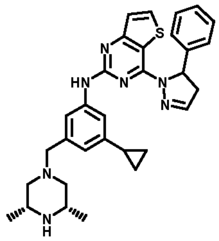 | *N*-(3-cyclopropyl-5-(((3*R*, 5*S*)-3,5-dimethylpiperazin-1-yl)methyl)phenyl)-4-(5-phenyl-4,5-dihydro-1*H*-pyrazol-1-yl)thieno[3,2-*d*] pyrimidin-2-amine | $^1$H NMR (400 MHz, DMSO-$d_6$) δ 9.87 (s, 1H), 8.26 (d, *J* = 5.5 Hz, 1H), 7.65 (s, 1H), 7.35-7.14 (m, 9H), 6.76 (s, 1H), 5.82 (dd, *J* = 11.3, 3.4 Hz, 1H), 3.76 (dd, *J* = 18.1, 12.0 Hz, 3H), 3.42 (s, 2H), 3.13 (s, 2H), 3.02 - 2.91 (m, 1H), 2.40 (s, 2H), 1.88 - 1.78 (m, 1H), 1.22 (d, *J* = 5.0 Hz, 6H), 1.21 - 1.15 (m, 2H), 0.97 (dd, *J* = 8.3, 2.1 Hz, 2H), 0.68 (dd, *J* = 10.6, 4.9 Hz, 2H) | 538.4 [M+H]+ |
| **13** | 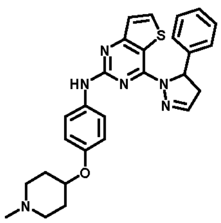 | *N*-(4-((1-methy lpiperidin-4-yl)oxy)phenyl)-4-(5-phenyl-4,5-dihydro-1*H*-pyrazol-1-yl) thieno[3,2-*d*]pyrimidin-2-amine | $^1$H NMR (400 MHz, DMSO-$d_6$) δ 8.82 (s, 1H), 8.07 (d, *J* = 5.5 Hz, 1H), 7.39 (s, 1H), 7.34 (t, *J* = 7.5 Hz, 2H), 7.25 (dd, *J* = 18.7, 8.7 Hz, 5H), 7.11 (d, *J* = 5.5 Hz, 1H), 6.71 (d, *J* = 9.0 Hz, 2H), 5.75 (dd, *J* = 11.9, 4.1 Hz, 1H), 4.27 (s, 1H), 3.67 (dd, *J* = 17.2, 12.1 Hz, 1H), 2.81 (dd, *J* = 16.9, 4.2 Hz, 3H), 2.34 (s, 3H), 1.94 (s, 2H), 1.69 (s, 2H) | 485.3 [M+H]+ |

(continued)

| Example compounds | structure | Compound name | $^1$H NMR | LC-MS (m/z) |
|---|---|---|---|---|
| **14** | 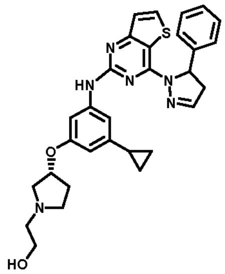 | 2-((3R)-3-(3-cyclopropyl-5-((4-(5-phenyl-4,5-dihydro-1*H*-pyrazol-1-yl)thieno[3,2-*d*]pyrimidin-2-yl)amino) phenoxy) pyrrolidin-1-yl) ethan-1-ol | $^1$H NMR (400 MHz, DMSO-$d_6$) δ 8.21 (d, *J* = 5.1 Hz, 2H), 7.59 (d, *J* = 6.0 Hz, 2H), 7.30 -724 (m, 4H), 7.21 (t, *J* = 5.5 Hz, 8H), 7.10 (s, 2H), 6.99 (d, *J* = 7.3 Hz, 2H), 6.23 (d, *J* = 10.0 Hz, 2H), 5.81 - 5.74 (m, 2H), 5.07 (d, *J* = 19.1 Hz, 3H), 4.00 (s, 2H), 3.75 (d, *J* = 18.5 Hz, 10H), 3.50 (s, 2H), 3.31 (d, *J* = 22.0 Hz, 8H), 2.94 (d, *J* = 18.8 Hz, 2H), 2.65 - 2.52 (m, 2H), 2.27 (dd, *J* = 21.2, 15.8 Hz, 2H), 1.88 - 1.76 (m, 2H), 0.95 (d, *J* = 8.5 Hz, 4H), 0.66 (dd, *J* = 8.6, 4.2 Hz, 4H). | 541.3 [M+H]+ |
| **15** | 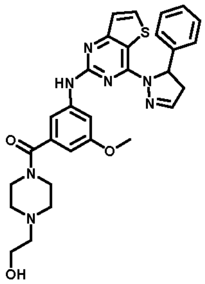 | (4-(2-hydroxyethyl)piperazin-1-yl)(3-methoxy-5-((4-(5-phenyl-4,5-dihydro-1*H*-pyrazol-1-yl)thieno[3,2-*d*]pyrimidin-2-yl)amino) phenyl)m ethanone | $^1$H NMR (400 MHz, DMSO-$d_6$) δ 9.08 (s, 1H), 8.11 (d, *J* = 5.5 Hz, 1H), 7.48 (s, 1H), 7.44 (s, 1H), 7.30 (d, *J*= 6.8 Hz, 1H), 7.28 - 7.19 (m, 4H), 7.18 (d, *J* = 5.5 Hz, 1H), 6.42 (s, 1H), 5.82 - 5.72 (m, 1H), 4.44 (s, 1H), 3.76 (s, 3H), 3.72 - 3.65 (m, 1H), 3.52 (d, *J* = 4.8 Hz, 2H), 3.32 (s, 5H), 2.86 (d, *J*= 20.0 Hz, 1H), 2.44 (s, 4H). | 558.4 [M+H]+ |
| **16** | 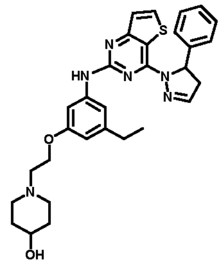 | 1-(2-(3-ethyl-5-((4-(5-phenyl-4,5-dihydro-1*H*-pyrazol-1-yl)thieno[3,2-*d*] pyrimidin-2-yl)amino) phenoxy) ethyl)piperidin-4-ol | $^1$H NMR (400 MHz, DMSO-$d_6$) δ 8.82 (s, 1H), 8.09 (d, *J* = 5.5 Hz, 1H), 7.42 (s, 1H), 7.32 - 7.17 (m, 6H), 7.15 (d, *J* = 5.5 Hz, 1H), 7.01 (s, 1H), 6.32 (s, 1H), 5.86 - 5.72 (m, 1H), 4.55 (s, 1H), 4.02 (s, 2H), 3.70 (dd, *J*= 18.1, 12.2 Hz, 1H), 3.32 (s, 3H), 2.77 (d, *J* = 59.2 Hz, 4H), 2.16 (s, 2H), 1.71 (s, 2H), 1.44 (t, *J* = 20.3 Hz, 2H), 1.14 (t, *J* = 7.6 Hz, 3H). | 543.3 [M+H]+ |
| **17** | 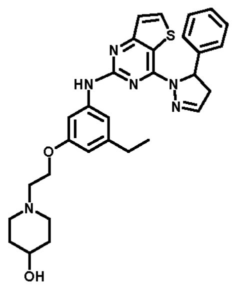 | *N*-(3-((R)-3-(dimethylamino) pyr rolidin-1-yl)phenyl)-4-(5-phenyl-4,5-dihydro-1*H*-pyrazol-1-yl)thieno[3,2-*d*]pyrimidin-2-amine | $^1$H NMR (400 MHz, DMSO-$d_6$) δ 8.64 (s, 1H), 8.07 (d, *J* = 5.5 Hz, 1H), 7.40 (s, 1H), 7.27 (dq, *J* = 14.8, 7.6 Hz, 5H), 7.13 (d, *J* = 5.5 Hz, 1H), 6.96 (d, *J* = 5.1 Hz, 1H), 6.89 (t, *J*= 8.0 Hz, 1H), 6.70 (d, *J*= 5.3 Hz, 1H), 5.77 (dd, *J* = 11.7, 3.9 Hz, 1H), 3.67 (dd, *J* = 18.2, 12.5 Hz, 1H), 3.22 - 3.08 (m, 1H), 2.98 (dd, *J* = 14.8, 6.8 Hz, 1H), 2.83 (d, *J* = 18.8 Hz, 2H), 2.22 (s, 3H), 2.16 - 2.12 (m, 1H), 1.85 - 1.73 (m, 1H), 0.83 (dd, *J*= 14.1, 6.7 Hz, 1H) | 484.3 [M+H]+ |

(continued)

| Example compounds | structure | Compound name | $^1$H NMR | LC-MS (m/z) |
|---|---|---|---|---|
| **18** | 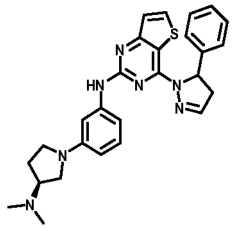 | *N*-(3-((S)-3-(dimethylamino) pyr rolidin-1-yl)phenyl)-4-(5-phenyl-4,5-dihydro-1*H*-pyrazol-1-yl)thieno[3,2-*d*]pyrimidin-2-amine | $^1$H NMR (400 MHz, DMSO-$d_6$) δ 8.64 (s, 1H), 8.07 (d, *J* = 5.5 Hz, 1H), 7.40 (s, 1H), 7.32 (t, *J* = 7.5 Hz, 2H), 7.23 (d, *J* = 7.4 Hz, 3H), 7.13 (d, *J* = 5.5 Hz, 1H), 6.96 (d, *J* = 5.5 Hz, 1H), 6.89 (t, *J* = 8.0 Hz, 1H), 6.70 (d, *J* = 5.6 Hz, 1H), 6.08 (d, *J* = 7.6 Hz, 1H), 5.77 (s, 1H), 3.67 (dd, *J* = 18.6, 11.9 Hz, 1H), 3.37 (d, *J* = 9.2 Hz, 2H), 3.22 - 3.10 (m, 2H), 2.98 (dt, *J* = 16.6, 7.9 Hz, 2H), 2.22 (s, 6H), 2.14 *(d, J*= 6.1 Hz, 1H), 1.83 - 1.76 (m, 1H). | 484.3 [M+H]+ |
| **19** | 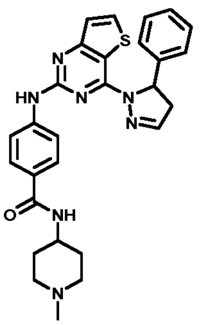 | *N*-(1-methylpiperidin-4-yl)-4-((4-(5-phenyl-4,5-dihydro-1*H*-pyrazol-1-yl)thieno[3,2-*d*]pyrimidin-2-yl)amino) benzamid e | $^1$H NMR (400 MHz, DMSO-$d_6$) δ 9.30 (s, 1H), 8.13 (d, *J* = 5.5 Hz, 1H), 7.95 (d, *J* = 7.8 Hz, 1H), 7.64 (d, *J* = 8.8 Hz, 2H), 7.51 (d, *J*= 8.8 Hz, 2H), 7.43 (s, 1H), 7.38 - 7.30 (m, 2H), 7.29 - 7.21 (m, 3H), 7.19 (d, *J* = 5.5 Hz, 1H), 5.81 (dd, *J* = 11.8, 4.1 Hz, 1H), 3.78 - 3.64 (m, 2H), 2.88 - 2.73 (m, 3H), 2.17 (s, 3H), 1.94 (t, *J* = 10.8 Hz, 2H), 1.76 (d, *J* = 10.7 Hz, 2H), 1.65 - 1.53 (m, 2H) | 512.3 [M+H]+ |
| **20** | 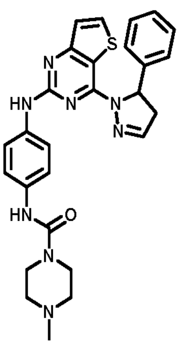 | 4-methyl-*N*-(4-((4-(5-phenyl-4,5-dihydro-1*H*-pyrazol-1-yl) thieno[3,2-*d*]pyrimidin-2-yl) amino)phenyl)pi perazine-1-carboxamide | $^1$H NMR (400 MHz, DMSO-$d_6$) δ 9.64 (s, 1H), 8.92 (s, 1H), 8.07 (d, *J* = 5.5 Hz, 1H), 7.40 - 7.33 (m, 5H), 7.29 - 7.21 (m, 5H), 7.13 (d, *J* = 5.5 Hz, 1H), 5.77 (dd, *J* = 11.8, 4.2 Hz, 1H), 3.67 (ddd, *J* = 18.5, 11.9, 1.3 Hz, 1H), 2.81 (ddd, *J* = 5.9, 5.4, 1.9 Hz, 3H), 2.29 - 2.20 (m, 1H), 2.17 (s, 3H), 1.91 - 1.83 (m, 2H), 1.77 - 1.63 (m, 4H) | 512.3 [M+H]+ |
| **21** | 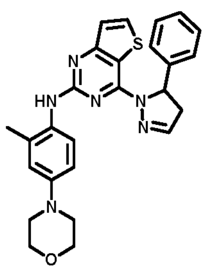 | *N*-(2-methyl-4-morpholinophenyl)-4-(5-phenyl-4,5-dihydro-1*H*-pyrazol-1-yl)thieno[3,2-*d*] pyrimidin-2-amine | $^1$H NMR (400 MHz, DMSO-$d_6$) δ 8.85 (s, 1H), 8.18 (d, *J* = 5.5 Hz, 1H), 7.60 (s, 1H), 7.24 (s, 2H), 7.16 (d, *J* = 5.5 Hz, 1H), 6.97 (d, *J* = 8.5 Hz, 2H), 6.85 (s, 1H), 6.72 (d, *J* = 8.5 Hz, 1H), 5.46 (d, *J*= 9.8 Hz, 1H), 3.81 - 3.72 (m, 4H), 3.70 - 3.60 (m, 1H), 3.13 (d, *J* = 4.3 Hz, 4H), 2.95 - 2.85 (m, 1H), 2.02 (s, 3H) | 471.3 [M+H]+ |

(continued)

| Example compounds | structure | Compound name | $^1$H NMR | LC-MS (m/z) |
|---|---|---|---|---|
| **22** | 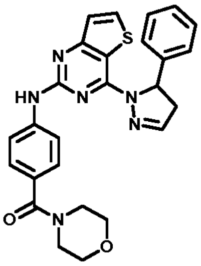 | morpholino(4-((4-(5-phenyl-4,5-dihydro-1*H*-pyrazol-1-yl) thieno[3,2-*d*]pyrimidin-2-yl) amino)phenyl)m ethanone | $^1$H NMR (400 MHz, DMSO-$d_6$) δ 9.33 (s, 1H), 8.13 (d, *J* = 5.5 Hz, 1H), 7.48 - 7.41 (m, 3H), 7.35 (d, *J* = 7.1 Hz, 2H), 7.26 (d, *J* = 7.6 Hz, 3H), 7.17 (dd, *J* = 7.1, 4.5 Hz, 3H), 5.82 (dd, *J* = 11.9, 4.2 Hz, 1H), 3.74 - 3.67 (m, 1H), 3.62 (d, *J* = 4.4 Hz, 4H), 3.49 (dd, *J* = 9.3, 4.4 Hz, 4H), 2.82 (ddd, *J* = 18.6, 4.2, 1.7 Hz, 1H) | 485.2 [M+H]+ |
| **23** | 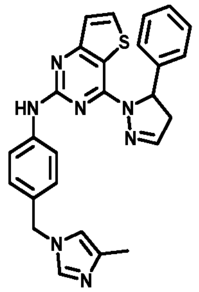 | *N*-(4-((4-methyl-1*H*-imidazol-1-yl)methyl) phenyl)-4-(5-phenyl-4,5-dihydro-1*H*-pyrazol-1-yl) thieno[3,2-*d*]pyrimidin-2-amine | $^1$H NMR (400 MHz, DMSO-$d_6$) δ 9.10 (s, 1H), 8.08 (d, *J* = 5.5 Hz, 1H), 7.54 (s, 2H), 7.41 - 7.36 (m, 3H), 7.24 - 7.18 (m, 3H), 7.03 - 6.98 (m, 2H), 6.96 (d, *J* = 8.3 Hz, 4H), 6.77 (s, 2H), 6.57 - 6.50 (m, 5H), 5.75 (dd, *J* = 11.9, 4.0 Hz, 1H), 4.85 (s, 2H), 3.65 (dd, *J* = 18.1, 12.5 Hz, 1H), 2.79 (dd, *J* = 18.7, 2.4 Hz, 1H), 2.05 (s, 3H) | 466.2 [M+H]+ |
| 24 | 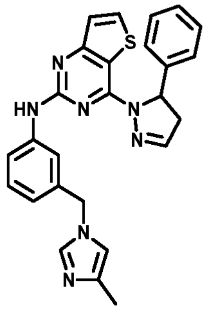 | *N*-(3-((4-methyl-1*H*-imidazol-1-yl)methyl) phenyl)-4-(5-phenyl-4,5-dihydro-1*H*-pyrazol-1-yl) thieno[3,2-*d*]pyrimidin-2-amine | | 466.3 [M+H]+ |
| **25** | 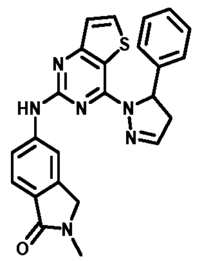 | 2-methyl-5-(( 4-(5-phenyl-4,5-dihydro-1*H*-pyrazol-1-yl) thieno[3,2-*d*]pyrimidin-2-yl) amino)isoindolin -1-one | $^1$H NMR (400 MHz, DMSO-$d_6$) δ 10.70 (s, 1H), 8.41 (d, *J* = 5.5 Hz, 1H), 7.79 (s, 1H), 7.47 (s, 1H), 7.37 (d, *J*= 5.7 Hz, 2H), 7.31 - 7.29 (m, 1H), 7.28 (s, 1H), 7.22 (d, *J* = 7.2 Hz, 2H), 5.90 (dd, *J* = 11.3, 3.6 Hz, 1H), 4.37 (dd, *J* = 51.0, 17.8 Hz, 2H), 3.88 - 3.76 (m, 1H), 3.07 (s, 3H), 2.95 (ddd, *J* = 19.0, 3.5, 1.7 Hz, 1H) | 441.3 [M+H]+ |
| **26** | 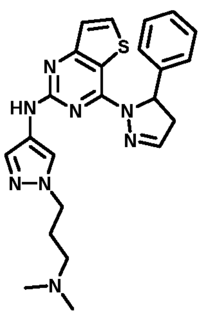 | *N*-(1-(3-(dimethylamino)propyl)-1*H*-pyrazol-4-yl)-4-(5-phenyl-4,5-dihydro-1*H*-pyrazol-1-yl)thieno[3,2-*d*] pyrimidin-2-amine | $^1$H NMR (400 MHz, DMSO-$d_6$) δ 9.71 (s, 1H), 8.30 (s, 1H), 7.70 (s, 1H), 7.40 - 7.20 (m, 7H), 5.89 (s, 1H), 4.08 (s, 2H), 3.76 (dd, *J*=18.6, 11.5 Hz, 1H), 3.03 (s, 2H), 2.94 (d, *J* = 19.0 Hz, 1H), 2.79 (d, *J* = 3.6 Hz, 6H), 2.09 (d, *J* = 4.6 Hz, 2H). | 448.3 [M+H]+ |

(continued)

| Example compounds | structure | Compound name | $^1$H NMR | LC-MS (m/z) |
|---|---|---|---|---|
| **27** | 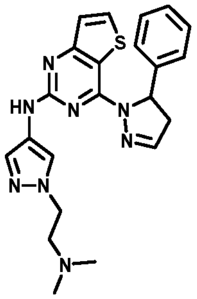 | *N*-(1-(2-(dimethylamino)ethyl)-1*H*-pyrazol-4-yl)-4-(5-phenyl-4,5-dihydro-1*H*-pyrazol-1-yl)thieno[3,2-*d*] pyrimidin-2-amine | $^1$H NMR (400 MHz, DMSO-$d_6$) δ 8.29 (d, *J* = 20.1 Hz, 1H), 7.72 - 7.54 (m, 2H), 7.35 (d, *J* = 7.4 Hz, 2H), 7.26 (t, *J* = 7.6 Hz, 4H), 5.91 (s, 1H), 3.75 (dd, *J* = 18.5, 12.0 Hz, 2H), 3.53 (s, 2H), 2.79 (s, 6H), 1.30 - 0.71 (m, 2H). | 433.3 [M+H] + |
| **28** | 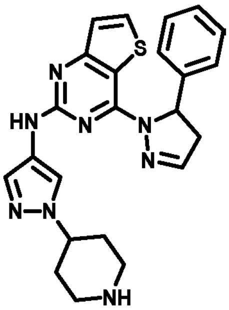 | 4-(5-phenyl-4,5-dihydro-1*H*-pyrazol-1-yl)-*N*-(1-(piperidin-4-yl)-1*H*-pyrazol-4-yl)thieno [3,2-*d*]pyrimidin-2-amine | $^1$H NMR (400 MHz, DMSO-$d_6$) δ 8.85 (s, 1H), 8.56 (d, *J* = 8.0 Hz, 1H), 8.30 (s, 1H), 7.35 (t, *J* = 7.4 Hz, 3H), 7.31 -7.17 (m, 4H), 5.88 (s, 1H), 4.35 (s, 1H), 3.76 (dd, *J*= 18.5, 11.6 Hz, 1H), 3.43 (s, 2H), 3.15 (d,*J*= 17.8 Hz, 2H), 2.95 (d, *J*= 19.0 Hz, 1H), 2.07 (dd, *J*= 26.7, 13.9 Hz, 4H). | 445.2 [M+H] + |
| **29** | 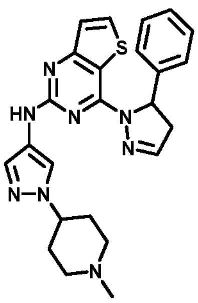 | *N*-(1-(1-methylpiperidin-4-yl)-1*H*-pyrazol-4-yl)-4-(5-phenyl-4,5-dihydro-1*H*-pyrazol-1-yl)thieno[3,2-*d*] pyrimidin-2-amine | $^1$H NMR (400 MHz, DMSO-$d_6$) δ 9.87 (s, 1H), 8.32 (s, 1H), 7.72 (s, 1H), 7.30 (ddd, *J* = 23.3, 13.8, 7.3 Hz, 8H), 5.90 (s, 1H), 4.29 (s, 1H), 3.77 (dd, *J* = 18.6, 11.7 Hz, 1H), 3.60 (s, 2H), 3.18 (d, *J* = 9.0 Hz, 2H), 2.96 (d, *J* = 20.2 Hz, 1H), 2.88 (d, *J* = 12.6 Hz, 3H), 2.29 - 1.91 (m, 4H). | 459.3 [M+H] + |
| **30** | 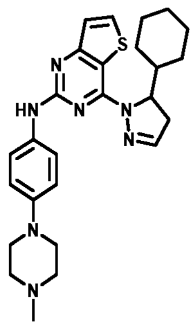 | 4-(5-cyclohexyl-4,5-dihydro-1*H*-pyrazol-1-yl)-*N*-(4-(4-methylpiperazin-1-yl)phenyl) thieno[3, 2-*d*]pyrimidin-2-amine | $^1$H NMR (400 MHz, DMSO-$d_6$) δ 11.25 (s, 1H), 9.83 (s, 1H), 8.22 (d, *J*= 5.5 Hz, 1H), 7.53 (s, 2H), 7.26 (d, *J* = 5.5 Hz, 1H), 6.99 (d, *J* = 9.0 Hz, 2H), 4.59 (dt, *J* = 10.9, 3.8 Hz, 1H), 3.36 (s, 4H), 3.10 (dd, *J* = 18.1, 11.3 Hz, 4H), 2.96 (dd, *J* = 19.0, 2.8 Hz, 1H), 2.80 (s, 3H), 2.28 *(d, J*= 11.1 Hz, 1H), 1.75 (s, 1H), 1.56 (dd, *J* = 36.3, 11.2 Hz, 4H), 1.27 - 1.14 (m, 3H), 1.11 - 0.97 (m, 3H) | 476.4 [M+H] + |

(continued)

| Example compounds | structure | Compound name | $^1$H NMR | LC-MS (m/z) |
|---|---|---|---|---|
| **31** | 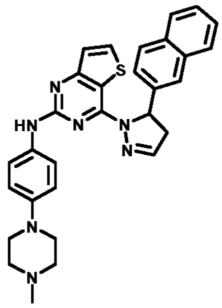 | *N*-(4-(4-methy lpiperazin-1-yl)phenyl)-4-(5-(naphthalen-2-yl)-4,5-dihydro-1*H*-pyrazol-1-yl)thieno[3,2-*d*] pyrimidin-2-amine | $^1$H NMR (400 MHz, DMSO-$d_6$) δ8.76 (s, 1H), 8.08 (d, *J* = 5.5 Hz, 1H), 7.89 (d, *J* = 5.0 Hz, 2H), 7.83 (s, 1H), 7.50 - 7.45 (m, 2H), 7.44 (d, *J* = 1.4 Hz, 1H), 7.35 (dd, *J* = 8.5, 1.6 Hz, 1H), 7.17 (d, *J* = 8.6 Hz, 2H), 7.11 (d, *J*= 5.5 Hz, 1H), 6.53 (d, *J*= 8.9 Hz, 2H), 5.90 (dd, *J* = 12.0, 4.8 Hz, 1H), 3.75 (ddd, *J* = 18.5, 12.1, 1.3 Hz, 1H), 3.03 (d, *J* = 4.8 Hz, 4H), 2.89 (ddd, *J* = 18.7, 4.8, 1.6 Hz, 1H), 2.72 (s, 4H), 2.42 (s, 3H) | 520.4 [M+H]+ |
| **32** | 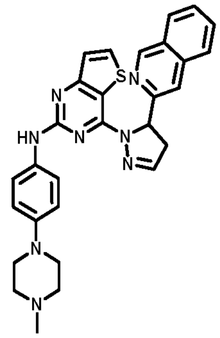 | 4-(5-(isoquinolin-3-yl)-4,5-dihydro-1*H*-pyrazol-1-yl)-*N*-(4-(4-methylpiperazin-1-yl)phenyl)thieno[3, 2-*d*] pyrimidin-2-amine | $^1$H NMR (400 MHz, DMSO-$d_6$) δ 9.30 (s, 1H), 8.74 (s, 1H), 8.06 (d, *J*= 5.5 Hz, 1H), 7.88 (d, *J*= 8.2 Hz, 1H), 7.79 (s, 1H), 7.77 - 7.70 (m, 1H), 7.65 - 7.59 (m, 1H), 7.43 (s, 1H), 7.27 (d, *J* = 8.6 Hz, 2H), 7.10 (d, *J* = 5.5 Hz, 1H), 6.65 (d, *J* = 9.0 Hz, 2H), 5.93 (dd, *J* = 12.1, 4.9 Hz, 1H), 3.76 - 3.65 (m, 1H), 3.16-3.09 (m, 4H), 3.05 (q, *J* = 7.3 Hz, 3H), 2.86 (s, 4H), 2.50 (s, 3H) | 521.4 [M+H]+ |
| **33** | 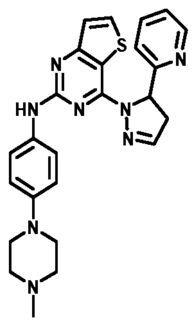 | *N*-(4-(4-methy lpiperazin-1-yl)phenyl)-4-(5-(pyridin-2-yl)-4,5-dihydro-1*H*-pyrazol-1-yl)thieno[3,2-*d*]pyrimidin-2-amine | $^1$H NMR (400 MHz, DMSO-$d_6$) δ 8.69 (s, 1H), 8.54 (d, *J* = 4.4 Hz, 1H), 8.04 (d, *J* = 5.5 Hz, 1H), 7.71 (t, *J* = 7.6 Hz, 1H), 7.37 (s, 1H), 7.34 - 7.23 (m, 4H), 7.10 (d, *J* = 5.5 Hz, 1H), 6.74 (d, *J*= 8.9 Hz, 2H), 5.76 (dd, *J* = 12.1, 4.6 Hz, 1H), 3.63 *(dd, J* = 18.3, 12.0 Hz, 1H), 3.10-3.02 (m, 4H), 2.97 (dd, *J* = 18.6, 4.0 Hz, 1H), 2.55 - 2.51 (m, 4H), 2.27 (s, 3H) | 471.4 [M+H]+ |
| **34** | 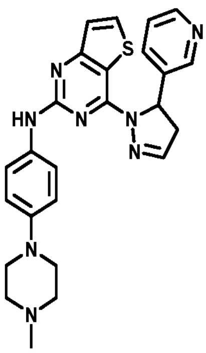 | *N*-(4-(4-methy lpiperazin-1-yl)phenyl)-4-(5-(pyridin-3-yl)-4,5-dihydro-1*H*-pyrazol-1-yl)thieno[3,2-*d*]pyrimidin-2-amine | $^1$H NMR (400 MHz, DMSO-$d_6$) δ 9.02 (s, 1H), 8.57 (d, *J* = 2.0 Hz, 1H), 8.48 (dd, *J* = 4.8, 1.5 Hz, 1H), 8.12 (d, *J* = 5.5 Hz, 1H), 7.59 (dd, *J* = 7.9, 1.8 Hz, 1H), 7.48 (s, 1H), 7.37 (dd, *J* = 7.8, 4.8 Hz, 1H), 7.28 (d, *J* = 8.4 Hz, 2H), 7.15 (d, *J* = 5.5 Hz, 1H), 7.05 (d, *J* = 8.9 Hz, 1H), 6.98 (d, *J* = 8.9 Hz, 1H), 6.82 (d, *J*= 9.0 Hz, 2H), 5.78 (dd, *J*= 11.9, 4.6 Hz, 1H), 3.71 (ddd, *J* = 18.6, 12.0, 1.3 Hz, 1H), 3.38 (dd, *J* = 14.5, 7.2 Hz, 4H), 3.05 (dd, *J* = 14.4, 7.2 Hz, 2H), 2.92 (ddd, *J* = 18.7, 4.6, 1.6 Hz, 1H), 2.80 (s, 3H) | 471.4 [M+H]+ |

(continued)

| Example compounds | structure | Compound name | $^1$H NMR | LC-MS (m/z) |
|---|---|---|---|---|
| **35** | 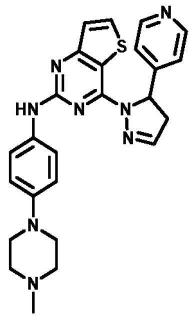 | *N*-(4-(4-methy lpiperazin-1-yl)phenyl)-4-(5-(pyridin-4-yl)-4,5-dihydro-1*H*-pyrazol-1-yl)thieno[3,2-*d*]pyrimidin-2-amine | $^1$H NMR (400 MHz, DMSO-$d_6$) δ 8.77 (s, 1H), 8.53 (dd, *J* = 4.5, 1.4 Hz, 2H), 8.08 (d, *J* = 5.5 Hz, 1H), 7.40 (s, 1H), 7.23 (dd, *J* = 9.3, 4.7 Hz, 4H), 7.12 (d, *J* = 5.5 Hz, 1H), 6.72 (d, *J* = 9.0 Hz, 2H), 5.73 (dd, *J* = 12.2, 4.8 Hz, 1H), 3.69 (ddd, *J* = 18.5, 12.2, 1.3 Hz, 1H), 3.12 (s, 4H), 2.83 (ddd, *J* = 18.7, 4.8, 1.6 Hz, 1H), 2.72 (s, 4H), 2.40 (s, 3H) | 471.4 [M+H]+ |
| **36** | 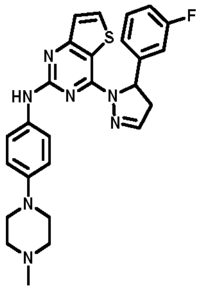 | 4-(5-(3-fluorophenyl)-4,5-dihydro-1*H*-pyrazol-1-yl)-*N*-(4-(4-methylpiperazin-1-yl)phenyl)thieno[3, 2-*d*] pyrimidin-2-amine | $^1$H NMR (400 MHz, DMSO-$d_6$) δ 11.22 (s, 1H), 9.18 (s, 1H), 8.15 (d, *J*= 5.5 Hz, 1H), 7.49 (s, 1H), 7.40 (dt, *J* = 7.5, 6.9 Hz, 1H), 7.26 (d, *J*= 8.6 Hz, 2H), 7.18 (d, *J*= 5.5 Hz, 1H), 7.11 - 7.02 (m, 3H), 6.81 (d, *J* = 9.0 Hz, 2H), 5.75 (dd, *J* = 11.9, 4.4 Hz, 1H), 3.69 (ddd, *J* = 18.6, 11.9, 1.3 Hz, 1H), 3.43 (d, *J* = 26.8 Hz, 4H), 3.17 (s, 4H), 2.87 (ddd, *J* = 18.8, 4.4, 1.6 Hz, 1H), 2.81 (s, 3H) | 488.4 [M+H]+ |
| **37** | 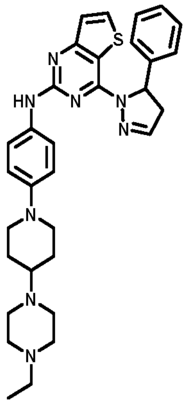 | *N*-(4-(4-(4-ethy lpiperazin-1-yl)piperidin-1-yl)phenyl)-4-(5-phenyl-4,5-dihydro-1*H*-pyrazol-1-yl)thieno[3,2-*d*]pyrimidin-2-amine | | 567.5 [M+H]+ |
| **38** | 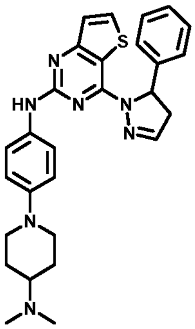 | *N*-(4-(4-(dimethylamino)piperidin-1-yl)phenyl)-4-(5-phenyl-4,5-dihydro-1*H*-pyrazol-1-yl)thieno[3,2-*d*] pyrimidin-2-amine | $^1$H NMR (400 MHz, DMSO-$d_6$) δ 8.71 (s, 1H), 8.05 (d, *J* = 5.5 Hz, 1H), 7.40 - 7.31 (m, 3H), 7.25 - 7.19 (m, 4H), 7.10 (d, *J* = 5.5 Hz, 1H), 6.71 (d, *J* = 9.0 Hz, 2H), 5.74 (dd, *J* = 11.9, 4.2 Hz, 1H), 3.66 (ddd, *J* = 18.5, 11.9, 1.4 Hz, 1H), 3.55 (d, *J* = 12.4 Hz, 2H), 2.80 (ddd, *J* = 18.6, 4.2, 1.7 Hz, 1H), 2.56 (t, *J* = 12.0 Hz, 2H), 2.20 (s, 6H), 1.84 (d, *J* = 11.9 Hz, 2H), 1.49 (qd, *J* = 12.1, 3.8 Hz, 2H) | 498.4 [M+H]+ |

(continued)

| Example compounds | structure | Compound name | $^1H$ NMR | LC-MS (m/z) |
|---|---|---|---|---|
| **39** | 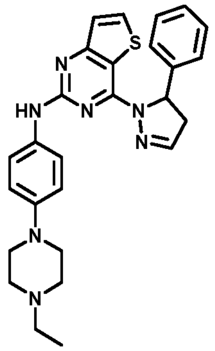 | *N*-(4-(4-ethy lpiperazin-1-yl) phenyl)-4-(5-phenyl-4,5-dihydro-1*H*-pyrazol-1-yl) thieno[3,2-*d*]pyrimidin-2-amine | $^1H$ NMR (400 MHz, DMSO-$d_6$) δ 8.72 (s, 1H), 8.05 (d, *J* = 5.5 Hz, 1H), 7.35 (dd, *J* = 15.3, 7.5 Hz, 3H), 7.24 (dd, *J* = 8.2, 6.7 Hz, 5H), 7.10 (d, *J* = 5.5 Hz, 1H), 6.71 (d, *J* = 9.1 Hz, 2H), 5.74 (dd, *J*= 11.9,4.2 Hz, 1H), 3.66 (ddd, *J* = 18.5, 12.0, 1.4 Hz, 1H), 3.07 - 2.95 (m, 4H), 2.80 (ddd, *J* = 18.6, 4.3, 1.7 Hz, 1H), 2.51 (d, *J* = 2.0 Hz, 4H), 2.37 (q, *J* = 7.2 Hz, 2H), 1.04 (t, *J*= 7.2 Hz, 3H) | 484.3 [M+H] + |
| **40** | 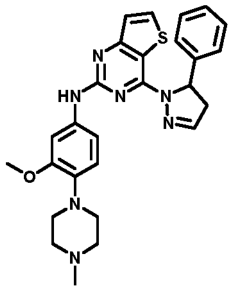 | *N*-(3-methoxy-4-(4-methy lpiperazin-1-yl)phenyl)-4-(5-phenyl-4,5-dihydro-1*H*-pyrazol-1-yl)thieno[3,2-*d*]pyrimidin-2-amine | $^1H$ NMR (400 MHz, DMSO-$d_6$) δ 8.76 (s, 1H), 8.07 (d, *J* = 5.5 Hz, 1H), 7.40 (s, 1H), 7.31 (d, *J* = 7.2 Hz, 2H), 7.23 (d, *J* = 6.6 Hz, 3H), 7.14 (d, *J* = 5.5 Hz, 2H), 6.67 (d, *J* = 8.6 Hz, 1H), 5.76 (dd, *J*= 11.8, 4.1 Hz, 1H), 3.73 (s, 3H), 3.69 - 3.63 (m, 1H), 3.04 (s, 4H), 2.88 (d, *J* = 11.4 Hz, 4H), 2.86 - 2.80 (m, 1H), 2.52 (s, 3H) | 500.4 [M+H] + |
| **41** | 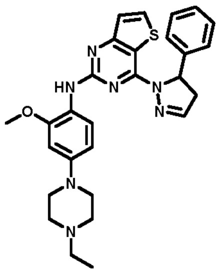 | *N*-(4-(4-ethy lpiperazin-1-yl)-2-methoxyphenyl)-4-(5-phenyl-4,5-dihydro-1*H*-pyrazol-1-yl)thieno[3,2-*d*] pyrimidin-2-amine | $^1H$ NMR (400 MHz, DMSO-$d_6$) δ 8.05 (d, *J* = 5.5 Hz, 1H), 7.61 (d, *J*= 8.1 Hz, 1H), 7.39 (s, 1H), 7.32 (t, *J* = 7.4 Hz, 2H), 7.24 (d, *J* = 7.3 Hz, 1H), 7.21 - 7.16 (m, 3H), 7.11 (d, *J* = 5.5 Hz, 1H), 6.58 (d, *J*= 2.4 Hz, 1H), 6.34 (dd, *J* = 8.8, 2.4 Hz, 1H), 5.68 (dd, *J* = 11.9, 4.5 Hz, 1H), 3.78 (s, 3H), 3.64 (ddd, *J* = 18.5, 11.9, 1.3 Hz, 1H), 3.12 - 3.04 (m, 4H), 2.82 (ddd, *J* = 18.6, 4.5, 1.6 Hz, 1H), 2.52 (s, 4H), 2.38 (q, *J* = 7.1 Hz, 2H), 1.04 (t, *J* = 7.2 Hz, 3H) | 514.4 [M+H] + |
| **42** | 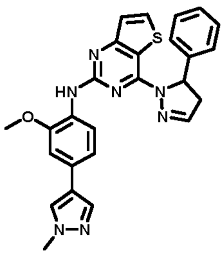 | *N*-(2-methoxy-4-(1-methyl-1*H*-pyrazol-4-yl)phenyl)-4-(5-phenyl-4,5-dihydro-1*H*-pyrazol-1-yl)thieno[3,2-*d*]pyrimidin-2-amine | $^1H$ NMR (400 MHz, DMSO-$d_6$) δ 8.11 (d,*J* = 5.5 Hz, 1H), 7.84 (d, *J*= 0.5 Hz, 1H), 7.43 (s, 1H), 7.36 (d, *J*= 2.9 Hz, 2H), 7.25 (dd, *J* = 5.1, 3.0 Hz, 3H), 7.18 (d, *J*= 5.5 Hz, 1H), 7.14 (d, *J* = 1.8 Hz, 1H), 6.97 (dd, *J* = 8.3, 1.7 Hz, 1H), 5.75 (dd, *J* = 11.9, 4.5 Hz, 1H), 3.88 (d, *J* = 3.2 Hz, 6H), 3.68 (ddd, *J* = 18.6, 11.9, 1.4 Hz, 1H), 2.84 (ddd, *J* = 18.7, 4.6, 1.7 Hz, 1H) | 482.3 [M+H] + |

(continued)

| Example compounds | structure | Compound name | $^1H$ NMR | LC-MS (m/z) |
|---|---|---|---|---|
| **43** | 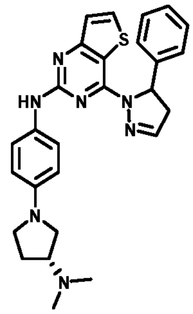 | *N*-(4-((*R*)-3-(dimethylamino) pyr rolidin-1-yl)phenyl)-4-(5-phenyl-4,5-dihydro-1*H*-pyrazol-1-yl)thieno[3,2-*d*]pyrimidin-2-amine | $^1H$ NMR (400 MHz, DMSO-$d_6$) δ 8.55 (s, 1H), 8.02 (d, *J* = 5.5 Hz, 1H), 7.34 (d, *J* = 12.6 Hz, 2H), 7.23 - 7.19 (m, 3H), 7.08 (d, *J* = 5.5 Hz, 1H), 6.37 (d, *J*= 8.9 Hz, 2H), 5.71 (dd, *J* = 11.9, 4.2 Hz, 1H), 3.70 - 3.57 (m, 1H), 3.38 (dd, *J* = 8.8, 7.5 Hz, 1H), 3.29 (td, *J* = 8.8, 2.2 Hz, 1H), 3.20 (dt, *J* = 9.1, 6.4 Hz, 1H), 3.10 - 3.04 (m, 1H), 2.99 - 2.90 (m, 1H), 2.83 - 2.75 (m, 1H), 2.29 (s, 6H), 2.21 - 2.09 (m, 1H), 1.86 (dq, *J* = 18.0, 9.0 Hz, 1H) | 484.3 [M+H] + |
| **44** | 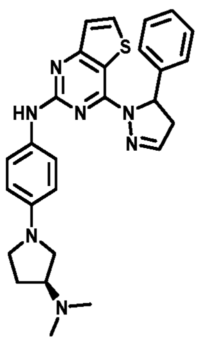 | *N*-(4-((S)-3-(dimethylamino) pyr rolidin-1-yl)phenyl)-4-(5-phenyl-4,5-dihydro-1*H*-pyrazol-1-yl)thieno[3,2-*d*]pyrimidin-2-amine | $^1H$ NMR (400 MHz, DMSO-$d_6$) δ 8.65 (s, 1H), 8.05 (d, *J* = 5.5 Hz, 1H), 7.36 (dd, *J* = 15.4, 8.0 Hz, 3H), 7.27 - 7.19 (m, 4H), 7.10 (d, *J* = 5.5 Hz, 1H), 6.44 (d, *J* = 8.9 Hz, 2H), 5.72 (dd, *J* = 11.9, 4.2 Hz, 1H), 3.93 (dd, *J* = 14.5, 7.3 Hz, 1H), 3.66 (ddd, *J* = 18.5, 11.9, 1.3 Hz, 1H), 3.51 (dt, *J* = 10.1, 6.7 Hz, 2H), 3.46 - 3.39 (m, 1H), 3.20 (dd, *J* = 16.9, 8.1 Hz, 1H), 2.83 (dd, *J* = 4.2, 1.7 Hz, 1H), 2.79 (s, 6H), 2.44 - 2.34 (m, 1H), 2.28 (dq, *J* = 12.8, 8.4 Hz, 1H) | 484.3 [M+H] + |
| **45** | 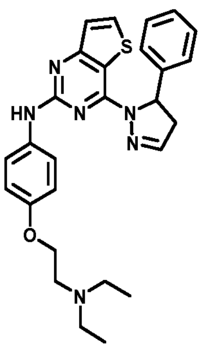 | *N*-(4-(2-(diethylamino)ethoxy)phenyl)-4-(5-phenyl-4,5-dihydro-1*H*-pyrazol-1-yl) thieno[3,2-*d*]pyrimidin-2-amine | $^1H$ NMR (400 MHz, DMSO-$d_6$) δ 8.82 (s, 1H), 8.07 (d, *J* = 5.5 Hz, 1H), 7.40 - 7.21 (m, 9H), 7.12 (d, *J* = 5.5 Hz, 1H), 6.71 (d, *J* = 9.0 Hz, 2H), 5.75 (dd, *J* = 11.9, 4.2 Hz, 1H), 4.04 (s, 2H), 3.67 (ddd, *J* = 18.5, 11.9, 1.3 Hz, 1H), 2.94 (d, *J* = 7.0 Hz, 4H), 2.81 (ddd, *J* = 18.7, 4.2, 1.7 Hz, 1H), 2.72 (s, 4H), 1.06 (t, *J* = 6.7 Hz, 6H) | 487.4 [M+H] + |
| **46** | 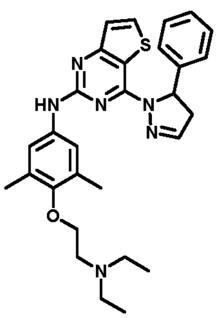 | *N*-(4-(2-(diethylamino)ethoxy)-3,5-dimethylphenyl)-4-(5-phenyl-4,5-dihydro-1*H*-pyrazol-1-yl)thieno[3,2-*d*] pyrimidin-2-amine | | 515.5 [M+H] + |

(continued)

| Example compounds | structure | Compound name | $^1H$ NMR | LC-MS (m/z) |
|---|---|---|---|---|
| **47** | 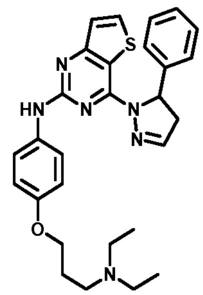 | *N*-(4-(3-(diethylamino)propoxy)phenyl)-4-(5-phenyl-4,5-dihydro-1*H*-pyrazol-1-yl)thieno[3,2-*d*]pyrimidin-2-amine | $^1H$ NMR (400 MHz, DMSO-$d_6$) δ 8.80 (s, 1H), 8.06 (d, *J* = 5.5 Hz, 1H), 7.36 (d, *J* = 17.4 Hz, 2H), 7.27 (d, *J* = 9.2 Hz, 2H), 7.24 - 7.21 (m, 2H), 7.11 (d, *J* = 5.5 Hz, 1H), 6.69 (d, *J* = 9.0 Hz, 2H), 5.74 (dd, *J* = 11.9, 4.2 Hz, 1H), 3.94 (t, *J* = 6.3 Hz, 2H), 3.71 - 3.62 (m, 1H), 2.80 (ddd, *J* = 18.6, 4.2, 1.6 Hz, 1H), 2.58 (dt, *J* = 20.5, 6.5 Hz, 4H), 1.83 (dt, *J* = 13.3, 6.5 Hz, 2H), 1.00 (t, *J* = 7.2 Hz, 6H) | 501.4 [M+H]+ |
| **48** | 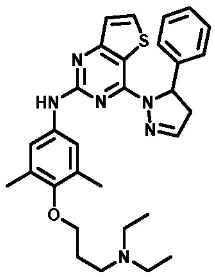 | *N*-(4-(3-(diethylamino)propoxy)-3,5-dimethylphenyl)-4-(5-phenyl-4,5-dihydro-1*H*-pyrazol-1-yl)thieno[3,2-*d]* pyrimidin-2-amine | | 529.5 [M+H]+ |
| **49** | 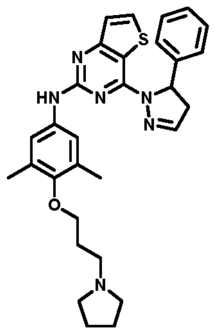 | *N*-(3,5-dimethyl-4-(3-(pyrrolidin-1-yl)propoxy)phenyl) -4-(5-phenyl-4,5-dihydro-1*H*-pyrazol-1-yl)thieno[3,2-*d*] pyrimidin-2-amine | $^1H$ NMR (400 MHz, DMSO-$d_6$) δ 8.70 (s, 1H), 8.06 (d, *J* = 5.5 Hz, 1H), 7.40 (s, 1H), 7.27 (d, *J* = 7.1 Hz, 2H), 7.22 (s, 3H), 7.13 (d, *J* = 5.5 Hz, 1H), 5.82 (dd, *J* = 11.7, 3.6 Hz, 1H), 3.70 (t, *J* = 6.3 Hz, 2H), 2.86 (ddd, *J* = 18.5, 3.6, 1.8 Hz, 1H), 2.75 (t, *J* = 7.1 Hz, 2H), 2.63 (s, 4H), 2.12 (d, *J* = 8.1 Hz, 6H), 1.74 (s, 4H) | 527.5 [M+H]+ |
| **50** | 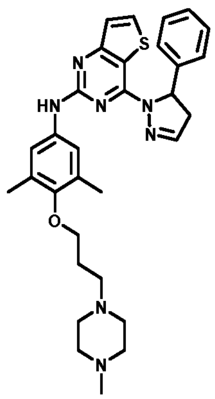 | *N*-(3,5-dimethyl-4-(3-(4-methylpiperazin-1-yl)propoxy)phenyl) -4-(5-phenyl-4,5-dihydro-1*H*-pyrazol-1-yl)thieno[3,2-*d*] pyrimidin-2-amine | | 556.4 [M+H]+ |

(continued)

| Example compounds | structure | Compound name | $^1$H NMR | LC-MS (m/z) |
|---|---|---|---|---|
| **51** | 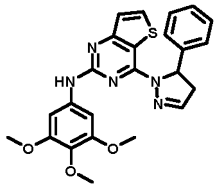 | 4-(5-phenyl-4,5-dihydro-1*H*-pyrazol-1-yl)-*N*-(3,4,5-trimethoxyphenyl)t hieno [3,2-*d*]pyrimidin-2-amine | $^1$H NMR (400 MHz, DMSO-$d_6$) δ 8.98 (s, 1H), 8.13 (d, *J* = 5.5 Hz, 1H), 7.51 (s, 1H), 7.28 - 7.24 (m, 2H), 7.19 (dd, *J* = 5.6, 4.6 Hz, 3H), 7.08 (s, 2H), 5.75 (dd, *J* = 11.5, 3.6 Hz, 1H), 3.73 (d, *J* = 7.5 Hz, 6H), 3.72 - 3.66 (m, 1H), 3.63 (s, 3H), 2.92 (ddd, *J* = 18.7, 3.6, 1.7 Hz, 1H) | 462.3 [M+H] + |
| **52** | 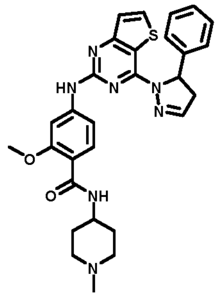 | 2-methoxy-*N*-(1-methylpiperidin-4-yl)-4-((4-(5-phenyl-4,5-dihydro-1*H*-pyrazol-1-yl)thieno[3,2-*d*]pyrimidin-2-yl)amino) benzamid e | $^1$H NMR (400 MHz, DMSO-$d_6$) δ 9.26 (s, 1H), 8.13 (d, *J* = 5.5 Hz, 1H), 7.81 (d, *J* = 7.6 Hz, 1H), 7.63 (d, *J* = 9.1 Hz, 1H), 7.44 (s, 1H), 7.35 - 7.30 (m, 3H), 7.27 - 7.24 (m, 2H), 7.22 (d, *J* = 5.5 Hz, 1H), 5.82 (dd, *J* = 11.8, 4.0 Hz, 1H), 3.86 (s, 2H), 3.75 - 3.65 (m, 1H), 2.86 (ddd, *J* = 18.6, 3.9, 1.7 Hz, 1H), 2.66 (d, *J* = 10.0 Hz, 2H), 2.19 (s, 3H), 2.10 (t, *J* = 10.1 Hz, 2H), 1.82 (d, *J* = 10.0 Hz, 2H), 1.54 (dd, *J* = 19.6, 9.4 Hz, 2H) | 542.4 [M+H] + |
| **53** | 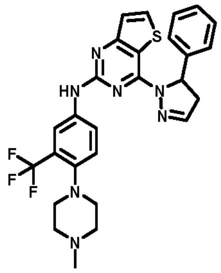 | *N*-(4-(4-methylpiperazin-1-yl)-3-(trifluoromethyl)ph enyl)-4-(5-phenyl-4,5-dihydro-1*H*-pyrazol-1-yl) thieno[3,2-*d*]pyrimidin-2-amine | $^1$H NMR (400 MHz, DMSO-$d_6$) δ 9.24 (s, 1H), 8.11 (d, *J* = 5.5 Hz, 1H), 7.78 (s, 1H), 7.43 (s, 1H), 7.31 (d, *J*= 7.3 Hz, 1H), 7.24 - 7.21 (m, 2H), 7.16 (d, *J* = 5.5 Hz, 1H), 5.77 (dd, *J* = 11.8, 4.1 Hz, 1H), 3.69 (ddd, *J* = 18.5, 11.8, 1.3 Hz, 1H), 2.86 (dd, *J* = 4.1, 1.7 Hz, 1H), 2.80 (dd, *J* = 6.2, 3.0 Hz, 4H), 2.44 (s, 4H), 2.23 (s, 3H) | 538.4 [M+H] + |
| **54** | 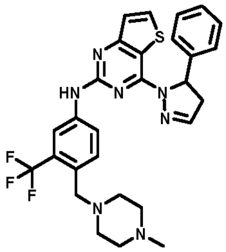 | *N*-(4-((4-methylpiperazin-1-yl)methyl)-3-(trifluoromethyl) ph enyl)-4-(5-phenyl-4,5-dihydro-1*H*-pyrazol-1-yl) thieno[3,2-*d*]pyrimidin-2-amine | $^1$H NMR (400 MHz, DMSO-$d_6$) δ 9.32 (s, 1H), 8.12 (d, *J* = 5.5 Hz, 1H), 7.79 (s, 1H), 7.43 (s, 1H), 7.30 (d, *J* = 7.2 Hz, 1H), 7.22 (dd, *J* = 5.1, 2.8 Hz, 2H), 7.18 (d, *J* = 5.5 Hz, 1H), 5.80 (dd, *J* = 11.8, 4.1 Hz, 1H), 3.70 (ddd, *J* = 18.5, 11.8, 1.3 Hz, 1H), 3.50 (s, 2H), 2.84 (ddd, *J* = 18.6, 4.1, 1.7 Hz, 1H), 2.38 (s, 8H), 2.18 (s, 3H) | 552.4 [M+H] + |

(continued)

| Example compounds | structure | Compound name | $^1H$ NMR | LC-MS (m/z) |
|---|---|---|---|---|
| **55** | 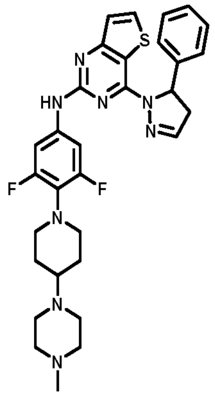 | *N*-(3,5-difluoro-4-(4-(4-methylpiperazin-1-yl) piperidin-1-yl)phenyl)-4-(5-phenyl-4,5-dihydro-1*H*-pyrazol-1-yl)thieno[3,2-*d*]pyrimidin-2-amine | $^1H$ NMR (400 MHz, DMSO-$d_6$) δ 9.25 (s, 1H), 8.12 (d, *J* = 5.5 Hz, 1H), 7.43 (s, 1H), 7.29 (d, *J* = 7.2 Hz, 2H), 7.23 (s, 2H), 7.18 (d, *J* = 5.5 Hz, 1H), 5.81 (dd, *J* = 11.7, 3.8 Hz, 1H), 3.75 - 3.64 (m, 1H), 3.09 - 2.90 (m, 4H), 2.83 (ddd, *J* = 18.6, 3.7, 1.7 Hz, 1H), 2.29 (dd, *J* = 13.0, 9.4 Hz, 4H), 2.14 (s, 3H), 1.76 (d, *J* = 11.3 Hz, 2H), 1.50 (qd, *J* = 11.8, 4.1 Hz, 2H) | 589.4 [M+H]+ |
| **56** | 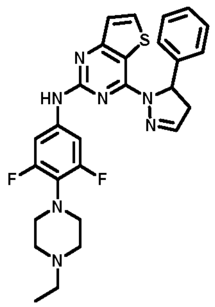 | *N*-(4-(4-ethylpiperazin-1-yl)-3,5-difluorophenyl)-4-(5-phenyl-4,5-dihydro-1*H*-pyrazol-1-yl)thieno[3,2-*d*] pyrimidin-2-amine | $^1H$ NMR (400 MHz, DMSO-$d_6$) δ 9.26 (s, 1H), 8.13 (d, *J* = 5.5 Hz, 1H), 7.44 (s, 1H), 7.31 - 7.20 (m, 6H), 7.18 (d, *J* = 5.5 Hz, 1H), 5.81 (dd, *J* = 11.7, 3.8 Hz, 1H), 3.76 - 3.64 (m, 1H), 3.01 (d, *J* = 4.3 Hz, 4H), 2.83 (ddd, *J* = 18.6, 3.7, 1.7 Hz, 1H), 2.46 (s, 4H), 2.37 (q, *J* = 7.1 Hz, 2H), 1.02 (t, *J* = 7.2 Hz, 3H) | 520.3 [M+H]+ |
| **57** | 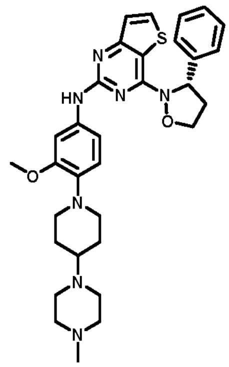 | (S)-*N*-(3-methoxy-4-(4-(4-methylpiperazin-1-yl) piperidin-1-yl)phenyl)-4-(3-pheny lisoxazolidin-2-yl) thieno [3,2-*d*]pyrimidin-2-amine | $^1H$ NMR (400 MHz, DMSO-$d_6$) δ 9.06 (s, 1H), 8.13 (d, *J* = 5.5 Hz, 1H), 7.50 - 7.45 (m, 2H), 7.42 - 7.34 (m, 3H), 7.33 - 7.27 (m, 1H), 7.21 (d, *J* = 5.5 Hz, 1H), 6.70 (d, *J* = 8.6 Hz, 1H), 5.89 (dd, *J* = 8.7, 5.3 Hz, 1H), 3.84 (q, *J* = 7.9 Hz, 1H), 3.62 (s, 3H), 3.31 - 3.25 (m, 2H), 2.97 - 2.87 (m, 1H), 2.54 (s, 2H), 2.46 - 2.22 (m, 9H), 2.19 (s, 3H), 1.79 (d, *J* = 12.0 Hz, 2H), 1.55 - 1.48 (m, 2H), 1.31 - 1.20 (m, 2H). | 586.4 [M+H]+ |
| **58** | 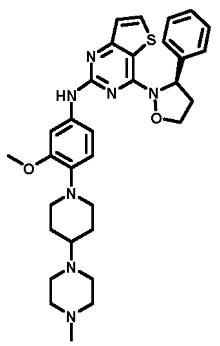 | (R)-*N*-(3-methoxy-4-(4-(4-methylpiperazin-l-yl) piperidin-1-yl)phenyl)-4-(3-pheny lisoxazolidin-2-yl) thieno [3,2-*d*]pyrimidin-2-amine | $^1H$ NMR (400 MHz, DMSO-$d_6$) δ 9.06 (s, 1H), 8.14 (d, *J* = 5.5 Hz, 1H), 7.48 (d, *J* = 7.2 Hz, 2H), 7.40 (dd, *J* = 8.4, 6.8 Hz, 2H), 7.34 (d, *J* = 2.3 Hz, 1H), 7.33 - 7.28 (m, 1H), 7.21 (d, *J* = 5.5 Hz, 1H), 6.70 (d, *J* = 8.6 Hz, 1H), 5.89 (dd, *J* = 8.7, 5.3 Hz, 1H), 4.33 (td, *J* = 7.8, 3.2 Hz, 1H), 3.85 (q, *J* = 8.0 Hz, 2H), 3.62 (s, 3H), 3.28 (d, *J* = 10.1 Hz, 4H), 3.00 - 2.85 (m, 2H), 2.44 (dd, *J* = 11.7, 2.3 Hz, 3H), 2.39 - 2.18 (m, 7H), 2.14 (s, 3H), 1.84 (s, 3H), 1.82 - 1.74 (m, 2H), 1.51 (qd, *J* = 12.0, 3.8 Hz, 2H). | 586.4 [M+H]+ |

(continued)

| Example compounds | structure | Compound name | $^1$H NMR | LC-MS (m/z) |
|---|---|---|---|---|
| **59** | 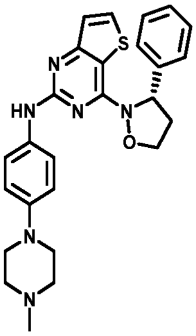 | (S)-N-(4-(4-methylpiperazin-l-yl)phenyl)-4-(3-phenylisoxazolidin-2-yl) thieno[3,2-d]pyrimidin-2-amine | $^1$H NMR (400 MHz, DMSO) δ 9.00 (s, 1H), 8.12 (d, *J* = 5.5 Hz, 1H), 7.53 - 7.47 (m, 4H), 7.41 (dd, *J* = 8.4, 6.9 Hz, 2H), 7.32 (d, *J* = 7.3 Hz, 1H), 7.18 (d, *J* = 5.5 Hz, 1H), 6.80 - 6.74 (m, 2H), 5.82 (dd, *J* = 8.6, 5.8 Hz, 1H), 4.36 (td, *J* = 7.9, 2.9 Hz, 1H), 3.84 (dt, *J* = 9.2, 7.3 Hz, 1H), 3.05 - 2.99 (m, 4H), 2.97 - 2.89 (m, 1H), 2.45 (t, *J* = 5.0 Hz, 4H), 2.34 - 2.26 (m, 1H), 2.22 (s, 3H) | 473.2 [M+H]+ |
| **60** | 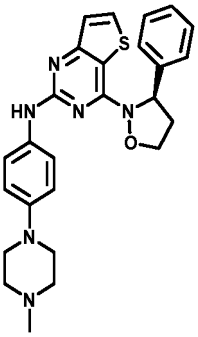 | (R)-*N*-(4-(4-methy lpiperazin-l-yl)phenyl)-4-(3-pheny lisoxazolidin-2-yl) thieno [3,2-*d*]pyrimidin-2-amine | $^1$H NMR (400 MHz, DMSO-$d_6$) δ 9.00 (s, 1H), 8.11 (d, *J* = 5.5 Hz, 1H), 7.51 - 7.46 (m, 3H), 7.40 (dd, *J* = 8.4, 6.8 Hz, 2H), 7.33 - 7.28 (m, 1H), 7.18 (d, *J* = 5.5 Hz, 1H), 6.80 - 6.73 (m, 2H), 5.82 (dd, *J* = 8.7, 5.8 Hz, 1H), 4.35 (td, *J* = 7.9, 3.0 Hz, 1H), 3.84 (dt, *J* = 9.4, 7.4 Hz, 1H), 3.02 (t, *J* = 5.1 Hz, 4H), 2.93 (tdd, *J* = 11.8, 7.1, 2.9 Hz, 1H), 2.45 (t, *J* = 5.0 Hz, 4H), 2.37 - 2.15 (m, 5H). | 473.3 [M+H]+ |
| **61** | 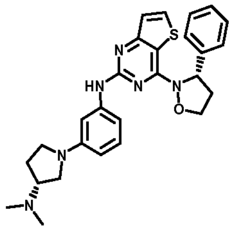 | N-(3-((R)-3-(dimethylamino) pyr rolidin-1-yl)phenyl)-4-((S)-3-pheny lisoxazolidin-2-yl)thieno [3,2-d]pyrimidin-2-amine | $^1$H NMR (400 MHz, DMSO-$d_6$) δ 9.02 (s, 1H), 8.15 (d, *J* = 5.5 Hz, 1H), 7.50 - 7.47 (m, 2H), 7.43 - 7.39 (m, 2H), 7.33 - 7.27 (m, 1H), 7.21 (d, *J* = 5.5 Hz, 1H), 7.09 - 7.04 (m, 1H), 6.96 (d, *J* = 1.8 Hz, 2H), 6.14 - 6.07 (m, 1H), 5.92 (dd, *J* = 8.7, 5.1 Hz, 1H), 3.86 (q, *J* = 7.8 Hz, 1H), 3.37 (dd, *J* = 9.1, 7.2 Hz, 3H), 3.21 (td, *J* = 9.0, 2.4 Hz, 1H), 3.11 (td, *J* = 9.4, 6.8 Hz, 1H), 2.99 (t, *J* = 8.4 Hz, 1H), 2.95 - 2.86 (m, 1H), 2.77 (t, *J* = 8.1 Hz, 1H), 2.41 - 2.31 (m, 2H), 2.19 (s, 6H), 2.11- 2.02 (m, 1H), 1.74 (dq, *J* = 11.8, 9.1 Hz, 1H). | 487.3 [M+H]+ |

(continued)

| Example compounds | structure | Compound name | $^1$H NMR | LC-MS (m/z) |
|---|---|---|---|---|
| **62** | 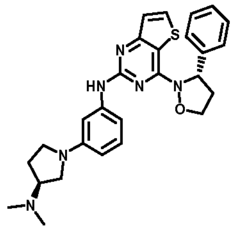 | *N*-(3-((S)-3-(dimethylamino) pyr rolidin-1-yl)phenyl)-4-((S)-3-pheny lisoxazolidin-2-yl)thieno [3,2-*d*]pyrimidin-2-amine | $^1$H NMR (400 MHz, DMSO-$d_6$) δ 9.02 (s, 1H), 8.15 (d, *J* = 5.5 Hz, 1H), 7.51 - 7.47 (m, 2H), 7.42 - 7.37 (m, 2H), 7.33 - 7.27 (m, 1H), 7.21 (d, *J* = 5.5 Hz, 1H), 7.06 (dd, *J* = 7.7, 1.9 Hz, 1H), 6.98 - 6.96 (m, 1H), 6.11 (dd, *J* = 8.2, 2.3 Hz, 1H), 5.93 (dd, *J* = 8.7, 5.0 Hz, 1H), 4.32 (td, *J* = 7.9, 3.5 Hz, 1H), 3.86 (q, *J* = 8.0 Hz, 1H), 3.39 - 3.35 (m, 1H), 3.24 (td, *J* = 9.1, 2.4 Hz, 1H), 3.11 (td, *J* = 9.4, 6.8 Hz, 1H), 2.99 (t, *J* = 8.4 Hz, 1H), 2.91 (dtt, *J* = 11.9, 8.0, 3.5 Hz, 1H), 2.81 - 2.70 (m, 1H), 2.41 - 2.30 (m, 1H), 2.20 (s, 6H), 2.06 (ddt, *J* = 9.4, 7.2, 3.6 Hz, 1H), 1.75 (dq, *J* = 11.7, 9.0 Hz, 1H). | 487.3 [M+H]+ |
| **63** | 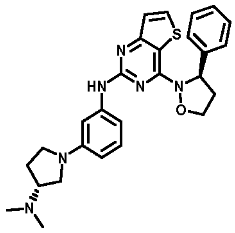 | *N*-(3-((R)-3-(dimethylamino) pyr rolidin-1-yl)phenyl)-4-((R)-3-pheny lisoxazolidin-2-yl)thieno [3,2-*d*]pyrimidin-2-amine | $^1$H NMR (400 MHz, DMSO-$d_6$) δ 9.03 (s, 1H), 8.15 (d, *J* = 5.5 Hz, 1H), 7.51 - 7.46 (m, 2H), 7.40 (dd, *J* = 8.4, 6.9 Hz, 2H), 7.33 - 7.27 (m, 1H), 7.21 (d, *J* = 5.5 Hz, 1H), 7.07 (dd, *J* = 7.9, 1.9 Hz, 1H), 6.98 (s, 1H), 6.14 - 6.09 (m, 1H), 5.93 (dd, *J* = 8.7, 5.0 Hz, 1H), 3.86 (q, *J* = 8.0 Hz, 1H), 3.42 - 3.34 (m, 2H), 3.25 (td, *J* = 9.1, 2.6 Hz, 2H), 3.14-3.03 (m, 2H), 2.97 - 2.86 (m, 2H), 2.40 - 2.25 (m, 8H), 2.14 - 2.06 (m, 1H), 1.87 - 1.75 (m, 1H). | 487.3 [M+H]+ |
| **64** | 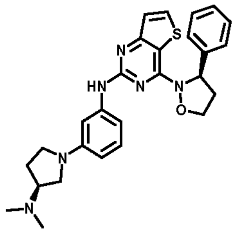 | *N*-(3-((S)-3-(dimethylamino) pyr rolidin-1-yl)phenyl)-4-((R)-3-pheny lisoxazolidin-2-yl)thieno [3,2-*d*]pyrimidin-2-amine | $^1$H NMR (400 MHz, DMSO-$d_6$) δ 9.02 (s, 1H), 8.15 (d, *J* = 5.5 Hz, 1H), 7.51 - 7.46 (m, 2H), 7.40 (dd, *J* = 8.4, 6.9 Hz, 3H), 7.32 - 7.27 (m, 1H), 7.21 (d, *J* = 5.5 Hz, 1H), 7.07 (dd, *J* = 8.3, 1.6 Hz, 1H), 6.96 (d, *J* = 2.2 Hz, 2H), 6.13 - 6.09 (m, 1H), 5.92 (dd, *J* = 8.7, 5.1 Hz, 1H), 3.90 - 3.80 (m, 1H), 3.42 - 3.34 (m, 3H), 3.21 (td, *J* = 9.1, 2.4 Hz, 1H), 3.11 (td, *J* = 9.4, 6.9 Hz, 1H), 2.99 (dd, *J* = 9.1, 7.8 Hz, 1H), 2.96 - 2.86 (m, 1H), 2.77 (p, *J* = 7.4 Hz, 1H), 2.36 (dtd, *J* = 12.0, 8.3, 5.2 Hz, 2H), 2.19 (s, 7H), 2.10 - 2.01 (m, 1H), 1.73 (dq, *J* = 11.7, 9.1 Hz, 1H). | 487.3 [M+H]+ |

(continued)

| Example compounds | structure | Compound name | $^1$H NMR | LC-MS (m/z) |
|---|---|---|---|---|
| **65** | 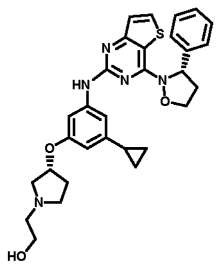 | 2-((R)-3-(3-cyclopropyl-5-((4-((S)-3-pheny lisoxazolidin-2-yl)thieno [3,2-*d*]pyrimidin-2-yl)amino) phenoxy) pyrrolidin-1-yl) ethan-l-ol | $^1$H NMR (400 MHz, DMSO-$d_6$) δ 9.15 (s, 1H), 8.16 (d, *J* = 5.5 Hz, 1H), 7.53 - 7.47 (m, 2H), 7.42 - 7.36 (m, 3H), 7.31 - 7.26 (m, 1H), 7.24 (s, 1H), 7.04 (t, *J* = 1.7 Hz, 1H), 6.07 (t, *J* = 1.9 Hz, 1H), 5.95 (dd, *J* = 8.8, 4.8 Hz, 1H), 4.72 (ddt, *J* = 9.3, 6.1, 2.9 Hz, 1H), 4.32 (td, *J* = 7.9, 3.5 Hz, 1H), 3.86 (q, *J* = 8.0 Hz, 1H), 3.49 (t, *J* = 6.3 Hz, 2H), 3.01 - 2.64 (m, 6H), 2.48 - 2.40 (m, 2H), 2.40 - 2.30 (m, 1H), 2.28 - 2.14 (m, 2H), 2.08 (s, 3H), 1.65 (tt, *J* = 8.4, 5.0 Hz, 1H), 0.87 - 0.79 (m, 2H), 0.57 (qd, *J* = 4.4, 1.2 Hz, 2H). | 544.3 [M+H] + |
| **66** | 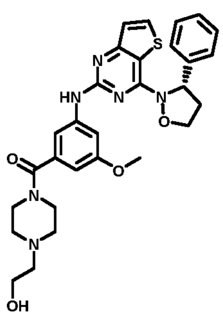 | (S)-(4-(2-hydroxyethyl)piper azin-1-yl)(3-methoxy-5-((4-(3-phenylisoxazolidin-2-yl)thieno[3,2-*d*]pyrimidin-2-yl)amino)phenyl)m ethanone | $^1$H NMR (400 MHz, DMSO-$d_6$) δ 9.43 (s, 1H), 8.19 (d, *J* = 5.5 Hz, 1H), 7.60 (t, *J* = 2.2 Hz, 1H), 7.51 - 7.48 (m, 2H), 7.39 (dd, *J* = 8.4, 6.8 Hz, 2H), 7.32 - 7.25 (m, 2H), 6.44 (dd, *J* = 2.4, 1.3 Hz, 1H), 5.93 (dd, *J* = 8.8, 4.9 Hz, 1H), 4.35 (td, *J* = 7.9, 3.5 Hz, 1H), 3.87 (q, *J* = 8.1 Hz, 1H), 3.69 (s, 3H), 3.50 (q, *J* = 6.0 Hz, 3H), 3.33 (s, 6H), 2.48 - 2.31 (m, 8H). | 561.3 [M+H] + |
| **67** | 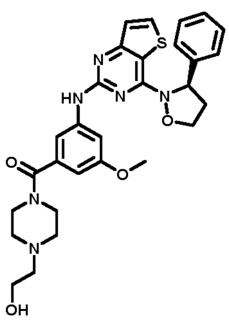 | (R)-(4-(2-hydroxyethyl)piper azin-1-yl)(3-methoxy-5-((4-(3-phenylisoxazolidin-2-yl)thieno[3,2-*d*]pyrimidin-2-yl)amino)phenyl)m ethanone | $^1$H NMR (400 MHz, DMSO-$d_6$) δ 9.43 (s, 1H), 8.19 (d, *J* = 5.5 Hz, 1H), 7.61 (t, *J* = 2.2 Hz, 1H), 7.51 - 7.48 (m, 2H), 7.39 (dd, *J* = 8.4, 6.8 Hz, 2H), 7.32 - 7.25 (m, 3H), 6.44 (dd, *J* = 2.5, 1.3 Hz, 1H), 5.93 (dd, *J* = 8.8, 4.8 Hz, 1H), 4.35 (td, *J* = 8.0, 3.5 Hz, 1H), 3.87 (q, *J* = 8.0 Hz, 1H), 3.69 (s, 3H), 3.51 (q, *J* = 5.9 Hz, 3H), 3.33 (s, 6H), 2.48 - 2.28 (m, 8H). | 561.3 [M+H] + |
| **68** | 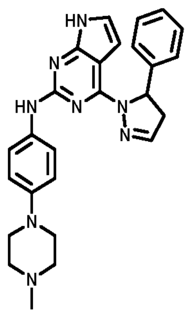 | *N*-(4-(4-methylpiperazin-1-yl)phenyl)-4-(5-phenyl-4,5-dihydro-1*H*-pyrazol-1yl)-7*H*-pyrrolo[2,3-*d*]pyrimidin-2-amine | $^1$H NMR (400 MHz, DMSO) δ 11.04 (s, 1H), 8.34 (s, 1H), 7.34 - 7.30 (m, 4H), 7.25 - 7.21 (m, 4H), 6.85 (dd, *J* = 3.5, 2.2 Hz, 1H), 6.74 - 6.71 (m, 2H), 6.68 (dd, *J* = 3.5, 2.0 Hz, 1H), 5.74 (dd, *J* = 11.9, 4.1 Hz, 1H), 3.13 (s, 4H), 2.79 (d, *J* = 30.6 Hz, 4H), 2.77 - 2.69 (m, 2H), 1.23 (s, 3H) | 453.2 [M+H] + |

(continued)

| Example compounds | structure | Compound name | $^1$H NMR | LC-MS (m/z) |
|---|---|---|---|---|
| **69** | 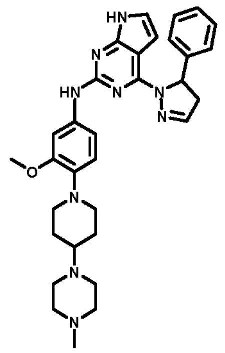 | *N*-(3-methoxy-4-(4-(4-methylpiperazin-1-yl) piperidin-1-yl)phenyl)-4-(5-phenyl-4,5-dihydro-1*H*-pyrazol-1yl)-7*H*-pyrrolo [2,3-*d*]pyrimidin-2-amine | $^1$H NMR (400 MHz, DMSO-$d_6$) δ 11.07 (s, 1H), 8.29 (s, 1H), 7.32 - 7.21 (m, 5H), 7.13 (d, *J* = 7.2 Hz, 2H), 6.86 (dd, *J* = 3.4, 2.2 Hz, 1H), 6.68 (dd, *J* = 3.4, 2.1 Hz, 1H), 6.62 (d, *J* = 9.0 Hz, 1H), 5.75 (dd, *J* = 11.9, 4.0 Hz, 1H), 3.72 (s, 3H), 3.63 - 3.52 (m, 1H), 3.32 (s, 8H), 3.26 (d, *J* = 11.6 Hz, 3H), 2.79 - 2.71 (m, 1H), 2.45 (d, *J* = 11.8 Hz, 3H), 2.16 (s, 4H), 1.80 (d, *J* = 12.0 Hz, 2H), 1.53 (q, *J* = 10.9 Hz, 3H) | 566.4 [M+H]+ |
| **70** | 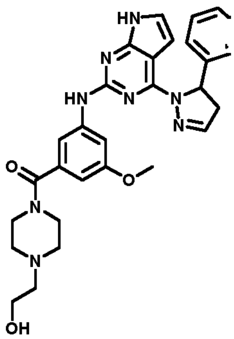 | (4-(2-hydroxyethyl)piperazin-1-yl)(3-methoxy-5-((4-(5-phenyl-4,5-dihydro-1*H*-pyrazol-1-yl)-7*H*-pyrrolo [2,3-*d*]pyrimidin-2-yl)amino) phenyl)m ethanone | | 541.3 [M+H]+ |
| **71** | 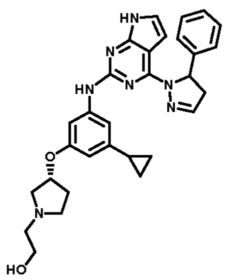 | 2-((3R)-3-(3-cyclopropyl-5-((4-(5-phenyl-4,5-dihydro-1*H*-pyrazol-1-yl)-7*H*-pyrrolo [2,3-*d*]pyrimidin-2-yl)amino) phenoxy) pyrrolidin-1-yl) ethan-1-ol | $^1$H NMR (400 MHz, DMSO-$d_6$) δ 11.19 (s, 1H), 8.35 (s, 1H), 7.43 - 7.33 (m, 1H), 7.30 - 7.23 (m, 7H), 7.18 (dt, *J* = 8.6, 4.2 Hz, 2H), 5.79 (dd, *J* = 11.8, 3.7 Hz, 1H), 3.60 (ddd, *J* = 18.4, 11.8, 1.6 Hz, 2H), 2.90 - 2.62 (m, 7H), 2.43 (dd, *J* = 15.4, 7.7 Hz, 2H), 2.32 - 2.16 (m, 2H), 1.77 - 1.69 (m, 3H), 1.54 (dt, *J* = 16.0, 8.1 Hz, 1H), 0.89 (ddt, *J* = 10.5, 8.1, 4.6 Hz, 3H), 0.67 - 0.55 (m, 2H). | 524.3 [M+H]+ |
| **72** | 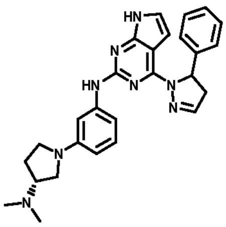 | *N*-(3-((R)-3-(dimethylamino) pyr rolidin-1-yl)phenyl)-4-(5-phenyl-4,5-dihydro-1*H*-pyrazol-1-yl)-7*H*-pyrrolo [2,3-*d*]pyrimidin-2-amine | $^1$H NMR (400 MHz, DMSO-$d_6$) δ 11.11 (t, *J* = 2.3 Hz, 1H), 8.24 (d, *J*= 1.5 Hz, 1H), 7.31 - 7.17 (m, 8H), 7.08 - 7.03 (m, 1H), 6.90 - 6.85 (m, 2H), 6.06 - 6.01 (m, 1H), 5.77 (dd, *J* = 11.9, 4.0 Hz, 1H), 3.57 (ddd, *J* = 18.4, 12.0, 1.7 Hz, 1H), 3.30 (td, *J* = 9.1, 2.4 Hz, 1H), 3.17 (s, 2H), 3.02 (ddd, *J* = 9.1, 7.8, 6.2 Hz, 1H), 2.90 - 2.69 (m, 3H), 2.23 (s, 6H), 2.14 (dtd, *J* = 11.8, 6.6, 2.2 Hz, 2H), 1.80 (dq, *J* = 12.1, 9.3 Hz, 2H). | 467.3 [M+H]+ |

(continued)

| Example compounds | structure | Compound name | $^1$H NMR | LC-MS (m/z) |
|---|---|---|---|---|
| **73** | 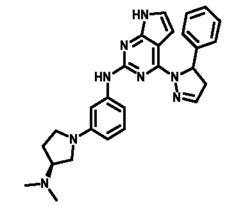 | *N*-(3-((S)-3-(dimethylamino) pyr rolidin-1-yl)phenyl)-4-(5-phenyl-4,5-dihydro-1*H*-pyrazol-1-yl)-7*H*-pyrrolo [2,3-*d*]pyrimidin-2-amine | $^1$H NMR (400 MHz, DMSO-$d_6$) δ 11.11 (d, *J* = 2.3 Hz, 1H), 8.29 (d, *J*= 1.4 Hz, 1H), 7.32 - 7.17 (m, 7H), 7.11 (dt, *J* = 8.3, 2.6 Hz, 1H), 6.93 (d, *J* = 8.1 Hz, 1H), 6.78 (q, *J* = 2.2 Hz, 1H), 6.70 (dd, *J* = 3.5, 2.0 Hz, 1H), 6.11 (dd, *J* = 8.0, 2.3 Hz, 1H), 5.76 (dd, *J* = 11.9, 4.0 Hz, 1H), 3.74 (s, 1H), 3.63 - 3.47 (m, 3H), 3.42 - 3.36 (m, 2H), 3.32 (ddd, *J* = 9.9, 6.5, 3.5 Hz, 1H), 3.23 - 3.18 (m, 1H), 2.72 (s, 6H), 2.40 - 2.30 (m, 1H), 2.12 (q, *J* = 7.3, 6.3 Hz, 1H). | 467.3 [M+H]+ |
| **74** | 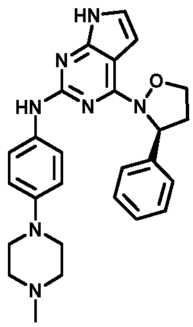 | (S)-*N*-(4-(4-methylpiperazin-1-yl)phenyl)-4-(3-pheny lisoxazolidin-2-yl)-7*H*-pyrrolo[2,3-*d*]pyrimidin-2-amine | $^1$H NMR (400 MHz, DMSO) δ 11.23 (s, 1H), 8.63 (s, 1H), 7.56 - 7.48 (m, 4H), 7.39 (dd, *J* = 8.4, 6.8 Hz, 2H), 7.31 - 7.25 (m, 1H), 6.93 (dd, *J* = 3.5, 2.2 Hz, 1H), 6.79 - 6.74 (m, 2H), 6.53 (dd, *J* = 3.5, 2.0 Hz, 1H), 5.86 (dd, *J* = 8.8, 5.1 Hz, 1H), 4.26 (td, *J* = 7.9, 3.3 Hz, 1H), 3.78 (q, *J* = 8.0 Hz, 1H), 3.01 (t, *J* = 5.0 Hz, 4H), 2.89 - 2.80 (m, 1H), 2.45 (t, *J* = 4.9 Hz, 4H), 2.31 - 2.24 (m, 1H), 2.22 (s, 3H) | 456.2 [M+H]+ |
| **75** | 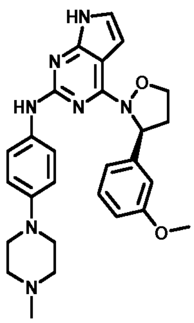 | (*S*)-4-(3-(3-methoxyphenyl) iso xazolidin-2-yl)-*N*-(4-(4-methylpiperazin-1-yl) phenyl)-7*H*-pyrrolo[2,3-*d*] pyrimidin-2-amine | $^1$H NMR (400 MHz, DMSO-$d_6$) δ 11.22 (d, *J* = 2.3 Hz, 1H), 8.67 (s, 1H), 7.58 - 7.52 (m, 2H), 7.30 (d, *J* = 7.9 Hz, 1H), 7.08 - 7.04 (m, 2H), 6.93 (dd, *J*= 3.5, 2.2 Hz, 1H), 6.86 (ddd, *J* = 8.3, 2.6, 1.0 Hz, 1H), 6.80 - 6.75 (m, 2H), 6.53 (dd, *J* = 3.5, 2.0 Hz, 1H), 5.82 - 5.77 (m, 1H), 4.27 (td, *J* = 7.8, 3.2 Hz, 1H), 3.77 (s, 3H), 3.10 (s, 4H), 2.77 (s, 3H), 2.45 (s, 3H), 2.31 - 2.22 (m, 1H), 1.91 (s, 3H) | 485.3 [M+H]+ |
| **76** | 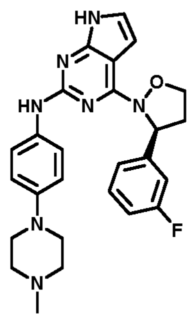 | (*S*)-4-(3-(3-fluorophenyl) isoxaz olidin-2-yl)-*N*-(4-(4-methylpiperazin-1-yl) phenyl)-7*H*-pyrrolo[2,3-*d*] pyrimidin-2-amine | $^1$H NMR (400 MHz, DMSO-$d_6$) δ 11.30 (t, *J* = 2.2 Hz, 1H), 8.79 (s, 1H), 7.60 - 7.55 (m, 2H), 7.48 - 7.43 (m, 1H), 7.36 - 7.28 (m, 2H), 7.12 (d, *J* = 2.7 Hz, 1H), 6.96 (dd, *J* = 3.5, 2.2 Hz, 1H), 6.86 - 6.80 (m, 2H), 6.54 (dd, *J* = 3.5, 2.0 Hz, 1H), 5.84 (dd, *J* = 8.7, 5.4 Hz, 1H), 4.28 (d, *J* = 3.3 Hz, 1H), 3.80 (d, *J* = 8.3 Hz, 1H), 3.66 (s, 2H), 3.51 (s, 2H), 3.18 (s, 2H), 2.88 (s, 2H), 2.87 (s, 3H), 2.86 - 2.83 (m, 1H), 2.34 - 2.25 (m, 1H) | 473.2 [M+H]+ |

(continued)

| Example compounds | structure | Compound name | $^1$H NMR | LC-MS (m/z) |
|---|---|---|---|---|
| **77** | 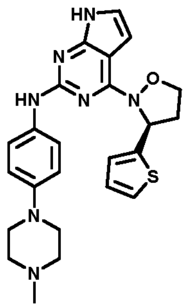 | (*S*)-*N*-(4-(4-methylpiperazin-1-yl)phenyl)-4-(3-(thiophen-2-yl)isoxazolidin-2-yl)-7*H*-pyrrolo[2,3-*d*]pyrimidin-2-amine | | 461.2 [M+H] + |
| **78** | 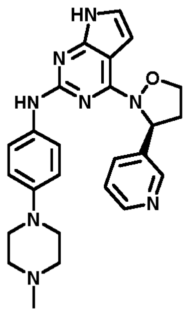 | (*S*)-*N*-(4-(4-methylpiperazin-1-yl)phenyl)-4-(3-(pyridin-3-yl)isoxazolidin-2-yl)-7*H*-pyrrolo[2,3-*d*]pyrimidin-2-amine | $^1$H NMR (400 MHz, DMSO-$d_6$) δ 11.26 (d, *J* = 2.3 Hz, 1H), 8.75 - 8.63 (m, 2H), 8.52 (dd, *J* = 4.8, 1.6 Hz, 1H), 7.88 (dd, *J* = 8.0, 2.1 Hz, 1H), 7.54 - 7.49 (m, 2H), 7.44 - 7.39 (m, 1H), 6.94 (dd, *J* = 3.5, 2.2 Hz, 1H), 6.79 - 6.74 (m, 2H), 6.53 (dd, *J* = 3.5, 2.0 Hz, 1H), 5.88 (dd, *J* = 8.8, 5.4 Hz, 1H), 4.30 (d, *J* = 3.3 Hz, 1H), 3.85 - 3.77 (m, 1H), 3.01 (t, *J* = 5.0 Hz, 4H), 2.92 - 2.83 (m, 1H), 2.45 (t, *J* = 5.0 Hz, 4H), 2.37 - 2.27 (m, 1H), 2.22 (s, 3H) | 456.2 [M+H] + |
| **79** | 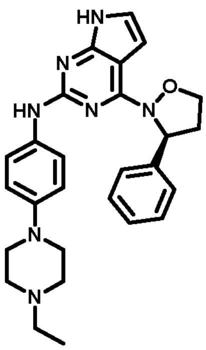 | (*S*)-*N*-(4-(4-ethylpiperazin-1-yl)phenyl)-4-(3-pheny lisoxazolidin-2-yl)-7*H*-pyrrolo[2,3-*d*]pyrimidin-2-amine | $^1$H NMR (400 MHz, DMSO-$d_6$) δ 11.23 (t, *J* = 2.2 Hz, 1H), 8.63 (s, 1H), 7.57 - 7.52 (m, 2H), 7.51 - 7.46 (m, 2H), 7.39 (dd, *J* = 8.4, 6.9 Hz, 2H), 7.31 - 7.27 (m, 1H), 6.93 (dd, *J* = 3.5, 2.2 Hz, 1H), 6.78 - 6.74 (m, 2H), 6.54 (dd, *J* = 3.5, 2.0 Hz, 1H), 5.86 (dd, *J* = 8.8, 5.2 Hz, 1H), 4.26 (td, *J* = 7.9, 3.4 Hz, 1H), 3.78 (d, *J* = 8.4 Hz, 1H), 3.35 (s, 1H), 3.02 (t, *J* = 5.0 Hz, 4H), 2.89 - 2.81 (m, 1H), 2.49 (dd, *J* = 3.9, 2.0 Hz, 3H), 2.37 (d, *J* = 7.2 Hz, 2H), 2.30 - 2.24 (m, 1H), 1.02 (d, *J* = 7.2 Hz, 3H) | 469.3 [M+H] + |

(continued)

| Example compounds | structure | Compound name | $^1$H NMR | LC-MS (m/z) |
|---|---|---|---|---|
| **80** | 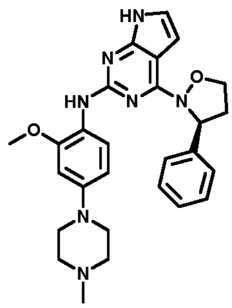 | (*S*)-*N*-(2-methoxy-4-(4-methylpiperazin-1-yl) phenyl)-4-(3-phenylisoxazolidin-2-yl)-7*H*-pyrrolo[2,3-*d*]pyrimidin-2-amine | $^1$H NMR (400 MHz, DMSO-$d_6$) δ 11.28 (t, *J* = 2.3 Hz, 1H), 8.01 (d, *J* = 8.7 Hz, 1H), 7.50 - 7.45 (m, 2H), 7.39 (dd, *J* = 8.4, 6.9 Hz, 2H), 7.31 - 7.26 (m, 1H), 7.21 (s, 1H), 6.95 (dd, *J* = 3.5, 2.3 Hz, 1H), 6.65 (d, *J* = 2.6 Hz, 1H), 6.54 (dd, *J* = 3.5, 2.0 Hz, 1H), 6.40 (dd, *J* = 8.8, 2.6 Hz, 1H), 5.83 - 5.78 (m, 1H), 4.27 (dt, *J* = 8.0, 3.9 Hz, 1H), 3.82 (s, 3H), 3.81 - 3.76 (m, 1H), 3.23 (s, 4H), 3.01 (s, 4H), 2.88 - 2.81 (m, 1H), 2.62 (s, 3H), 2.33 - 225 (m, 1H) | 485.3 [M+H]+ |
| **81** | 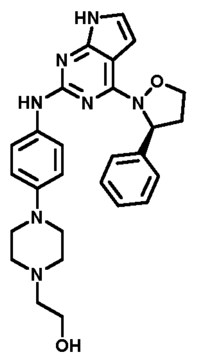 | (*S*)-2-(4-(4-((4-(3-phenylisoxazolidin-2-yl)-7*H*-pyrrolo[2,3-*d*]pyrimidin-2-yl) amino)phenyl)pi perazin-1-yl)ethan-1-ol | $^1$H NMR (400 MHz, DMSO-$d_6$) δ 11.22 (d, *J* = 2.2 Hz, 1H), 8.63 (s, 1H), 7.56 - 7.51 (m, 2H), 7.50 (dd, *J* = 7.6, 1.7 Hz, 2H), 7.39 (dd, *J* = 8.4, 6.9 Hz, 2H), 7.31 - 7.26 (m, 1H), 6.93 (dd, *J* = 3.5, 2.2 Hz, 1H), 6.79 - 6.73 (m, 2H), 6.53 (dd, *J* = 3.5, 2.0 Hz, 1H), 5.86 (dd, *J* = 8.8, 5.2 Hz, 1H), 4.46 (s, 1H), 4.26 (td, *J* = 7.9, 3.4 Hz, 1H), 3.79 (q, *J* = 8.0 Hz, 1H), 3.55 (q, *J* = 5.7 Hz, 2H), 3.02 (t, *J* = 4.9 Hz, 4H), 2.84 (dd, *J* = 7.6, 3.7 Hz, 1H), 2.59 (s, 4H), 2.47 (s, 2H), 2.31 - 2.23 (m, 1H) | 486.5 [M+H]+ |
| **82** | 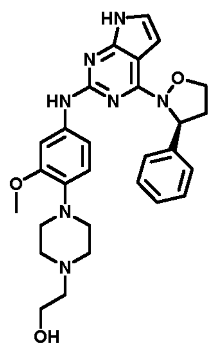 | (*S*)-2-(4-(2-methoxy-4-((4-(3-phenylisoxazolidin-2-yl)-7*H*-pyrrolo[2,3-*d*] pyrimidin-2-yl)amino) phenyl)pi perazin-1-yl)etlian-1-ol | $^1$H NMR (400 MHz, DMSO-$d_6$) δ 11.26 (d, *J* = 2.3 Hz, 1H), 8.70 (s, 1H), 7.51 - 7.47 (m, 2H), 7.43 - 7.36 (m, 3H), 7.31 - 7.26 (m, 1H), 7.23 (dd, *J* = 8.6, 2.3 Hz, 1H), 6.96 (dd, *J* = 3.5, 2.2 Hz, 1H), 6.69 (d, *J* = 8.7 Hz, 1H), 6.54 (dd, *J* = 3.5, 2.0 Hz, 1H), 5.91 (dd, *J* = 8.8, 4.8 Hz, 1H), 4.42 (s, 1H), 4.24 (td, *J* = 7.9, 3.6 Hz, 1H), 3.79 (q, *J* = 8.0 Hz, 1H), 3.65 (s, 3H), 3.53 (q, *J* = 5.8 Hz, 2H), 2.94 - 2.79 (m, 5H), 2.56 (s, 4H), 2.45 (s, 2H), 2.30 (ddt, *J* = 12.7, 8.2, 4.3 Hz, 1H) | 516.5 [M+H]+ |

(continued)

| Example compounds | structure | Compound name | $^1$H NMR | LC-MS (m/z) |
|---|---|---|---|---|
| **83** | 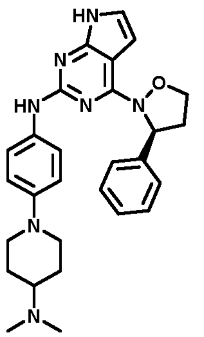 | (*S*)-*N*-(4-(4-(dimethylamino) pip eridin-1-yl)phenyl)-4-(3-phenylisoxazolidin-2-yl)-7*H*-pyrrolo[2,3-*d*]pyrimidin-2-amine | $^1$H NMR (400 MHz, DMSO-$d_6$) δ 11.59 (s, 1H), 10.11 (s, 1H), 9.32 (s, 1H), 7.52 (d, *J* = 8.5 Hz, 2H), 7.48 - 7.45 (m, 2H), 7.41 (dd, *J* = 8.5, 6.8 Hz, 2H), 7.34 - 726 (m, 1H), 7.00 - 6.95 (m, 2H), 6.58 (dd, *J* = 3.6, 2.0 Hz, 1H), 5.80 (dd, *J* = 8.7, 5.4 Hz, 1H), 4.35 (td, *J* = 7.8, 3.6 Hz, 1H), 3.92 (d, *J* = 8.1 Hz, 1H), 3.72 (d, *J* = 12.1 Hz, 2H), 3.40 - 3.30 (m, 1H), 2.96 - 2.84 (m, 3H), 2.81 (d, *J* = 4.0 Hz, 6H), 2.35 - 2.26 (m, 1H), 2.14 (d, *J* = 12.2 Hz, 2H), 1.83 (d, *J* = 11.6 Hz, 2H) | 483.3 [M+H]+ |
| **84** | 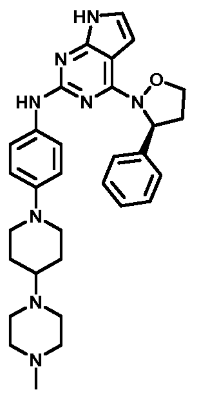 | (*S*)-*N*-(4-(4-(4-methylpiperazin-1-yl) piperidin-1-yl)phenyl)-4-(3-phenylisoxazolidin-2-yl)-7*H*-pyrrolo[2,3-*d*]pyrimidin-2-amine | $^1$H NMR (400 MHz, DMSO-$d_6$) δ 11.69 (s, 1H), 9.58 (s, 1H), 7.59 (s, 2H), 7.47 - 7.42 (m, 5H), 7.31 (d, *J* = 6.4 Hz, 2H), 7.03 (dd, *J* = 3.5, 2.3 Hz, 1H), 6.58 (s, 1H), 5.79 (dd, *J* = 8.7, 5.4 Hz, 1H), 4.39 - 4.37 (m, 1H), 3.97 (s, 1H), 3.72 (s, 8H), 3.46 (s, 5H), 2.95 (s, 1H), 2.85 (s, 3H), 2.33 - 2.29 (m, 2H) | 538.3 [M+H]+ |
| **85** | 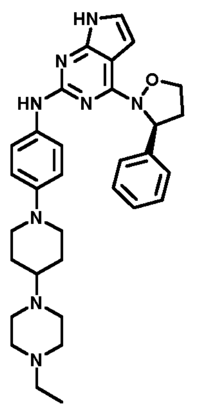 | (*S*)-*N*-(4-(4-(4-ethylpiperazin-1-yl)piperidin-1-yl)phenyl)-4-(3-phenylisoxazolidin-2-yl)-7*H*-pyrrolo[2,3-*d*]pyrimidin-2-amine | | 552.3 [M+H]+ |

(continued)

| Example compounds | structure | Compound name | $^1$H NMR | LC-MS (m/z) |
|---|---|---|---|---|
| **86** | 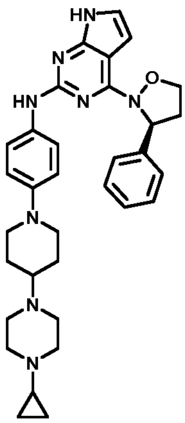 | (*S*)-*N*-(4-(4-(4-cyclopropylpiperazi n-1-yl) piperidin-1-yl)phenyl)-4-(3-phenylisoxazolidin-2-yl)-7*H*-pyrrolo[2,3-*d*]pyrimidin-2-amine | $^1$H NMR (400 MHz, DMSO-$d_6$) δ 11.24 (s, 1H), 8.61 (s, 1H), 7.54 - 7.47 (m, 4H), 7.39 (dd, *J* = 8.4, 6.9 Hz, 2H), 7.31 - 7.27 (m, 1H), 6.92 (dd, *J* = 3.5, 2.2 Hz, 1H), 6.79 - 6.73 (m, 2H), 6.53 (dd, *J* = 3.5, 1.9 Hz, 1H), 5.86 (dd, *J* = 8.8, 5.2 Hz, 1H), 4.26 (d, *J* = 3.4 Hz, 1H), 3.78 (d, *J* = 8.3 Hz, 1H), 3.54 (d, *J* = 11.6 Hz, 2H), 2.85 (td, *J* = 8.3, 4.4 Hz, 1H), 2.58 - 2.53 (m, 3H), 2.46 (s, 4H), 2.28 - 2.21 (m, 2H), 1.83 (s, 8H), 1.80 (s, 1H), 1.56 (dd, *J* = 6.7, 3.2 Hz, 1H), 1.50 (dd, *J* = 11.8, 3.8 Hz, 2H) | 564.3 [M+H]+ |
| **87** | 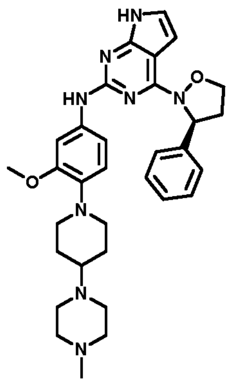 | (*S*)-*N*-(3-methoxy-4-(4-(4-methylpiperazin-1-yl) piperidin-1-yl)phenyl)-4-(3-phenylisoxazolidin-2-yl)-7*H*-pyrrolo[2,3-*d*]pyrimidin-2-amine | $^1$H NMR (400 MHz, DMSO-$d_6$) δ 11.33 (d, *J* = 2.4 Hz, 1H), 8.70 (s, 1H), 7.48 (d, *J* = 7.3 Hz, 2H), 7.42 (d, *J* = 2.4 Hz, 1H), 7.38 (t, *J* = 7.6 Hz, 2H), 7.27 (t, *J* = 7.3 Hz, 1H), 7.21 (dd, *J* = 8.6, 2.4 Hz, 1H), 6.97 - 6.93 (m, 1H), 6.54 (dd, *J* = 3.5, 2.0 Hz, 1H), 4.23 (td, *J* = 7.9, 3.6 Hz, 1H), 3.64 (s, 3H), 3.46 (d, *J* = 53.0 Hz, 8H), 3.27 (d, *J* = 10.9 Hz, 3H), 2.83 (dtd, *J* = 11.9, 8.1, 3.6 Hz, 1H), 2.48 - 2.41 (m, 5H), 2.29 (qt, *J* = 8.1, 4.6 Hz, 3H), 2.21 (s, 3H), 1.84 - 1.74 (m, 2H), 1.52 (tt, *J* = 12.5, 6.0 Hz, 2H). | 569.4 [M+H]+ |
| **88** | 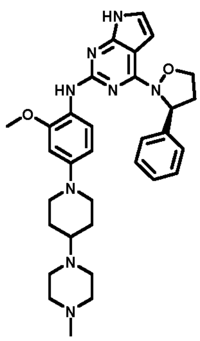 | (*S*)-*N*-(2-methoxy-4-(4-(4-methylpiperazin-1-yl) piperidin-1-yl)phenyl)-4-(3-phenylisoxazolidin-2-yl)-7*H*-pyrrolo[2,3-*d*]pyrimidin-2-amine | $^1$H NMR (400 MHz, DMSO-$d_6$) δ 7.83 (s, 1H), 7.45 - 7.37 (m, 5H), 7.33 - 7.28 (m, 1H), 7.01 (dd, *J* = 3.5, 2.3 Hz, 1H), 6.80 (s, 1H), 6.54 (s, 2H), 5.75 (d, *J* = 3.4 Hz, 2H), 4.38 (d, *J* = 3.9 Hz, 1H), 3.96 (s, 1H), 3.84 (s, 3H), 3.77 (d, *J* = 12.2 Hz, 2H), 3.50 (s, 4H), 3.12 (s, 3H), 2.94 (s, 3H), 2.82 (s, 3H), 2.36 - 2.28 (m, 1H), 2.08 (d, *J* = 5.9 Hz, 3H), 1.80 - 1.66 (m, 3H) | 568.3 [M+H]+ |

(continued)

| Example compounds | structure | Compound name | $^1$H NMR | LC-MS (m/z) |
|---|---|---|---|---|
| **89** | 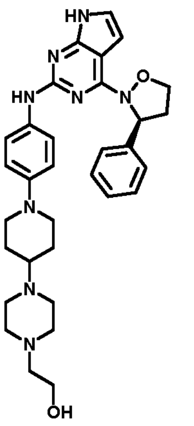 | (*S*)-2-(4-(1-(4-((4-(3-phenylisoxazolidin-2-yl)-7*H*-pyrrolo[2,3-*d*]pyrimidin-2-yl) amino)phenyl)pi peridin-4-yl) piperazin-1-yl)ethan-1-ol | $^1$H NMR (400 MHz, DMSO-$d_6$) δ 11.22 (d, *J* = 2.5 Hz, 1H), 8.61 (s, 1H), 7.54 - 7.46 (m, 4H), 7.39 (dd, *J* = 8.4, 6.9 Hz, 2H), 7.31 - 7.26 (m, 1H), 6.92 (dd, *J* = 3.5, 2.2 Hz, 1H), 6.79 - 6.73 (m, 2H), 6.53 (dd, *J* = 3.5, 2.0 Hz, 1H), 5.86 (dd, *J* = 8.8, 5.2 Hz, 1H), 4.26 (td, *J* = 7.8, 3.2 Hz, 1H), 3.82 - 3.76 (m, 1H), 3.55 (d, *J* = 11.9 Hz, 2H), 3.48 (t, *J* = 6.4 Hz, 2H), 2.88 - 2.81 (m, 1H), 2.60 - 2.51 (m, 6H), 2.46 - 2.37 (m, 4H), 2.35 (t, *J* = 6.4 Hz, 2H), 2.28 - 2.18 (m, 2H), 1.85 - 1.79 (m, 2H), 1.54 - 1.45 (m, 2H) | 569.5 [M+H]+ |
| **90** | 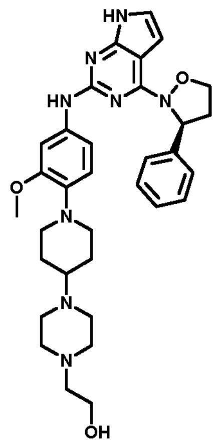 | (*S*)-2-(4-(1-(2-methoxy-4-((4-(3-phenylisoxazolidin-2-yl)-7*H*-pyrrolo[2,3-*d*] pyrimidin-2-yl)amino) phenyl)pi peridin-4-yl) piperazin-1-yl)ethan-1-ol | $^1$H NMR (400 MHz, DMSO-$d_6$) δ 11.26 (t, *J* = 2.3 Hz, 1H), 8.69 (s, 1H), 7.52 - 7.45 (m, 2H), 7.43 - 7.35 (m, 3H), 7.31 - 7.26 (m, 1H), 7.22 (dd, *J* = 8.6, 2.3 Hz, 1H), 6.96 (dd, *J* = 3.5, 2.3 Hz, 1H), 6.69 (d, *J* = 8.6 Hz, 1H), 6.54 (dd, *J* = 3.5, 2.0 Hz, 1H), 5.91 (dd, *J* = 8.8, 4.8 Hz, 1H), 4.35 (t, *J* = 5.3 Hz, 1H), 4.24 (td, *J* = 8.0, 3.6 Hz, 1H), 3.79 (q, *J* = 8.1 Hz, 1H), 3.65 (s, 3H), 3.48 (q, *J* = 6.0 Hz, 2H), 3.27 (d, *J* = 12.3 Hz, 2H), 2.84 (ddp, *J* = 11.9, 8.2, 3.6 Hz, 1H), 2.49 - 2.41 (m, 6H), 2.40 (s, 4H), 2.35 (t, *J* = 6.3 Hz, 2H), 2.30 (dt, *J* = 8.4, 4.5 Hz, 1H), 2.25 - 2.18 (m, 1H), 1.79 (d, *J* = 11.9 Hz, 2H), 1.57 - 1.46 (m, 2H) | 599.6 [M+H]+ |

(continued)

| Example compounds | structure | Compound name | $^1$H NMR | LC-MS (m/z) |
|---|---|---|---|---|
| **91** | 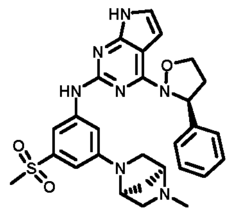 | *N*-(3-((1*S*,4*S*)-5-methyl-2,5-diazabicyclo[2.2.1] heptan-2-yl)-5-(methylsulfonyl)phenyl)-4-((*S*)-3-phenylisoxazolidin-2-yl)-7*H*-pyrrolo[2,3-*d*]pyrimidin-2-amine | $^1$H NMR (400 MHz, DMSO-$d_6$) δ 11.46 (d, *J* = 2.3 Hz, 1H), 9.63 (s, 1H), 9.22 (s, 1H), 7.75 (d, *J* = 1.7 Hz, 1H), 7.54 - 7.47 (m, 2H), 7.39 (dd, *J* = 8.4, 6.9 Hz, 2H), 7.31 - 7.26 (m, 1H), 7.06 (dd, *J* = 3.5, 2.3 Hz, 1H), 6.64 (t, *J* = 2.0 Hz, 1H), 6.58 (dd, *J* = 3.5, 2.0 Hz, 1H), 5.97 (dd, *J* = 8.8, 4.2 Hz, 1H), 4.60 (s, 1H), 4.29 (s, 1H), 4.25 (td, *J* = 7.9, 3.9 Hz, 1H), 3.83 (d, *J* = 8.1 Hz, 1H), 3.61 (dd, *J* = 11.5, 6.0 Hz, 1H), 3.54 - 3.46 (m, 1H), 3.28 (s, 1H), 3.12 (s, 3H), 3.10 (d, *J* = 3.7 Hz, 1H), 2.88 (d, *J* = 5.0 Hz, 3H), 2.84 (dd, *J* = 8.3, 4.0 Hz, 1H), 2.75 (d, *J* = 4.3 Hz, 1H), 2.41 - 2.30 (m, 2H), 2.13 (d, *J* = 11.3 Hz, 1H) | 545.2 [M+H]+ |
| **92** | 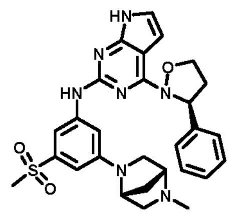 | *N*-(3-((1*R*,4*R*)-5-methyl-2,5-diazabicyclo[2.2.1] heptan-2-yl)-5-(methylsulfonyl)phenyl)-4-((*S*)-3-phenylisoxazolidin-2-yl)-7*H*-pyrrolo[2,3-*d*]pyrimidin-2-amine | $^1$H NMR (400 MHz, DMSO-$d_6$) δ 11.45 (t, *J* = 2.3 Hz, 1H), 9.60 (s, 1H), 9.21 (s, 1H), 7.74 (t, *J* = 1.7 Hz, 1H), 7.52 (dd, *J* = 8.0, 1.4 Hz, 2H), 7.39 (dd, *J* = 8.4, 6.9 Hz, 2H), 7.32 - 7.25 (m, 1H), 7.06 (dd, *J* = 3.5, 2.3 Hz, 1H), 6.64 (t, *J* = 2.0 Hz, 1H), 6.58 (dd, *J* = 3.5, 2.0 Hz, 1H), 5.98 (dd, *J* = 8.7, 4.1 Hz, 1H), 4.56 (s, 1H), 4.30 - 4.20 (m, 2H), 3.83 (q, *J* = 8.0 Hz, 1H), 3.64 - 3.54 (m, 2H), 3.34 (d, *J* = 10.8 Hz, 1H), 3.12 (s, 3H), 3.07 - 3.02 (m, 1H), 2.87 (d, *J* = 5.0 Hz, 3H), 2.83 (dt, *J* = 8.1, 3.9 Hz, 1H), 2.78 (d, *J* = 7.5 Hz, 1H), 2.36 (dt, *J* = 11.0, 3.4 Hz, 2H), 2.13 (d, *J* = 11.3 Hz, 1H) | 545.2 [M+H]+ |
| **93** | 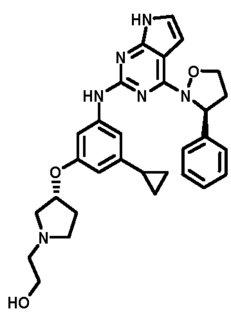 | 2-((R)-3-(3-cyclopropyl-5-((4-((S)-3-pheny lisoxazolidin-2-yl)-7*H*-pyrrolo[2,3-*d*]pyrimidin-2-yl) amino)phenoxy) pyrrolidin-1-yl)ethan-1-ol | $^1$H NMR (400 MHz, DMSO-$d_6$) δ 11.36 (d, *J* = 2.3 Hz, 1H), 8.77 (s, 1H), 7.54 - 7.49 (m, 2H), 7.38 (dd, *J* = 8.3, 6.9 Hz, 2H), 7.28 (dd, *J* = 4.7, 2.5 Hz, 2H), 6.56 (dd, *J* = 3.5, 1.9 Hz, 1H), 6.03 (t, *J* = 1.9 Hz, 1H), 5.97 (dd, *J* = 8.9, 4.4 Hz, 1H), 4.72 (ddd, *J* = 9.2, 6.5, 3.0 Hz, 1H), 4.23 (td, *J* = 8.0, 3.8 Hz, 1H), 3.80 (q, *J* = 8.1 Hz, 1H), 3.49 (t, *J* = 6.3 Hz, 2H), 3.17 (s, 1H), 2.88 - 2.76 (m, 2H), 2.76 - 2.69 (m, 1H), 2.66 (dd, *J* = 10.4, 2.8 Hz, 1H), 2.44 - 2.15 (m, 4H), 2.08 (s, 1H), 1.80 - 1.59 (m, 3H), 0.86 - 0.81 (m, 2H), 0.58 (dt, *J* = 6.5, 3.2 Hz, 2H). | 527.4 [M+H]+ |

(continued)

| Example compounds | structure | Compound name | $^1$H NMR | LC-MS (m/z) |
|---|---|---|---|---|
| **94** | 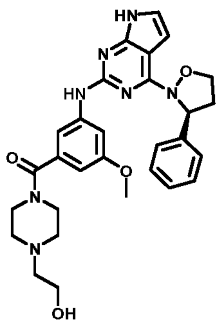 | (S)-(4-(2-hydroxyethyl)piperazin-1-yl)(3-methoxy-5-((4-(3-phenylisoxazolidin-2-yl)-7*H*-pyrrolo[2,3-*d*]pyrimidin-2-yl)amino)phenyl)m ethanone | $^1$H NMR (400 MHz, DMSO-$d_6$) δ 11.42 (s, 1H), 9.11 (s, 1H), 7.69 (t, *J* = 2.2 Hz, 1H), 7.52 - 7.47 (m, 2H), 7.38 (dd, *J* = 8.4, 6.9 Hz, 2H), 7.30 (dt, *J* = 4.4, 1.5 Hz, 1H), 7.02 (dd, *J* = 3.5, 2.3 Hz, 1H), 6.57 (dd, *J* = 3.5, 2.0 Hz, 1H), 5.94 (dd, *J* = 8.8, 4.4 Hz, 1H), 4.26 (td, *J* = 7.9, 3.7 Hz, 1H), 3.81 (q, *J* = 8.0 Hz, 2H), 3.70 (s, 3H), 3.59 (s, 2H), 3.47 - 3.31 (m, 6H), 2.84 (dtt, *J* = 12.0, 8.3, 3.8 Hz, 3H), 2.32 (dtd, *J* = 12.4, 8.3, 4.4 Hz, 1H), 2.08 (s, 1H), 1.91 (s, 4H). | 544.4 [M+H]+ |
| **95** | 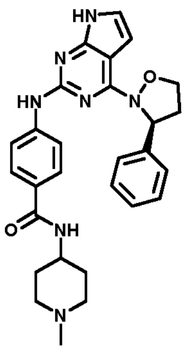 | (*S*)-*N*-(1-methylpiperidin-4-yl)-4-((4-(3-phenylisoxazolidin-2-yl)-7*H*-pyrrolo[2,3-*d*]pyrimidin-2-yl)amino)benzamid e | $^1$H NMR (400 MHz, DMSO-$d_6$) δ 11.26 (t, *J* = 2.3 Hz, 1H), 8.75 (s, 1H), 8.28 (s, 1H), 7.60 - 7.54 (m, 2H), 7.52 - 7.47 (m, 2H), 7.40 (dd, *J* = 8.4, 6.9 Hz, 2H), 7.30 (d, *J* = 7.4 Hz, 1H), 7.26 - 7.19 (m, 2H), 6.95 (dd, *J* = 3.5, 2.2 Hz, 1H), 6.54 (dd, *J*= 3.5, 2.0 Hz, 1H), 5.88 (dd, *J* = 8.8, 5.1 Hz, 1H), 4.29 - 4.23 (m, 1H), 3.79 (d, *J* = 8.3 Hz, 1H), 3.41 (t, *J* = 5.0 Hz, 4H), 3.33 (s, 4H), 2.89 - 2.81 (m, 1H), 2.31 (t, *J* = 5.0 Hz, 4H) | 497.3 [M+H]+ |
| **96** | 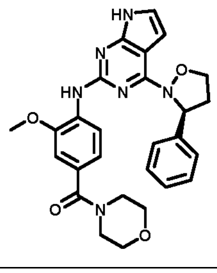 | (S)-(3-methoxy-4-((4-(3-phenylisoxazolidin-2-yl)-7*H*-pyrrolo[2,3-*d*]pyrimidin-2-yl)amino)phenyl)(m orpholino)methano ne | | 501.2 [M+H]+ |
| **97** | 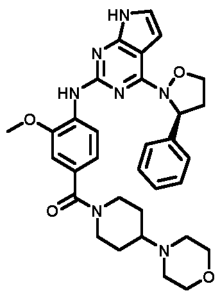 | (*S*)-(3-methoxy-4-((4-(3-phenylisoxazolidin-2-yl)-7*H*-pyrrolo[2,3-*d*]pyrimidin-2-yl)amino)phenyl)(4 morpholinopiperidi n-1-yl)methanone | | 584.3 [M+H]+ |

(continued)

| Example compounds | structure | Compound name | $^1$H NMR | LC-MS (m/z) |
|---|---|---|---|---|
| **98** | 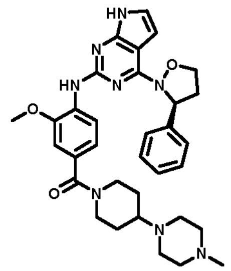 | (*S*)-(3-methoxy -4-((4-(3-phenylisoxazolidin-2-yl)-7*H*-pyrrolo[2,3-*d*]pyrimidin-2-yl) amino)phenyl)(4 -(4-methylpiperazin-1-yl) piperidin-1-yl)methanone | $^1$H NMR (400 MHz, DMSO-$d_6$) δ 11.43 (d, *J* = 2.3 Hz, 1H), 8.36 (d, *J* = 8.3 Hz, 1H), 7.53 - 7.48 (m, 3H), 7.41 (dd, *J* = 8.4, 6.9 Hz, 2H), 7.31 (d, *J* = 7.4 Hz, 1H), 7.04 (dd, *J* = 3.5, 2.3 Hz, 1H), 6.99 (d, *J* = 1.8 Hz, 1H), 6.83 - 6.78 (m, 1H), 6.59 (dd, *J* = 3.5, 2.0 Hz, 1H), 5.82 - 5.75 (m, 1H), 4.31 (d, *J* = 3.2 Hz, 1H), 3.88 (s, 3H), 3.83 (d, *J* = 8.3 Hz, 1H), 3.33 (s, 3H), 2.97 (s, 3H), 2.90 (ddt, *J* = 8.5, 4.9, 2.8 Hz, 3H), 2.78 (s, 3H), 2.28 (ddd, *J* = 9.4, 5.7, 4.1 Hz, 2H), 1.78 (s, 2H), 1.38 (s, 2H), 1.28 - 1.20 (m, 2H) | 596.3 [M+H]$^+$ |
| **99** | 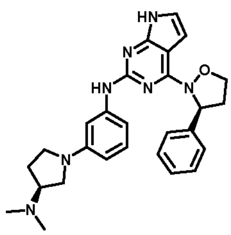 | *N*-(3-((S)-3-(dimethylamino) pyr rolidin-1-yl)phenyl)-4-((S)-3-phenylisoxazolidin-2-yl)-7*H*-pyrrolo[2,3-*d*] pyrimidin-2-amine | $^1$H NMR (400 MHz, DMSO-$d_6$) δ 11.30 (s, 1H), 8.64 (s, 1H), 7.51 - 7.48 (m, 2H), 7.41 - 7.35 (m, 3H), 7.30 - 7.25 (m, 2H), 7.10 (dd, *J* = 7.5, 1.7 Hz, 1H), 6.93 (d, *J* = 8.0 Hz, 1H), 6.55 (d, *J* = 3.4 Hz, 1H), 6.06 (ddd, *J* = 8.2, 2.4, 0.9 Hz, 1H), 5.95 (dd, *J* = 8.8, 4.5 Hz, 1H), 4.23 (td, *J* = 8.0, 3.8 Hz, 1H), 3.80 (q, *J* = 8.0 Hz, 1H), 3.36 (dd, *J* = 9.1, 7.2 Hz, 1H), 3.25 (td, *J* = 9.0, 2.3 Hz, 1H), 3.12 (td, *J* = 9.2, 6.7 Hz, 1H), 3.00 (dd, *J* = 9.1, 7.7 Hz, 1H), 2.88 - 2.68 (m, 3H), 2.31 (dtd, *J* = 12.5, 8.2, 4.5 Hz, 1H), 2.19 (s, 6H), 2.06 - 2.01 (m, 1H), 1.78 - 1.71 (m, 1H). | 470.4 [M+H]$^+$ |
| **100** | 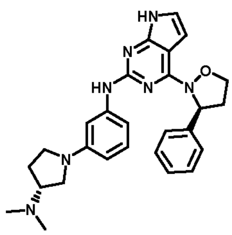 | *N*-(3-((R)-3-(dimethylamino) pyr rolidin-1-yl)phenyl)-4-((S)-3-phenylisoxazolidin-2-yl)-7*H*-pyrrolo[2,3-*d*] pyrimidin-2-amine | $^1$H NMR (400 MHz, DMSO-$d_6$) δ 11.13 (s, 1H), 9.12 (s, 1H), 7.50 (d, *J* = 7.7 Hz, 2H), 7.39 (t, *J* = 7.6 Hz, 2H), 7.28 (t, *J* = 7.3 Hz, 1H), 7.16 (dd, *J* = 8.0, 1.8 Hz, 1H), 7.10 (d, *J* = 2.2 Hz, 1H), 6.56 (t, *J* = 2.7 Hz, 1H), 6.15 (dd, *J* = 8.1, 2.3 Hz, 1H), 5.93 (dd, *J* = 8.8, 4.7 Hz, 1H), 3.99 - 3.93 (m, 1H), 3.81 (q, *J* = 8.0 Hz, 2H), 3.58 (ddq, *J* = 10.5, 6.5, 3.2 Hz, 5H), 3.34 (td, *J* = 9.2, 3.2 Hz, 1H), 2.81 (s, 6H), 2.38 - 2.13 (m, 4H). | 470.4 [M+H]$^+$ |

(continued)

| Example compounds | structure | Compound name | $^1$H NMR | LC-MS (m/z) |
|---|---|---|---|---|
| **101** | 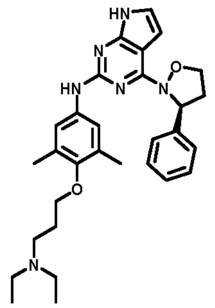 | (*S*)-*N*-(4-(3-(diethylamino) prop oxy)-3,5-dimethylphenyl)-4-(3-phenylisoxazolidin-2-yl)-7*H*-pyrrolo[2,3-*d*]pyrimidin-2-amine | $^1$H NMR (400 MHz, DMSO-$d_6$) δ 11.25 (t, *J* = 2.2 Hz, 1H), 8.64 (s, 1H), 7.52 - 7.46 (m, 2H), 7.38 (dd, *J* = 8.4, 6.9 Hz, 2H), 7.33 (s, 2H), 7.30 - 7.25 (m, 1H), 6.95 (dd, *J* = 3.5, 2.2 Hz, 1H), 6.54 (dd, *J* = 3.5, 2.0 Hz, 1H), 5.93 (dd, *J* = 8.9, 4.9 Hz, 1H), 4.25 (td, *J* = 7.9, 3.4 Hz, 1H), 3.82 - 3.74 (m, 1H), 3.67 (t, *J* = 6.3 Hz, 2H), 2.92 - 2.83 (m, 1H), 2.57 (dd, *J* = 7.9, 6.4 Hz, 2H), 2.49 - 2.45 (m, 4H), 2.30 (ddt, *J* = 12.1, 8.2, 4.1 Hz, 1H), 1.88 (s, 6H), 1.80 (dt, *J* = 12.9, 6.6 Hz, 2H), 0.96 (t, *J* = 7.1 Hz, 6H) | 515.5 [M+H]+ |
| **102** | 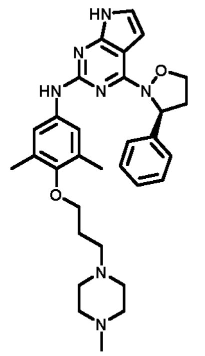 | (*S*)-*N*-(3,5-dimethyl-4-(3-(4-methylpiperazin-1-yl) propoxy)phenyl) -4-(3-phenylisoxazolidin-2-yl)-7*H*-pyrrolo[2,3-d]pyrimidin-2-amine | $^1$H NMR (400 MHz, DMSO-$d_6$) δ 11.24 (t, *J* = 2.2 Hz, 1H), 8.65 (s, 1H), 7.49 (dd, *J* = 8.0, 1.4 Hz, 2H), 7.38 (dd, *J* = 8.4, 6.9 Hz, 2H), 7.33 (s, 2H), 7.29 (d, *J* = 7.3 Hz, 1H), 6.96 (dd, *J* = 3.5, 2.3 Hz, 1H), 6.54 (dd, *J* = 3.4, 2.0 Hz, 1H), 5.93 (dd, *J* = 8.9, 4.9 Hz, 1H), 4.25 (td, *J* = 8.0, 3.4 Hz, 1H), 3.78 (d, *J* = 8.3 Hz, 1H), 3.66 (t, *J* = 6.2 Hz, 2H), 3.61 (t, *J* = 6.2 Hz, 1H), 2.92 - 2.83 (m, 1H), 2.43 (s, 5H), 2.32 - 2.27 (m, 1H), 2.23 (d, *J* = 2.3 Hz, 4H), 2.06 (s, 9H), 1.85 - 1.81 (m, 2H) | 541.3 [M+H]+ |
| **103** | 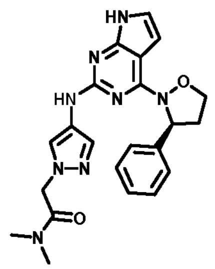 | (*S*)-*N,N*-dimethyl-2-(4-((4-(3-pheny lisoxazolidin-2-yl)-7*H*-pyrrolo[2,3-*d*]pyrimidin-2-yl) amino)-1*H*-pyrazol-1-yl) acetamide | $^1$H NMR (400 MHz, DMSO-$d_6$) δ 11.22 (s, 1H), 8.81 (s, 1H), 7.51 - 7.46 (m, 2H), 7.42 - 7.35 (m, 3H), 7.33 - 7.22 (m, 2H), 6.92 (dd, *J* = 3.5, 2.2 Hz, 1H), 6.53 (dd, *J* = 3.6, 2.0 Hz, 1H), 5.83 (t, *J* = 7.2 Hz, 1H), 4.28 (q, *J* = 5.9 Hz, 1H), 3.84 - 3.77 (m, 1H), 3.64 - 3.59 (m, 1H), 3.13 (td, *J* = 7.4, 4.3 Hz, 1H), 2.54 (s, 6H), 2.09 (s, 2H) | 433.3 [M+H]+ |
| **104** | 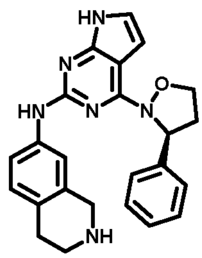 | (*S*)-*N*-(4-(3-phenylisoxazolidin-2-yl)-7*H*-pyrrolo[2,3-*d*]pyrimidin-2-yl)-1,2,3,4-tetrahydroisoquinolin-7-amine | | 412.2 [M+H]+ |

(continued)

| Example compounds | structure | Compound name | $^1$H NMR | LC-MS (m/z) |
|---|---|---|---|---|
| **105** | 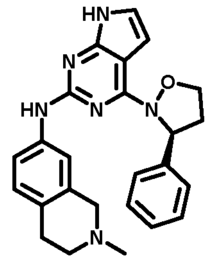 | (*S*)-2-methyl-*N*-(4-(3-phenylisoxazolidin-2-yl)-7*H*-pyrrolo[2,3-*d*]pyrimidin-2-yl)-1,2,3,4-tetrahydroisoquinolin-7-amine | $^1$H NMR (400 MHz, DMSO-$d_6$) δ 11.27 (t, *J* = 2.2 Hz, 1H), 8.78 (s, 1H), 7.52 - 7.47 (m, 3H), 7.42 - 7.34 (m, 3H), 7.32 - 7.27 (m, 1H), 6.97 (dd, *J* = 3.5, 2.2 Hz, 1H), 6.86 (d, *J* = 8.3 Hz, 1H), 6.55 (dd, *J* = 3.5, 2.0 Hz, 1H), 5.89 (dd, *J* = 8.9, 5.2 Hz, 1H), 4.27 (td, *J* = 7.9, 3.2 Hz, 1H), 3.81 - 3.75 (m, 1H), 3.16 (q, *J* = 14.9 Hz, 3H), 2.94 - 2.84 (m, 1H), 2.69 (t, *J* = 5.8 Hz, 2H), 2.53 (s, 1H), 2.33 - 2.29 (m, 1H), 2.28 (s, 3H) | 426.2 [M+H]+ |
| **106** | 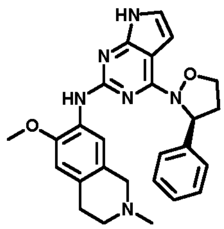 | (*S*)-6-methoxy-2-methyl-*N*-(4-(3-phenylisoxazolidin-2-yl)-7*H*-pyrrolo[2,3-*d*]pyrimidin-2-yl)-1,2,3,4-tetrahydroisoquinoli n-7-amine | $^1$H NMR (400 MHz, DMSO-$d_6$) δ 11.32 (d, *J* = 2.6 Hz, 1H), 7.95 (s, 1H), 7.51 - 7.46 (m, 2H), 7.40 (dd, *J* = 8.4, 6.8 Hz, 2H), 7.32 - 7.27 (m, 2H), 6.99 (dd, *J* = 3.5, 2.2 Hz, 1H), 6.67 (s, 1H), 6.57 (dd, *J* = 3.4, 2.0 Hz, 1H), 5.86 (dd, *J* = 8.9, 5.4 Hz, 1H), 4.30 - 4.26 (m, 1H), 3.80 (s, 3H), 3.79 - 3.75 (m, 1H), 3.09 (d, *J* = 14.6 Hz, 1H), 3.00 - 2.86 (m, 3H), 2.71 (t, *J* = 5.9 Hz, 2H), 2.48 (s, 1H), 2.33 - 2.29 (m, 1H), 2.27 (s, 3H) | 456.2 [M+H]+ |
| **107** | 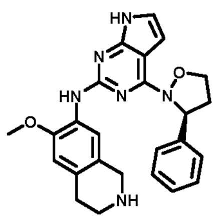 | (*S*)-6-methoxy-*N*-(4-(3 -phenylisoxazolidin-2-yl)-7*H*-pyrrolo[2,3-*d*]pyrimidin-2-yl)-1,2,3,4-tetrahydroisoquinolin-7-amine | | 442.2 [M+H]+ |
| **108** | 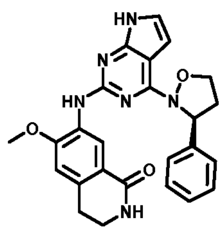 | (*S*)-6-methoxy-7-((4-(3-phenylisoxazolidin-2-yl)-7*H*-pyrrolo[2,3-*d*]pyrimidin-2-yl) amino)-3,4-dihydroisoquinolin-1 (2*H*)-one | $^1$H NMR (400 MHz, DMSO-$d_6$) δ 11.45 (s, 1H), 8.74 (s, 1H), 7.73 (t, *J* = 2.8 Hz, 1H), 7.54 - 7.48 (m, 2H), 7.42 (s, 1H), 7.37 - 7.31 (m, 2H), 7.28 - 7.23 (m, 1H), 6.98 (d, *J* = 3.5 Hz, 1H), 6.92 (s, 1H), 6.57 (d, *J* = 3.4 Hz, 1H), 5.95 (dd, *J* = 8.8, 4.4 Hz, 1H), 4.27 (td, *J* = 7.9, 3.9 Hz, 1H), 3.89 (s, 3H), 3.82 (q, *J* = 8.0 Hz, 1H), 3.36 (td, *J* = 6.5, 2.8 Hz, 2H), 2.86 (t, *J* = 6.5 Hz, 2H), 2.83 - 2.77 (m, 1H), 2.35 - 2.27 (m, 1H) | 456.2 [M+H]+ |

(continued)

| Example compounds | structure | Compound name | $^1$H NMR | LC-MS (m/z) |
|---|---|---|---|---|
| **109** | 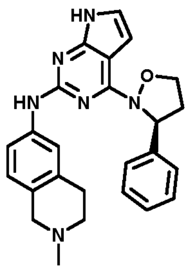 | (*S*)-2-methyl-*N*-(4-(3-phenylisoxazolidin-2-yl)-7*H*-pyrrolo[2,3-*d*]pyrimidin-2-yl)-1,2,3,4-tetrahydroisoquinolin-6-amine | $^1$H NMR (400 MHz, DMSO-$d_6$) δ 11.30 (s, 1H), 8.77 (s, 1H), 7.50 - 7.35 (m, 6H), 7.31 - 7.26 (m, 1H), 6.96 (dd, *J* = 3.6, 2.0 Hz, 1H), 6.80 (d, *J* = 8.3 Hz, 1H), 6.55 (dd, *J* = 3.5, 1.7 Hz, 1H), 5.90 (dd, *J* = 8.9, 5.0 Hz, 1H), 4.26 (td, *J* = 7.9, 3.3 Hz, 1H), 3.78 (dd, *J* = 8.8, 7.4 Hz, 1H), 3.37 (s, 2H), 2.94 - 2.83 (m, 1H), 2.61 (t, *J* = 5.8 Hz, 2H), 2.53 (s, 2H), 2.30 (s, 3H), 2.27 (d, *J* = 6.2 Hz, 1H) | 426.2 [M+H]+ |
| **110** | 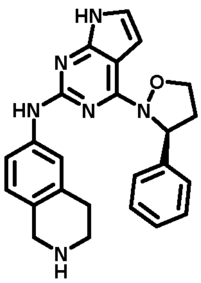 | (*S*)-*N*-(4-(3-phenylisoxazolidin-2-yl)-7*H*-pyrrolo[2,3-*d*]pyrimidin-2-yl)-1,2,3,4-tetrahydroisoquinolin-6-amine | | 412.2 [M+H]+ |
| **111** | 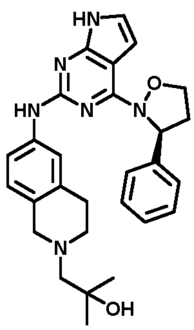 | (*S*)-2-methyl-1-(6-((4-(3-phenylisoxazolidin-2-yl)-7*H*-pyrrolo[2,3-*d*]pyrimidin-2-yl)amino)-3,4-dihydroisoquinolin-2(1*H*)-yl)propan-2-ol | | 484.3 [M+H]+ |
| **112** | 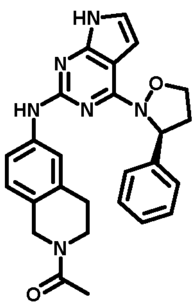 | (*S*)-1-(6-((4-(3-phenylisoxazolidin-2-yl)-7*H*-pyrrolo[2,3-*d*]pyrimidin-2-yl)amino)-3,4-dihydroisoquinolin-2(1*H*)-yl)ethan-1-one | | 454.2 [M+H]+ |

(continued)

| Example compounds | structure | Compound name | $^1$H NMR | LC-MS (m/z) |
|---|---|---|---|---|
| **113** | 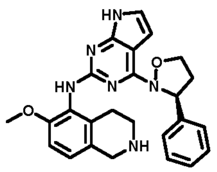 | (*S*)-6-methoxy-*N*-(4-(3 -phenylisoxazolidin-2-yl)-7*H*-pyrrolo[2,3-*d*]pyrimidin-2-yl)-1,2,3,4-tetrahydroisoquinoli n-5-amine | $^1$H NMR (400 MHz, DMSO-$d_6$) δ 11.21 (s, 1H), 8.99 (s, 2H), 7.95 (s, 1H), 7.34 (d, *J* = 4.6 Hz, 3H), 7.28 - 7.24 (m, 1H), 7.14 - 7.09 (m, 1H), 7.00 (d, *J* = 8.6 Hz, 1H), 6.87 (dd, *J* = 3.5, 2.2 Hz, 1H), 6.49 (d, *J* = 3.2 Hz, 1H), 5.74 (d, *J* = 15.5 Hz, 1H), 4.28 (dt, *J* = 7.9, 3.8 Hz, 1H), 4.22 (d, *J* = 4.9 Hz, 2H), 3.82 (q, *J* = 7.9 Hz, 1H), 3.28 (s, 1H), 3.18 (d, *J* = 9.5 Hz, 1H), 2.84 (d, *J* = 5.2 Hz, 3H), 2.29 (ddd, *J* = 12.9, 10.6, 6.6 Hz, 1H) | 442.2 [M+H] + |
| **114** | 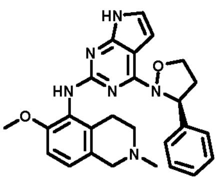 | (*S*)-6-methoxy-2-methyl-*N*-(4-(3-phenylisoxazolidin-2-yl)-7*H*-pyrrolo[2,3-*d*] pyrimidin-2-yl)-1,2,3,4-tetrahydroisoquinoli n-5-amine | $^1$H NMR (400 MHz, DMSO-$d_6$) δ 11.20 (s, 1H), 7.45 (s, 1H), 7.40 (d, *J* = 7.7 Hz, 2H), 7.34 (t, *J* = 7.6 Hz, 2H), 7.26 (d, *J* = 7.2 Hz, 1H), 6.90 (d, *J* = 8.5 Hz, 1H), 6.84 - 6.78 (m, 2H), 6.47 (dd, *J* = 3.5, 1.8 Hz, 1H), 5.79 (d, *J* = 7.4 Hz, 1H), 4.23 (td, *J* = 7.9, 3.5 Hz, 1H), 3.76 (d, *J* = 8.3 Hz, 1H), 3.64 (s, 3H), 3.42 (s, 2H), 2.83 - 2.73 (m, 1H), 2.72 - 2.63 (m, 2H), 2.48 - 2.40 (m, 2H), 2.29 (s, 3H), 2.26 (s, 1H) | 456.2 [M+H] + |
| **115** | 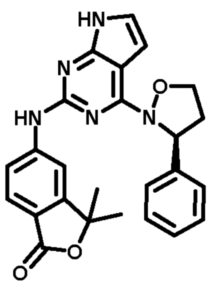 | (*S*)-3,3-dimethyl-5-((4-(3-pheny lisoxazolidin-2-yl)-7*H*-pyrrolo[2,3-*d*]pyrimidin-2-yl) amino)isobenzof uran-1 (3H)-one | $^1$H NMR (400 MHz, DMSO-$d_6$) δ 11.51 (t, *J* = 2.3 Hz, 1H), 9.69 (s, 1H), 8.14 (d, *J* = 1.8 Hz, 1H), 7.70 (dd, *J* = 8.5, 1.8 Hz, 1H), 7.54 - 7.51 (m, 3H), 7.41 (dd, *J* = 8.4, 6.9 Hz, 2H), 7.33 - 7.29 (m, 1H), 7.10 (dd, *J* = 3.5, 2.2 Hz, 1H), 6.62 (dd, *J* = 3.5, 1.9 Hz, 1H), 5.94 (dd, *J* = 8.9, 4.9 Hz, 1H), 4.28 (td, *J* = 7.9, 3.4 Hz, 1H), 3.88 - 3.80 (m, 1H), 3.00 - 2.91 (m, 1H), 2.35 (dt, *J* = 8.6, 4.6 Hz, 1H), 1.43 (s, 3H), 1.34 (s, 3H) | 441.2 [M+H] + |
| **116** | 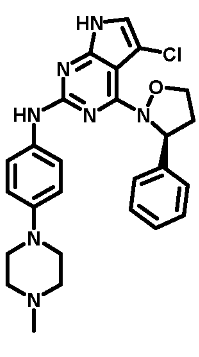 | (*S*)-5-chloro-*N*-(4-(4-methylpiperazin-1-yl) phenyl)-4-(3-pheny lisoxazolidin-2-yl)-7*H*-pyrrolo[2,3-*d*]pyrimidin-2-amine | $^1$H NMR (400 MHz, DMSO-$d_6$) δ 11.63 - 11.49 (m, 1H), 8.82 (s, 1H), 7.57 - 7.50 (m, 2H), 7.49 (dd, *J* = 7.6, 1.5 Hz, 2H), 7.38 (dd, *J* = 8.4, 6.9 Hz, 2H), 7.31 - 7.24 (m, 1H), 7.11 (d, *J* = 2.1 Hz, 1H), 6.82 - 6.74 (m, 2H), 5.86 (dd, *J* = 8.6, 4.7 Hz, 1H), 4.17 (td, *J* = 7.7, 4.0 Hz, 1H), 3.86 (q, *J* = 7.9 Hz, 1H), 3.03 (t, *J* = 5.0 Hz, 4H), 2.78 (dtd, *J* = 12.1, 8.1, 4.1 Hz, 1H), 2.45 (t, *J* = 5.0 Hz, 4H), 2.31 - 2.25 (m, 1H), 2.22 (s, 3H) | 490.3 [M+H] + |

(continued)

| Example compounds | structure | Compound name | $^1$H NMR | LC-MS (m/z) |
|---|---|---|---|---|
| **117** | 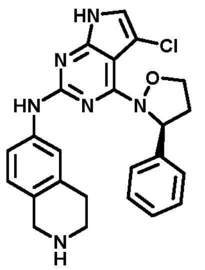 | (*S*)-*N*-(5-chloro-4-(3-phenylisoxazolidin-2-yl)-7*H*-pyrrolo[2,3-*d*]pyrimidin-2-yl)-1,2,3,4-tetrahydroisoquinolin-6-amine | $^1$H NMR (400 MHz, DMSO-$d_6$) δ 11.63 (s, 1H), 8.98 (s, 1H), 7.49 (d, *J* = 7.5 Hz, 4H), 7.39 (dd, *J* = 8.4, 6.9 Hz, 2H), 7.30 - 7.25 (m, 1H), 7.16 (d, *J* = 1.3 Hz, 1H), 6.90 (dd, *J* = 29.1, 8.3 Hz, 1H), 5.90 (dd, *J* = 8.6, 4.5 Hz, 1H), 4.18 (td, *J* = 7.7, 4.0 Hz, 1H), 3.95 - 3.78 (m, 3H), 3.01 (t, *J* = 5.9 Hz, 2H), 2.80 (dq, *J* = 12.2, 4.2 Hz, 1H), 2.69 - 2.57 (m, 2H), 2.34 - 2.27 (m, 1H) | 447.2 [M+H]+ |
| **118** | 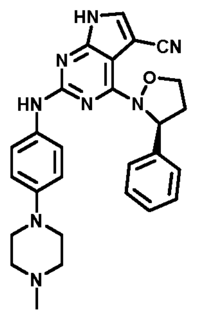 | (*S*)-2-((4-(4-methylpiperazin-1-yl)phenyl)amino)-4-(3-phenylisoxazolidin-2-yl)-7*H*-pyrrolo[2,3-*d*]pyrimidine-5-carbo nitrile | $^1$H NMR (400 MHz, DMSO-$d_6$) δ 12.25 (s, 1H), 8.89 (s, 1H), 7.97 (s, 1H), 7.51 - 7.43 (m, 4H), 7.43 - 7.38 (m, 2H), 7.32 - 7.28 (m, 1H), 6.75 (d, *J* = 9.0 Hz, 2H), 5.83 (t, *J* = 7.3 Hz, 1H), 4.32 (td, *J* = 7.8, 3.1 Hz, 1H), 3.85 (q, *J* = 8.1 Hz, 1H), 3.02 (t, *J* = 5.1 Hz, 4H), 2.94 - 2.86 (m, 1H), 2.45 (t, *J* = 5.0 Hz, 4H), 2.33 - 2.27 (m, 1H), 2.22 (s, 3H) | 481.3 [M+H]+ |
| **119** | 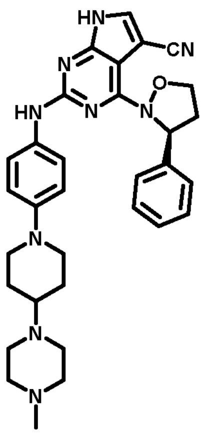 | (*S*)-2-((4-(4-(4-methylpiperazin-1-yl)piperidin-1-yl)phenyl)amino)-4-(3-phenylisoxazolidin-2-yl)-7*H*-pyrrolo[2,3-*d*]pyrimidine-5-carbonitrile | $^1$H NMR (400 MHz, DMSO-$d_6$) δ 12.34 (s, 1H), 9.16 (s, 1H), 8.02 (d, *J* = 2.7 Hz, 1H), 7.60 (d, *J* = 8.5 Hz, 2H), 7.52 - 7.46 (m, 2H), 7.42 (dd, *J* = 8.4, 6.9 Hz, 2H), 7.33 - 7.28 (m, 1H), 7.04 (s, 2H), 5.84 - 5.78 (m, 1H), 4.34 (q, *J* = 4.7 Hz, 1H), 3.92 - 3.83 (m, 1H), 3.66 (d, *J* = 11.9 Hz, 2H), 3.50 (s, 5H), 2.99 (s, 5H), 2.95 - 2.88 (m, 2H), 2.82 (s, 3H), 2.31 (ddt, *J* = 9.2, 5.6, 2.9 Hz, 1H), 2.07 (d, *J* = 12.4 Hz, 2H), 1.77 (d, *J* = 12.3 Hz, 2H) | 563.3 [M+H]+ |
| **120** | 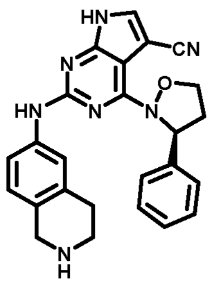 | (*S*)-4-(3-phenylisoxazolidin-2-yl)-2-((1,2,3,4-tetrahydroisoquinolin-6-yl)amino)-7*H*-pyrrolo[2,3-*d*]pyrimidine-5-carbo nitrile | $^1$H NMR (400 MHz, DMSO-$d_6$) δ 8.99 (s, 1H), 8.00 (s, 1H), 7.51 - 7.46 (m, 2H), 7.39 (ddd, *J* = 13.9, 6.5, 2.1 Hz, 4H), 7.32 - 7.27 (m, 1H), 6.78 (d, *J* = 8.2 Hz, 1H), 5.89 (dd, *J* = 8.8, 5.5 Hz, 1H), 4.31 (td, *J* = 7.9, 3.2 Hz, 1H), 3.85 (dt, *J* = 8.9, 7.4 Hz, 1H), 3.75 (s, 2H), 2.97 - 2.90 (m, 1H), 2.88 (t, *J* = 5.9 Hz, 2H), 2.47 (d, *J* = 4.7 Hz, 2H), 2.36 - 2.29 (m, 1H) | 438.3 [M+H]+ |

(continued)

| Example compounds | structure | Compound name | $^1$H NMR | LC-MS (m/z) |
|---|---|---|---|---|
| **121** | 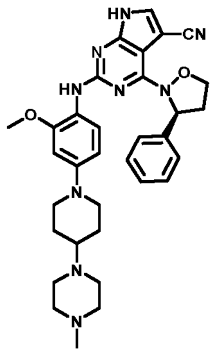 | (*S*)-2-((2-methoxy-4-(4-(4-methylpiperazin-1-yl) piperidin-1-yl)phenyl) amino)-4-(3-pheny lisoxazolidin-2-yl)-7*H*-pyrrolo[2,3-*d*]pyrimidine-5-carbonitrile | $^1$H NMR (400 MHz, DMSO-$d_6$) δ 12.31 (s, 1H), 7.97 (s, 1H), 7.54 (s, 1H), 7.47 - 7.42 (m, 2H), 7.39 (dd, *J* = 8.4, 6.8 Hz, 2H), 7.32 - 7.25 (m, 2H), 6.68 (s, 1H), 6.42 (s, 1H), 5.79 - 5.74 (m, 1H), 4.33 (d, *J* = 3.1 Hz, 1H), 3.88 - 3.82 (m, 1H), 3.79 (s, 3H), 3.72 (d, *J* = 12.2 Hz, 2H), 3.39 (s, 5H), 3.05 - 2.86 (m, 4H), 2.74 (s, 6H), 2.35 - 2.26 (m, 1H), 1.98 (s, 2H), 1.63 (d, *J* = 12.3 Hz, 2H) | 593.3 [M+H] + |
| **122** | 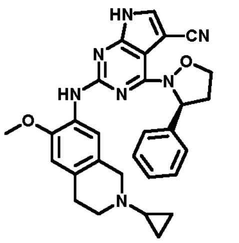 | (*S*)-2-((2-cyclopropyl-6-methoxy-1,2,3,4-tetrahydroisoquinoli n-7-yl) amino)-4-(3-pheny lisoxazolidin-2-yl)-7H-pyrrolo[2,3-*d*]pyrimidine-5-carbonitrile | $^1$H NMR (400 MHz, DMSO-$d_6$) δ 12.28 (s, 1H), 8.03 (s, 1H), 7.81 (s, 1H), 7.53 (s, 1H), 7.49 - 7.45 (m, 2H), 7.40 (dd, *J* = 8.4, 6.8 Hz, 2H), 7.33 - 7.29 (m, 1H), 6.67 (s, 1H), 5.86 (dd, *J* = 8.8, 5.6 Hz, 1H), 4.33 (dd, *J* = 7.9, 3.1 Hz, 1H), 3.90 - 3.83 (m, 1H), 3.78 (s, 3H), 3.40 - 3.21 (m, 2H), 2.95 (dt, *J* = 8.4, 2.4 Hz, 1H), 2.71 (dd, *J* = 23.4, 5.6 Hz, 4H), 2.37 - 2.28 (m, 1H), 1.72 (dt, *J* = 6.5, 3.1 Hz, 1H), 0.54 - 0.48 (m, 2H), 0.35 (dq, *J* = 6.1, 2.9, 2.5 Hz, 2H) | 507.2 [M+H] + |
| **123** | 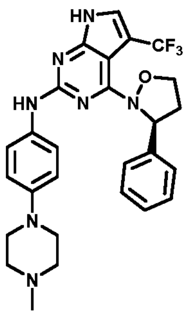 | (*S*)-*N*-(4-(4-methylpiperazin-1-yl)phenyl)-4-(3-pheny lisoxazolidin-2-yl)-5-(trifluoromethyl)-7*H*-pyrrolo[2,3-*d*]pyrimidin-2-amine | $^1$H NMR (400 MHz, DMSO-$d_6$) δ 11.99 (s, 1H), 8.86 (s, 1H), 7.59 (d, *J* = 2.5 Hz, 1H), 7.48 (dd, *J* = 8.5, 6.4 Hz, 4H), 7.39 (dd, *J* = 8.4, 6.8 Hz, 2H), 7.31 - 7.26 (m, 1H), 6.78 (d, *J* = 9.1 Hz, 2H), 6.03 - 5.96 (m, 1H), 4.15 (dt, *J* = 7.9, 4.0 Hz, 1H), 3.64 (q, *J* = 8.1 Hz, 1H), 3.03 (t, *J* = 5.0 Hz, 4H), 2.87 - 2.79 (m, 1H), 2.47 (t, *J* = 4.6 Hz, 4H), 2.23 (s, 4H) | 524.3 [M+H] + |
| **124** | 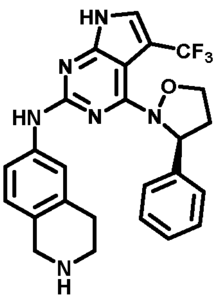 | (*S*)-*N*-(4-(3-pheny lisoxazolidin-2-yl)-5-(trifluoromethyl)-7*H*-pyrrolo[2,3-*d*]pyrimidin-2-yl)-1,2,3,4-tetrahydroisoquinoli n-6-amine | $^1$H NMR (400 MHz, DMSO-$d_6$) δ 12.11 (s, 1H), 9.19 (s, 1H), 9.00 (d, *J* = 14.5 Hz, 1H), 7.68 (s, 1H), 7.54 (d, *J* = 9.2 Hz, 2H), 7.50 - 7.45 (m, 2H), 7.43 - 7.38 (m, 2H), 7.32 - 7.27 (m, 1H), 7.00 (d, *J* = 8.3 Hz, 1H), 6.03 (dd, *J* = 8.8, 5.6 Hz, 1H), 4.17 (d, *J* = 10.0 Hz, 3H), 3.69 - 3.62 (m, 1H), 3.30 (d, *J* = 6.3 Hz, 2H), 2.87 (dtt, *J* = 8.8, 6.5, 3.5 Hz, 1H), 2.78 (q, *J* = 5.9 Hz, 2H), 2.27 (dddd, *J* = 11.8, 9.4, 8.0, 5.6 Hz, 1H) | 481.3 [M+H] + |

(continued)

| Example compounds | structure | Compound name | $^1$H NMR | LC-MS (m/z) |
|---|---|---|---|---|
| **125** | 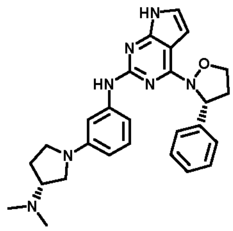 | *N*-(3-((R)-3-(dimethylamino) pyr rolidin-1-yl)phenyl)-4-((R)-3-pheny lisoxazolidin-2-yl)-7*H*-pyrrolo[2,3-*d*] pyrimidin-2-amine | $^1$H NMR (400 MHz, DMSO-$d_6$) δ 11.30 (s, 1H), 8.64 (s, 1H), 7.51 - 7.48 (m, 2H), 7.38 (dd, *J* = 8.4, 6.9 Hz, 2H), 7.30 - 7.25 (m, 1H), 7.09 (dd, *J* = 7.7, 1.9 Hz, 1H), 6.97 (d, *J* = 3.5 Hz, 1H), 6.55 (d, *J* = 3.5 Hz, 1H), 6.08 - 6.04 (m, 1H), 5.95 (dd, *J* = 8.7, 4.5 Hz, 1H), 3.80 (q, *J* = 8.0 Hz, 1H), 3.36 (dd, *J* = 9.1, 7.2 Hz, 1H), 3.25 (td, *J* = 9.1, 2.4 Hz, 1H), 3.12 (td, *J* = 9.3, 6.8 Hz, 2H), 3.03 - 2.96 (m, 1H), 2.79 (dddd, *J* = 23.8, 16.6, 11.2, 5.5 Hz, 3H), 2.31 (dtd, *J* = 12.4, 8.1, 4.4 Hz, 2H), 2.19 (s, 8H), 2.06 (dtd, *J* = 12.2, 6.8, 2.5 Hz, 2H), 1.78 - 1.70 (m, 1H). | 470.3 [M+H]+ |
| **126** | 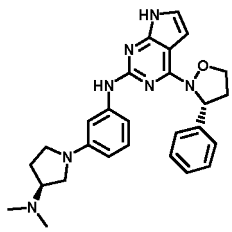 | *N*-(3-((S)-3-(dimethylamino) pyr rolidin-1-yl)phenyl)-4-((R)-3-pheny lisoxazolidin-2-yl)-7*H*-pyrrolo[2,3-*d*] pyrimidin-2-amine | $^1$H NMR (400 MHz, DMSO-$d_6$) δ 11.29 (s, 1H), 8.63 (s, 1H), 7.51 - 7.48 (m, 2H), 7.39 (dd, *J* = 8.3, 6.9 Hz, 2H), 7.30 - 7.26 (m, 1H), 7.11 - 7.08 (m, 1H), 6.97 (dd, *J* = 3.5, 2.1 Hz, 1H), 6.54 (dd, *J* = 3.5, 1.8 Hz, 1H), 6.09 - 6.04 (m, 1H), 5.95 (dd, *J* = 8.8, 4.6 Hz, 1H), 3.80 (q, *J* = 8.0 Hz, 2H), 3.38 - 3.33 (m, 1H), 3.26 - 3.19 (m, 2H), 3.14 - 3.10 (m, 1H), 3.02 - 2.94 (m, 2H), 2.87 - 2.69 (m, 3H), 2.32 (dtd, *J* = 12.7, 8.3, 4.5 Hz, 2H), 2.18 (s, 8H), 2.04 (dd, *J* = 7.2, 5.0 Hz, 1H), 1.79 - 1.68 (m, 1H). | 470.4 [M+H]+ |
| **127** | 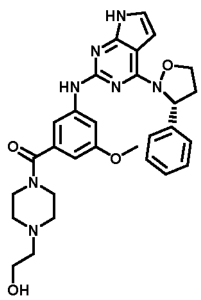 | (R)-(4-(2-hydroxyethyl)piper azin-1-yl)(3-methoxy-5-((4-(3-phenylisoxazolidin-2-yl)-7*H*-pyrrolo[2,3-*d*] pyrimidin-2-yl)amino) phenyl)m ethanone | $^1$H NMR (400 MHz, DMSO-$d_6$) δ 11.41 (t, *J* = 2.2 Hz, 1H), 9.09 (s, 1H), 7.67 (t, *J* = 2.2 Hz, 1H), 7.52 - 7.48 (m, 2H), 7.41 - 7.35 (m, 2H), 7.30 (q, *J* = 1.5 Hz, 1H), 7.02 (dd, *J* = 3.5, 2.3 Hz, 1H), 6.57 (dd, *J* = 3.5, 2.0 Hz, 1H), 5.95 (dd, *J* = 8.8, 4.4 Hz, 1H), 4.26 (td, *J* = 7.9, 3.7 Hz, 1H), 3.81 (q, *J* = 8.1 Hz, 1H), 3.69 (s, 3H), 3.52 (t, *J* = 6.0 Hz, 3H), 3.44 (s, 1H), 3.37 (d, *J* = 14.8 Hz, 5H), 2.83 (ddt, *J* = 12.0, 8.3, 3.7 Hz, 1H), 2.32 (dtd, *J* = 12.4, 8.3, 4.4 Hz, 1H), 2.08 (s, 3H), 1.91 (s, 4H). | 544.4 [M+H]+ |

(continued)

| Example compounds | structure | Compound name | $^1$H NMR | LC-MS (m/z) |
|---|---|---|---|---|
| **128** | 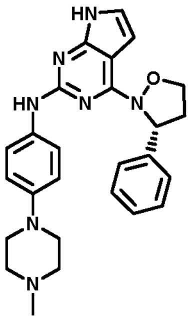 | (R)-*N*-(4-(4-methylpiperazin-1-yl)phenyl)-4-(3-phenylisoxazolidin-2-yl)-7H-pyrrolo[2,3-*d*[pyrimidin-2-amine | $^1$H NMR (400 MHz, DMSO-$d_6$) δ 11.26 (s, 1H), 8.63 (s, 1H), 7.51 (dd, *J* = 17.2, 8.2 Hz, 5H), 7.39 (t, *J* = 7.5 Hz, 2H), 6.94 - 6.90 (m, 1H), 6.76 (d, *J* = 9.0 Hz, 2H), 6.53 (dd, *J* = 3.5, 1.9 Hz, 1H), 4.26 (td, *J* = 7.9, 3.3 Hz, 2H), 3.34 - 3.25 (m, 1H), 3.01 (t, *J* = 5.0 Hz, 4H), 2.84 (dtd, *J* = 11.7, 7.9, 3.2 Hz, 2H), 2.45 (t, *J* = 4.9 Hz, 4H), 2.32 - 2.14 (m, 5H). | 456.4 [M+H]+ |
| **129** | 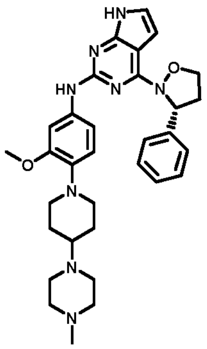 | (R)-*N*-(3-methoxy-4-(4-(4-methylpiperazin-1-yl) piperidin-1-yl)phenyl)-4-(3-pheny lisoxazolidin-2-yl)-7*H*-pyrrolo[2,3-*d*]pyrimidin-2-amine | $^1$H NMR (400 MHz, DMSO-$d_6$) δ 11.27 (d, *J* = 2.3 Hz, 1H), 8.69 (s, 1H), 7.51 - 7.47 (m, 2H), 7.43 - 7.36 (m, 3H), 7.31 - 7.26 (m, 1H), 7.21 (dd, *J* = 8.6, 2.3 Hz, 1H), 6.96 (dd, *J* = 3.5, 2.2 Hz, 1H), 6.54 (dd, *J* = 3.5, 2.0 Hz, 1H), 4.24 (td, *J* = 7.9, 3.6 Hz, 1H), 3.65 (s, 3H), 3.44 - 3.19 (m, 10H), 2.84 (qq, *J* = 8.2, 3.7 Hz, 1H), 2.48 - 2.39 (m, 4H), 2.30 (ddt, *J* = 11.9, 7.9, 4.3 Hz, 3H), 2.24 (s, 3H), 1.81 (d, *J* = 12.0 Hz, 2H), 1.54 (q, *J* = 11.6 Hz, 2H). | 569.3 [M+H]+ |

**Experimental Example 1: Evaluation of inhibitory activity against various kinases**

The activities of the compounds according to the present invention to more diverse enzymes were evaluated. Specifically, it was decided that the enzyme selectivity of Example 74 selected from the compounds of Examples 1 to 129 was measured at the request of DiscoverX, and the experiment was conducted using a scanMAX™ kinase assay panel. At this time, as a drug to treat the enzyme, a solution prepared by dissolving in DMSO at a concentration of 1 μM was used, and the control percentage (% control) was determined by the method of Equation 1 below, and the results were shown in Table 2 below.

[Equation 1]

$$\text{Control percentage (\% control)} = \frac{(Example\ compound - Positive\ control)}{(Negative\ control - Positive\ control)} \text{X}100$$

In Equation above, the positive control and negative control mean compounds showing control percentages of 0% and 100% as DMSO, respectively. At this time, when the calculated control percentage value for each enzyme was less than 35%, it was determined that it had an inhibitory activity for the relevant enzyme. Accordingly, among the tested series of enzymes, the enzymes in which the compounds of the present invention exhibited inhibitory activities were summarized and shown in Table 2 below together with the control percentages thereof.

[Table 2]

| Kinase | Example 74 | Kinase | Example 74 | Kinase | Example 74 |
|---|---|---|---|---|---|
| ABL1 (H396P)-nonphosphorylated | 18 | FLT3(K663Q) | 2.3 | PIK4CB | 16 |
| ABL 1 (Q252H)-nonphosphorylated | 24 | FLT3(N841I) | 0.3 | PIP5K1A | 1.2 |
| ABL 1 (T315I)-nonphosphorylated | 29 | GRK4 | 9.3 | PIP5K2B | 18 |
| ABL 1(T315I)-phosphorylated | 8.4 | HPK1 | 0.85 | PIP5K2C | 26 |
| ALK | 31 | INSR | 19 | PRP4 | 20 |
| ALK(C1156Y) | 15 | IRAK3 | 24 | PYK2 | 27 |
| ARK5 | 8.9 | ITK | 5.1 | RET | 20 |
| AXL | 3.2 | JAK1(JH2domain-pseudokinase) | 1.2 | RET(M918T) | 20 |
| BIKE | 1.8 | JAK2(JH1domain-catalytic) | 0 | RET(V804L) | 5.3 |
| BLK | 19 | JAK3 (JH1domain-catalytic) | 0 | RET(V804M) | 3.9 |
| CDK4 | 10 | JNK1 | 2.7 | RIOK1 | 5.5 |
| CDK4-cyclinD1 | 0.55 | JNK2 | 29 | RIOK3 | 3.6 |
| CDK4-cyclinD3 | 3 | JNK3 | 4.5 | ROCK1 | 11 |
| CDK7 | 21 | KIT(L576P) | 31 | ROCK2 | 8 |
| CDKL2 | 8.2 | LCK | 12 | RSK3(Kin.Dom.2-C-terminal) | 17 |
| CIT | 0.3 | LOK | 8.7 | RSK4(Kin.Dom.2-C-terminal) | 31 |
| CLK1 | 3.4 | LRRK2 | 28 | SLK | 0.25 |
| CLK2 | 9.2 | LRRK2(G2019S) | 25 | SNARK | 3.7 |
| CLK4 | 6.4 | LTK | 27 | SRPK1 | 14 |
| CSNK1G2 | 21 | MAP3K2 | 14 | SRPK3 | 13 |
| DCAMKL3 | 15 | MAP3K3 | 25 | STK16 | 26 |
| DLK | 9.2 | MAP4K2 | 12 | SYK | 32 |
| DMPK | 0.65 | MAP4K3 | 13 | TAK1 | 16 |
| DYRK1B | 11 | MAP4K4 | 30 | TAOK3 | 22 |
| EGFR | 28 | MAP4K5 | 21 | TIE1 | 16 |
| EGFR(E746-A750del) | 0 | MARK3 | 5.3 | TNIK | 27 |
| EGFR(L858R) | 20 | MARK4 | 18 | TRKA | 1.1 |
| EGFR(L858R,T790M) | 17 | MEK1 | 16 | TRKB | 8.3 |
| EGFR(L861Q) | 30 | MEK2 | 17 | TRKC | 21 |
| EGFR(T790M) | 3.7 | MEK5 | 26 | TTK | 13 |
| EPHB4 | 31 | MELK | 2 | TYK2(JH1domain-catalytic) | 5.6 |

(continued)

| Kinase | Example 74 | Kinase | Example 74 | Kinase | Example 74 |
|---|---|---|---|---|---|
| FES | 32 | MERTK | 29 | TYK2(JH2domain-pseudokinase) | 2.6 |
| FGR | 17 | MET | 4.5 | ULK1 | 8.1 |
| FLT3 | 3.9 | MET(M1250T) | 11 | ULK2 | 2.5 |
| FLT3(D835H) | 21 | MET(Y1235D) | 7 | ULK3 | 13 |
| FLT3(D835V) | 1.7 | MLK1 | 24 | VRK2 | 33 |
| FLT3(D835Y) | 0.15 | MST3 | 32 | YSK4 | 8.6 |
| FLT3(ITD) | 0.45 | MUSK | 0.4 | | |
| FLT3(ITD,D835V) | 0 | NEK10 | 0 | | |
| FLT3 (ITD,F69 1L) | 0 | PAK3 | 23 | | |

As shown in Table 2 above, the compounds according to the present invention showed values of less than 35% of the control percentage for ABL1(H396P)-nonphosphorylated, ABL1(Q252H)-nonphosphorylated, ABL1(T315I)-nonphosphorylated, ABL1(T315I)-phosphorylated, ALK, ALK(C1156Y), ARK5, AXL, BIKE, BLK, CDK4, CDK4-cyclinD1, CDK4-cyclinD3, CDK7, CDKL2, CIT, CLK1, CLK2, CLK4, CSNK1G2, DCAMKL3, DLK, DMPK, DYRK1B, EGFR, EGFR(E746-A750del), EGFR(L858R), EGFR(L858R,T790M), EGFR(L861Q), EGFR(T790M), EPHB4, FES, FGR, FLT3, FLT3(D835H), FLT3(D835V), FLT3(D835Y), FLT3(ITD), FLT3(ITD,D835V), FLT3(ITD,F691L), FLT3(K663Q), FLT3(N841I), GRK4, HPK1, INSR, IRAK3, ITK, JAK1(JH2domain-pseudokinase), JAK2(JH1domain-catalytic), JAK3(JHldomain-catalytic), JNK1, JNK2, JNK3, KIT(L576P), LCK, LOK, LRRK2, LRRK2(G2019S), LTK, MAP3K2, MAP3K3, MAP4K2, MAP4K3, MAP4K4, MAP4K5, MARK3, MARK4, MEK1, MEK2, MEK5, MELK, MERTK, MET, MET(M1250T), MET(Y1235D), MLK1, MST3, MUSK, NEK10, PAK3, PIK4CB, PIP5K1A, PIP5K2B, PIP5K2C, PRP4, PYK2, RET, RET(M918T), RET(V804L), RET(V804M), RIOK1, RIOK3, ROCK1, ROCK2, RSK3(Kin.Dom.2-C-terminal), RSK4(Kin.Dom.2-C-terminal), SLK, SNARK, SRPK1, SRPK3, STK16, SYK, TAK1, TAOK3, TIE1, TNIK, TRKA, TRKB, TRKC, TTK, TYK2(JH1domain-catalytic), TYK2(JH2domain-pseudokinase), ULK1, ULK2, ULK3, VRK2 and YSK4 kinases. This indicates that the compound of the present invention has inhibitory activities against the above-listed enzymes, thereby suggesting that it may exhibit useful effects by application to diseases related to the above-listed enzymes.

Therefore, the compound of the present invention can be usefully used as a composition for treating or preventing diseases related to the above-listed enzymes.

**Experimental Example 2: FLT3 enzyme inhibitory ability evaluation**

The inhibitory activities of the example compounds according to the present invention to the FLT3-ITD enzyme were evaluated. Specifically, the compound of the present invention was reacted with a purified human FLT3-ITD enzyme (Signalchem). A composition of 40 mM Tris-HCl pH 7.4, 20 mM $MgCl_2$, 0.5 mg/mL BSA and 50 μM DTT was used as a reaction buffer, and reactions of all test matters were performed on the reaction buffer. Upon the test, human FLT3-ITD enzyme (10 ng), purified ATP (10 μM) and a specific substrate solution were reacted at 25° C for 1 hour, and then the enzyme activity was identified using an in vitro ADP-Glo™ kinase assay (Promega). The enzyme activity reaction solution, the ADP-Glo reaction solution and the enzyme ability detection solution were reacted in a ratio of 2:2:1, and the activity inhibition degree of the enzyme was measured as luminescence. Using the solvent-treated group that was not treated with the compound as a control group, the enzyme activity inhibition degree according to the treatment concentration of each compound was calculated based on the enzyme activity fluorescence degree, and the concentration of each compound that inhibited 50%, $IC_{50}$ (nM), was determined using Prism Software (version 5.01, GraphPad). The enzyme inhibitory ability was measured as described above, and the calculated $IC_{50}$ (nM) values were shown in Table 3 below.

**Experimental Example 3: Evaluation of AML cell proliferation inhibitory activity expressing FLT3 gene**

The inhibitory activities of the example compounds according to the present invention on the proliferation of

acute myeloid leukemia (AML) cells expressing the FLT3 mutant gene were evaluated. Specifically, RS4;11 and MV4-11 cells as acute myeloid leukemia cells expressing the FLT3 mutant gene were cultured in RPMI-1640 (Invitrogen) adding 10% FBS. The cells were seeded into each well of a clear bottom white 96-well plate (Corning) at 3000 to 5000 cells 24 hours before compound treatment. The compound was diluted in DMSO (diluted each three-fold, to a total of 12 concentrations) and injected at 0.5μL each so as to be a final concentration of 0.3nM to 50μM. After 72 hours of the compound treatment, they were treated with CellTiter-Glo Luminescent Cell Viability assay (Promega) and stored at room temperature for 10 minutes, and luminescence intensities were measured with a reader (SynergyNeo, Biotek) to count viable cells. Each experiment was performed three times repeatedly. The result values were calculated as the cell growth rate (%) compared to the control treated with the solvent. The graph was derived using the GraphPad Prism program (version 5.0), the $GI_{50}$ (nM) values were calculated, and the results were shown in Table 3 below.

**Experimental Example 4: HPK1 enzyme inhibitory ability evaluation**

In order to evaluate the inhibitory activities of the example compounds according to the present invention on the **HPK1** (hematopoietic progenitor kinase 1) enzyme, the following experiment was performed. The example compounds were reacted with the purified human GST-HPK1 (1-346, Signalchem) enzyme to evaluate the enzyme inhibitory ability by the following method. As the reaction buffer, a composition of 40 mM Tris-HCl pH 7.4, 20 mM $MgCl_2$, 0.5 mg/mL BSA and 50 uM DTT was used, and reactions of all test matters were performed on the reaction buffer. Upon the test, the human GST-HPK1 (3ng) enzyme, the purified ATP (3uM) and a specific substrate solution were reacted at 25°C for 2 hours, and then the enzyme activity was identified using an in vitro ADP-Glo™ kinase assay (promega). The enzyme activity reaction solution, the ADP-Glo reaction solution and the enzyme ability detection solution were reacted in a ratio of 2:2:1 to measure luminescence. The enzyme activity inhibition degree according to the treatment concentrations of the respective compounds was calculated based on the fluorescence of the enzyme activity of the solvent control group that was not treated with the compound, where the concentration of each compound that inhibited enzyme activity inhibition by 50% was determined as an $IC_{50}$ (nM) value. The $IC_{50}$ of each compound was calculated using GraphPad Prism 8.3.0 (GraphPad software Inc., San Diego) software, and the results were shown in Table 3 below.

**Experimental Example 5: Evaluation of CDK4 and CDK6 enzyme inhibitory ability**

In order to evaluate the CDK4 and CDK6 enzyme inhibitory activities of the example compounds according to the present invention, $IC_{50}$ (nM) was measured for the CDK4 cyclin D1 and CDK6 cyclin D1 enzymes in Reaction Biology (USA), and the results were shown in Table 3 below.

**Experimental Example 6: Evaluation of breast cancer cell line cell proliferation inhibitory activity**

In order to evaluate the breast cancer cell line cell proliferation inhibitory activities of Examples according to the present invention, the following experiment was performed. Breast cancer cells of MCF-7 (Korean Cell Line Bank) were planted in a clear bottom white 96-well plate (Corning) so as to be $3 \times 10^3$/150μl/well, and a culture solution comprising the compound continuously diluted in multiples of 3 at 12 concentrations (0.00001-2mM) and a DMSO control group was added by 0.5 μl/well and treated so that the final concentration was 0.00005-10μM, and then cultured in a 37°C $CO_2$ incubator for 120 hours. After 120 hours, the plate treated with the compound is taken out, and CellTiter-Glo® 2.0 Assay (Promega) solution is treated at 150 μl/well and then they mix well. They mix well at room temperature for 10 minutes or so, and the fluorescence is measured with a microplate reader. The data were expressed as a percentage in proportion to the vehicle standard treated cells, the $GI_{50}$ (nM) values were calculated using GraphPad Prism 8.3.0 (GraphPad software Inc., San Diego), and the results were shown in Table 3 below.

The result values of Experimental Examples 2 to 6 in Table 3 below were indicated as follows.

A = 250nM or more; B = more than 250nM and 500nM or less; C = more than 500nM;

[Table 3]

| Example | Enzyme assay ($IC_{50}$) | | | | Cell assay ($GI_{50}$) | | |
|---|---|---|---|---|---|---|---|
| | FLT3-ITD | HPK1 | CDK4 cyclin D1 | CDK6 cyclin D1 | RS4;11 | MV4-11 | MCF-7 |
| 1 | B | C | | | | C | C |
| 2 | B | | | | C | A | |
| 3 | | | | | | C | |
| 4 | C | | | | | C | |

(continued)

| Example | Enzyme assay ($IC_{50}$) | | | | Cell assay ($GI_{50}$) | | |
|---|---|---|---|---|---|---|---|
| | FLT3-ITD | HPK1 | CDK4 cyclin D1 | CDK6 cyclin D1 | RS4;11 | MV4-11 | MCF-7 |
| 5 | C | C | | | C | C | C |
| 6 | C | C | | | C | C | C |
| 7 | A | C | | | C | B | C |
| 8 | C | C | | | | A | C |
| 9 | C | | | | C | C | |
| 10 | A | | | | | C | |
| 11 | C | C | C | C | C | C | C |
| 12 | C | C | | | C | B | C |
| 13 | C | C | | | C | B | C |
| 14 | C | C | | | | | C |
| 15 | C | C | | | | | C |
| 16 | C | C | | | | C | C |
| 17 | C | C | | | | | C |
| 18 | C | C | | | | | C |
| 19 | A | C | | | C | A | C |
| 20 | A | | | | C | B | |
| 21 | | | | | | C | |
| 22 | | | | | | C | |
| 23 | | | | | | C | |
| 24 | | | | | | C | |
| 25 | C | C | | | C | B | C |
| 26 | C | C | | | C | C | C |
| 27 | | | | | | C | C |
| 28 | A | C | | | C | A | C |
| 29 | C | C | | | C | B | C |
| 30 | C | C | | | A | B | C |
| 31 | C | C | | | C | C | C |
| 32 | C | | | | C | C | |
| 33 | C | | | | C | C | |
| 34 | C | C | | | C | C | C |
| 35 | C | C | | | C | B | C |
| 36 | C | C | | | C | A | |
| 37 | B | C | | | C | A | C |
| 38 | A | C | | | C | A | C |
| 39 | A | | | | C | A | |
| 40 | B | | | | C | A | |
| 41 | A | | | | C | A | |

(continued)

| Example | Enzyme assay ($IC_{50}$) | | | | Cell assay ($GI_{50}$) | | |
|---|---|---|---|---|---|---|---|
| | FLT3-ITD | HPK1 | CDK4 cyclin D1 | CDK6 cyclin D1 | RS4;11 | MV4-11 | MCF-7 |
| 42 | C | | | | | | |
| 43 | B | C | | | C | A | C |
| 44 | C | C | | | C | A | C |
| 45 | B | C | | | C | B | C |
| 46 | C | | | | C | B | |
| 47 | B | C | | | C | A | C |
| 48 | C | C | | | C | C | C |
| 49 | C | | | | | | |
| 50 | C | | | | | | |
| 51 | C | | | | C | A | |
| 52 | C | | | | | A | |
| 53 | C | | | | | | |
| 54 | C | | | | | | |
| 55 | C | | | | | | |
| 56 | C | | | | | | |
| 57 | B | C | | | | C | C |
| 58 | C | C | | | | | C |
| 59 | A | C | | | | C | C |
| 60 | C | C | | | | | C |
| 61 | C | C | | | | | C |
| 62 | C | C | | | | | C |
| 63 | C | C | | | | | C |
| 64 | C | C | | | | | C |
| 65 | C | C | | | | | C |
| 66 | C | C | | | | | C |
| 67 | C | C | | | | | C |
| 68 | B | C | | | | | C |
| 69 | B | C | | | | C | C |
| 70 | | | | | | | |
| 71 | | | | | | | |
| 72 | | | | | | | |
| 73 | | | | | | | |
| 74 | C | A | A | A | | A | C |
| 75 | B | C | | | | C | C |
| 76 | A | A | | | | C | C |
| 77 | A | A | | | | B | C |
| 78 | A | A | | | | C | C |

(continued)

| Example | Enzyme assay ($IC_{50}$) | | | | Cell assay ($GI_{50}$) | | |
|---|---|---|---|---|---|---|---|
| | FLT3-ITD | HPK1 | CDK4 cyclin D1 | CDK6 cyclin D1 | RS4;11 | MV4-11 | MCF-7 |
| 79 | B | A | | | C | A | C |
| 80 | | C | | | | | |
| 81 | B | A | | | | | C |
| 82 | A | A | | | | | C |
| 83 | A | A | | | | A | C |
| 84 | A | A | | | A | A | C |
| 85 | C | A | | | | | C |
| 86 | | C | | | | | |
| 87 | B | A | | | | A | C |
| 88 | | C | | | | | |
| 89 | B | A | | | | B | C |
| 90 | B | A | | | | | C |
| 91 | C | A | | | | | C |
| 92 | C | A | | | | | C |
| 93 | C | C | | | | | C |
| 94 | C | C | | | | | |
| 95 | C | A | | | | | C |
| 96 | A | C | | | | C | C |
| 97 | | C | | | | | |
| 98 | | C | | | | | |
| 99 | C | C | | | | | C |
| 100 | C | C | | | | | C |
| 101 | A | A | | | | C | C |
| 102 | | A | | | | | |
| 103 | | C | | | | | |
| 104 | A | A | | | | C | C |
| 105 | A | A | | | | B | C |
| 106 | | C | | | | | |
| 107 | | C | | | | | |
| 108 | | A | | | | | |
| 109 | A | A | | | | A | C |
| 110 | A | A | | | | C | B |
| 111 | B | A | | | | A | C |
| 112 | | C | | | | | |
| 113 | | C | | | | | |
| 114 | | C | | | | | |
| 115 | C | C | | | | | C |

(continued)

| Example | Enzyme assay ($IC_{50}$) | | | | Cell assay ($GI_{50}$) | | |
|---|---|---|---|---|---|---|---|
| | FLT3-ITD | HPK1 | CDK4 cyclin D1 | CDK6 cyclin D1 | RS4;11 | MV4-11 | MCF-7 |
| 116 | A | A | A | A | | C | C |
| 117 | A | A | | | | C | A |
| 118 | A | A | A | A | | C | C |
| 119 | A | A | | | | A | C |
| 120 | A | A | | | | A | A |
| 121 | C | C | | | | | A |
| 122 | | C | | | | | |
| 123 | A | C | A | A | | C | C |
| 124 | | C | | | | | |
| 125 | C | C | | | | | C |
| 126 | C | C | | | | | C |
| 127 | | | | | | | |
| 128 | | | | | | B | |
| 129 | C | | | | | B | |

As shown in Table 3 above, the compounds of the present invention inhibited FLT3, CDK4, CDK6 and HPK1 activities, and effectively inhibited the proliferation of cancer cells, such as acute myeloid leukemia cells and breast cancer cells, expressing the FLT3 mutant gene. This indicates that the compounds of the present invention can be usefully used in a pharmaceutical composition for preventing or treating cancer diseases.

## Claims

1. A compound represented by the following Formula 1, or an isomer thereof, a solvate thereof, a hydrate thereof, or a pharmaceutically acceptable salt thereof:

[Formula 1]

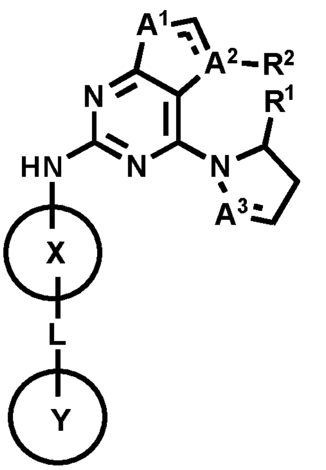

wherein,

$A^1$ is CH or NH;
$A^2$ is CH or S;
$A^3$ is N or O;
------ is a single bond or a double bond, which is determined according to the valence of atoms located at both ends thereof;
$R^1$ is phenyl, thiophenyl, pyridinyl, $(C_{3-7})$cycloalkyl, naphthalenyl, or isoquinolinyl, where the phenyl may be substituted with $(C_{1-3})$alkoxy or halogen;
$R^2$ is absent, hydrogen, haloalkyl, cyano, or halogen;
ring X is benzene, pyridine, pyrazole, isoindoline, tetrahydroisoquinoline, dihydroisoquinoline, or isobenzofuran, where the benzene, pyridine, pyrazole, isoindoline, tetrahydroisoquinoline, dihydroisoquinoline, or isobenzofuran may be substituted with one or more non-hydrogen substituents selected from the group consisting of $(C_{1-5})$alkyl, $(C_{1-5})$alkoxy, (di$(C_{1-3})$alkylamino$(C_{1-5})$alkyl)$(C_{1-3})$alkylamino, di$(C_{1-3})$alkylamino$(C_{1-5})$alkyl, (di$(C_{1-3})$alkylamino)$(C_{1-5})$alkoxy, di$(C_{1-3})$alkylaminocarbonyl$(C_{1-3})$alkyl, $(C_{3-7})$cycloalkyl, oxo('=O'), $(C_{1-3})$alkylsulfonyl, haloalkyl, $(C_{1-3})$alkylcarbonyl, hydroxy$(C_{1-5})$alkyl, and halogen;
L and ring Y are absent, or
when L and ring Y are present, L is a direct bond, -$(C_{1-5})$alkylene-, -O-, -(C=O)-, -O-$(C_{i-5})$alkylene-, -(C=O)-NH-, or piperidine, where the ring Y is a heterocyclic group selected from the group consisting of piperazine, pyrrolidine, morpholino, piperidine, imidazole, diazabicyclo[2,2,1]heptane, and pyrazole, where the heterocyclic group may be substituted with one or more non-hydrogen substituents selected from the group consisting of $(C_{1-5})$alkyl, di$(C_{1-3})$alkylamino, hydroxy$(C_{0-5})$alkyl, and $(C_{3-7})$cycloalkyl;
the case where both of L and ring Y are piperidine is excluded; and
the case where both of $A^1$ and $A^2$ are CH or are not CH is excluded.

**2.** The compound represented by Formula 1, or an isomer thereof, a solvate thereof, a hydrate thereof, or a pharmaceutically acceptable salt thereof, according to claim 1, wherein,

when $A^1$ is CH, $A^2$ is S, and $A^3$ is N,
$R^1$ is phenyl, pyridinyl, $(C_{3-7})$cycloalkyl, naphthalenyl, or isoquinolinyl, where the phenyl may be substituted with halogen;
$R^2$ is absent;
ring X is benzene, pyridine, pyrazole, or isoindoline, where the benzene, pyridine, pyrazole, or isoindoline may be substituted with one or more non-hydrogen substituents selected from the group consisting of $(C_{1-5})$alkyl, $(C_{1-5})$alkoxy, (di$(C_{1-3})$alkylamino$(C_{1-5})$alkyl)$(C_{1-3})$alkylamino, di$(C_{1-3})$alkylamino$(C_{1-5})$alkyl, (di$(C_{1-3})$alkylamino)$(C_{1-5})$alkoxy, $(C_{3-7})$cycloalkyl, oxo ('=O'), haloalkyl, and halogen;
both of L and ring Y are absent; or
when L and ring Y are present, L is a direct bond, -$(C_{1-5})$alkylene-, -O-, -(C=O)-, -O-$(C_{1-5})$alkylene-, -(C=O)-NH-, or piperidine, where the ring Y is a heterocyclic group selected from the group consisting of piperazine, pyrrolidine, morpholino, piperidine, imidazole, and pyrazole; where the heterocyclic group may be substituted with one or more non-hydrogen substituents selected from the group consisting of $(C_{1-5})$alkyl, di$(C_{1-3})$alkylamino, and hydroxy$(C_{0-5})$alkyl, and the case where both of L and ring Y are piperidine is excluded.

**3.** The compound represented by Formula 1, or an isomer thereof, a solvate thereof, a hydrate thereof, or a pharmaceutically acceptable salt thereof, according to claim 2,

wherein $R^1$ is phenyl, pyridinyl, cyclohexyl, naphthalenyl, or isoquinolinyl, where the phenyl may be substituted with fluoro;
$R^2$ is absent;
ring X is benzene, pyridine, pyrazole, or isoindoline, where the benzene, pyridine, pyrazole, or isoindoline may be substituted with one or more non-hydrogen substituents selected from the group consisting of methyl, ethyl, methoxy, ethoxy, (dimethylaminoethyl)methylamino, (dimethylamino)ethyl, (dimethylamino)propyl, (diethylamino)ethoxy, (diethylamino)propoxy, cyclopropyl, oxo ('=O'), trifluoromethyl, and fluoro;
both of L and ring Y are absent; or
when L and ring Y are present, L is a direct bond, $-CH_2-$, -O-, -(C=O)-, $-O-CH_2CH_2-$, $-O-CH_2CH_2CH_2-$, -(C=O)-NH-, or piperidine, where the ring Y is a heterocyclic group selected from the group consisting of piperazine, pyrrolidine, morpholino, piperidine, imidazole, and pyrazole, where the heterocyclic ring group may be substituted with one or more non-hydrogen substituents selected from the group consisting of methyl, ethyl, dimethylamino, and hydroxyethyl, and the case where both of L and Y are piperidine is excluded.

**4.** The compound represented by Formula 1, or an isomer thereof, a solvate thereof, a hydrate thereof, or a pharmaceutically acceptable salt thereof, according to claim 2, wherein when L and ring Y are present,

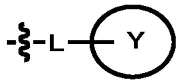

is

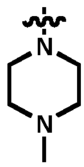 , 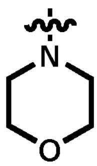 , 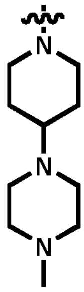 , 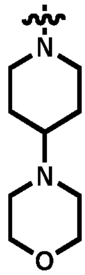 , 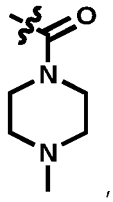 , 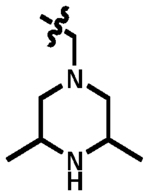 , 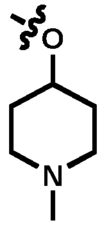 ,

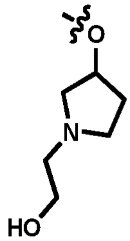 , 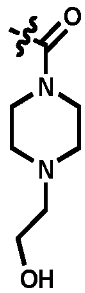 , 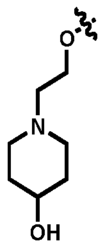 , 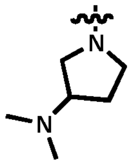 , 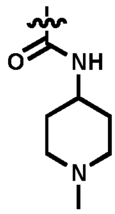 , 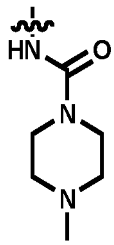 , 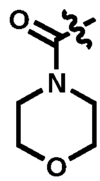 , 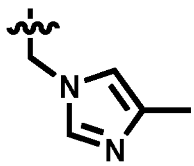 , 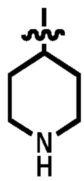 ,

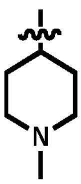 , 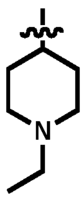 , 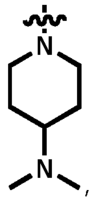 , 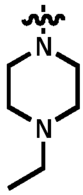 , 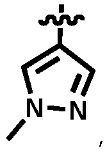 , 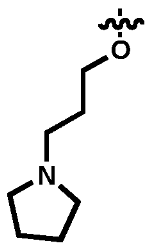 , 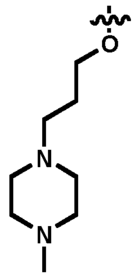 , or 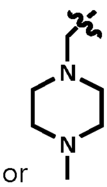 .

**5.** The compound represented by Formula 1, or an isomer thereof, a solvate thereof, a hydrate thereof or a pharmaceutically acceptable salt thereof, according to claim 1, wherein,

when $A^1$ is NH, $A^2$ is CH, and $A^3$ is N,
$R^1$ is phenyl, thiophenyl, or pyridinyl, where the phenyl may be substituted with $(C_{1-3})$alkoxy or halogen;
$R^2$ is hydrogen, haloalkyl, cyano, or halogen;
ring X is benzene, pyrazole, tetrahydroisoquinoline, dihydroisoquinoline, or isobenzofuran, where the benzene, pyrazole, tetrahydroisoquinoline, dihydroisoquinoline, or isobenzofuran may be substituted with one or more non-hydrogen substituents selected from the group consisting of $(C_{1-5})$alkyl, $(C_{1-5})$alkoxy, $(di(C_{1-3})alkylamino)(C_{1-5})$alkoxy, $di(C_{1-3})alkylaminocarbonyl(C_{1-3})$alkyl, $(C_{3-7})$cycloalkyl, oxo('=O'), $(C_{1-3})$alkylsulfonyl, $(C_{1-3})$alkylcarbonyl, and hydroxy$(C_{1-5})$alkyl;
L and ring Y are absent; or
when L and ring Y are present, L is a direct bond, -O-, -(C=O)-, -O-$(C_{1-5})$alkylene-, - (C=O)-NH-, or piperidine, where the ring Y is a heterocyclic group selected from the group consisting of piperazine, pyrrolidine, morpholino, piperidine, and diazabicyclo[2,2,1]heptane, where the heterocyclic group may be substituted with one or more

non-hydrogen substituents selected from the group consisting of $(C_{1-5})$alkyl, di$(C_{1-3})$alkylamino, hydroxy$(C_{0-5})$alkyl, and $(C_{3-7})$cycloalkyl, and the case where both of L and ring Y are piperidine is excluded.

**6.** The compound represented by Formula 1, or an isomer thereof, a solvate thereof, a hydrate thereof, or a pharmaceutically acceptable salt thereof, according to claim 5, wherein

$R^1$ is phenyl, thiophenyl, or pyridinyl, where the phenyl may be substituted with methoxy or fluoro;
$R^2$ is hydrogen, trifluoromethyl, cyano, or chloro;
ring X is benzene, pyrazole, tetrahydroisoquinoline, dihydroisoquinoline, or isobenzofuran, where the benzene, pyrazole, tetrahydroisoquinoline, dihydroisoquinoline, or isobenzofuran may be substituted with one or more non-hydrogen substituents selected from the group consisting of methyl, ethyl, methoxy, (diethylamino)propoxy, (dimethylamino)carbonylmethyl, cyclopropyl, oxo ('=O'), methylsulfonyl, methylcarbonyl, and branched hydroxybutyl;
L and ring Y are absent; or
when L and ring Y are present, L is a direct bond, -O-, -(C=O)-, $-OCH_2CH_2CH_2-$, - (C=O)-NH-, or piperidine, and ring Y is a heterocyclic group selected from the group consisting of piperazine, pyrrolidine, morpholino, piperidine, and diazabicyclo[2,2,1]heptane, where the heterocyclic group may be substituted with one or more non-hydrogen substituents selected from the group consisting of methyl, ethyl, dimethylamino, hydroxyethyl, and cyclopropyl, and the case where both of L and ring Y are piperidine is excluded.

**7.** The compound represented by Formula 1, or an isomer thereof, a solvate thereof, a hydrate thereof or a pharmaceutically acceptable salt thereof, according to claim 5, wherein,
when L and ring Y are present,

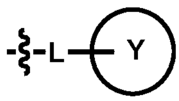 is 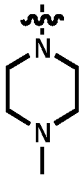, 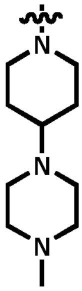, 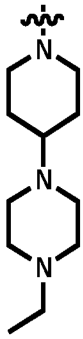, 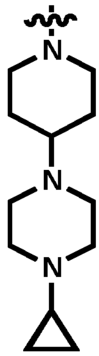, 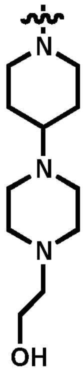, 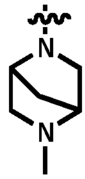, 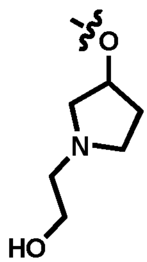,

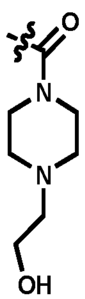, 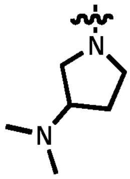, 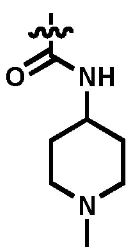, 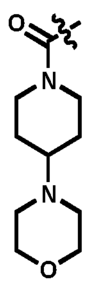, 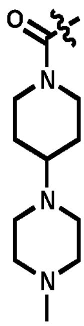, 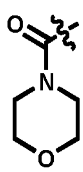, 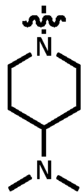, 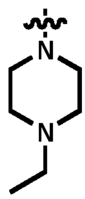, 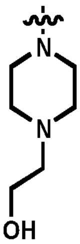,

or

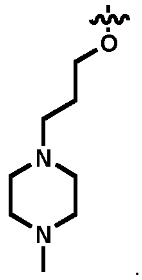

8. The compound represented by Formula 1, or an isomer thereof, a solvate thereof, a hydrate thereof, or a pharmaceutically acceptable salt thereof, according to claim 1, wherein,

when $A^1$ is CH, $A^2$ is S and $A^3$ is O, or
when $A^1$ is NH, $A^2$ is CH and $A^3$ is N,
$R^1$ is phenyl;
$R^2$ is absent;
ring X is benzene, where the benzene may be substituted with $(C_{1-3})$alkoxy or $(C_{3-7})$cycloalkyl;
L is a direct bond, -O-, -(C=O)-, or piperidine; and
ring Y is piperazine or pyrrolidinyl, where the piperazine or pyrrolidine may be substituted with $(C_{1-3})$alkyl, hydroxy$(C_{1-3})$alkyl or di$(C_{1-3})$alkylamino.

9. The compound represented by Formula 1, or an isomer thereof, a solvate thereof, a hydrate thereof or a pharmaceutically acceptable salt thereof, according to claim 8, wherein,

ring X is benzene, where the benzene may be substituted with methoxy or cyclopropyl;
L is a direct bond, -O-, -(C=O)-, or piperidine; and
ring Y is piperazine or pyrrolidine, where the piperazine or pyrrolidine may be substituted with methyl, hydroxyethyl, or dimethylamino.

10. The compound represented by Formula 1, or an isomer thereof, a solvate thereof, a hydrate thereof or a pharmaceutically acceptable salt thereof, according to claim 8, wherein,

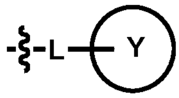 is 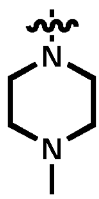, 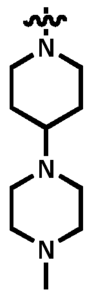, 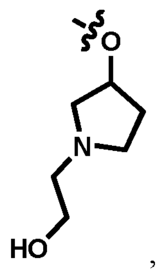, 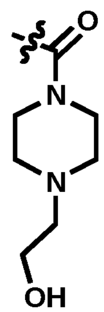, or 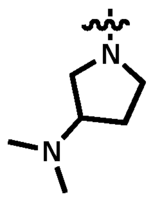.

11. The compound represented by Formula 1, or a stereoisomer thereof, a hydrate thereof, or a pharmaceutically acceptable salt thereof, according to claim 1, wherein the compound represented by Formula 1 is selected from the group consisting of the following compounds:

(1) *N*-(4-(4-methylpiperazin-1-yl)phenyl)-4-(5 -phenyl -4,5 -dihydro-1*H*-pyrazol-1-yl)thieno[3,2-*d*]pyrimidin-2-amine,
(2) *N*-(2-(dimethylamino)ethyl)-*N*-methyl-*N*-((4-(5-phenyl-4,5-dihydro-1*H*-pyrazol-1-yl)thieno[3,2-*d*]pyrimidin-2-yl)benzene-1,4-diamine,
(3) *N*-(4-morpholinophenyl)-4-(5-phenyl-4,5-dihydro-1*H*-pyrazol-1-yl)thieno[3,2-*d*]pyrimidin-2-amine,
(4) *N*-(5-(4-methylpiperazin-1-yl)pyridin-2-yl)-4-(5-phenyl -4,5 -dihydro-1*H*-pyrazol-1 - yl)thieno[3,2-*d*]pyrimidin-2-amine,
(5) *N*-(6-(4-methylpiperazin-1-yl)pyridin-3-yl)-4-(5-phenyl-4,5-dihydro-1*H*-pyrazol-1-yl)thieno[3,2-*d*]pyrimidin-2-amine,

(6) *N*-(2-methoxy-4-(4-methylpiperazin-1-yl)phenyl)-4-(5-phenyl-4,5-dihydro-1*H*-pyrazol-1-yl)thieno[3,2-*d*]pyrimidin-2-amine,
(7) *N*-(4-(4-(4-methylpiperazin-1-yl)piperidin-1-yl)phenyl)-4-(5-phenyl-4,5-dihydro-1*H*-pyrazol-1-yl)thieno[3,2-*d*]pyrimidin-2-amine,
(8) *N*-(3-methoxy-4-(4-(4-methylpiperazin-1-yl)piperidin-1-yl)phenyl)-4-(5 -phenyl-4,5 - dihydro-1*H*-pyrazol-1-yl)thieno[3,2-*d*]pyrimidin-2-amine,
(9) *N*-(4-(4-morpholinopiperidin-1-yl)phenyl)-4-(5 -phenyl -4,5 -dihydro-1*H*-pyrazol-1 - yl)thieno[3,2-*d*]pyrimidin-2-amine,
(10) *N*-(2-methoxy-4-(4-(4-methylpiperazin-1-yl)piperidin-1-yl)phenyl)-4-(5 -phenyl-4,5 - dihydro-1*H*-pyrazol-1-yl)thieno[3,2-*d*]pyrimidin-2-amine,
(11) (4-methylpiperazin-1-yl)(4-((4-(5-phenyl-4,5-dihydro-1*H*-pyrazol-1-yl)thieno[3,2-*d*]pyrimidin-2-yl)amino)phenyl)methanone,
(12) *N*-(3-cyclopropyl-5-(((3*R*,5*S*)-3,5-dimethylpiperazin-1-yl)methyl)phenyl)-4-(5 -phenyl-4,5-dihydro-1*H*-pyrazol-1-yl)thieno[3,2-*d*]pyrimidin-2-amine,
(13) *N*-(4-((1-methylpiperidin-4-yl)oxy)phenyl)-4-(5-phenyl-4,5-dihydro-1*H*-pyrazol-1-yl)thieno[3,2-*d*]pyrimidin-2-amine,
(14) 2-((3R)-3-(3-cyclopropyl-5-((4-(5-phenyl-4,5-dihydro-1*H*-pyrazol-1-yl)thieno[3,2-*d*]pyrimidin-2-yl)amino)phenoxy)pyrrolidin-1-yl)ethan-1-ol,
(15) (4-(2-hydroxyethyl)piperazin-1-yl)(3-methoxy-5-((4-(5-phenyl-4,5-dihydro-1*H*-pyrazol-1-yl)thieno[3,2-*d*]pyrimidin-2-yl)amino)phenyl)methanone,
(16) 1-(2-(3-ethyl-5-((4-(5-phenyl-4,5-dihydro-1*H*-pyrazol-1-yl)thieno[3,2-d]pyrimidin-2-yl)amino)phenoxy)ethyl)piperidin-4-ol,
(17) *N*-(3-((R)-3-(dimethylamino)pyrrolidin-1-yl)phenyl)-4-(5 -phenyl -4,5-dihydro-1*H*-pyrazol-1-yl)thieno[3,2-*d*]pyrimidin-2-amine,
(18) *N*-(3-((S)-3-(dimethylamino)pyrrolidin-1-yl)phenyl)-4-(5-phenyl-4,5-dihydro-1*H*-pyrazol-1-yl)thieno[3,2-*d*]pyrimidin-2-amine,
(19) *N*-(1-methylpiperidin-4-yl)-4-((4-(5-phenyl-4,5-dihydro-1*H*-pyrazol-1-yl)thieno[3,2-*d*]pyrimidin-2-yl)amino)benzamide,
(20) 4-methyl-*N*-(4-((4-(5-phenyl-4,5-dihydro-1*H*-pyrazol-1-yl)thieno[3,2-*d*]pyrimidin-2-yl)amino)phenyl)piperazine-1-carboxamide,
(21) *N*-(2-methyl-4-morpholinophenyl)-4-(5-phenyl-4,5-dihydro-1*H*-pyrazol-1-yl)thieno[3,2-*d*]pyrimidin-2-amine,
(22) morpholino(4-((4-(5-phenyl-4,5-dihydro-1*H*-pyrazol-1-yl)thieno[3,2-*d*]pyrimidin-2-yl)amino)phenyl)methanone,
(23) *N*-(4-((4-methyl-1*H*-imidazol-1-yl)methyl)phenyl)-4-(5-phenyl-4,5-dihydro-1*H*-pyrazol-1-yl)thieno[3,2-*d*]pyrimidin-2-amine,
(24) *N*-(3-((4-methyl-1*H*-imidazol-1-yl)methyl)phenyl)-4-(5-phenyl-4,5-dihydro-1*H*-pyrazol-1-yl)thieno[3,2-*d*]pyrimidin-2-amine,
(25) 2-methyl-5-((4-(5-phenyl-4,5-dihydro-1*H*-pyrazol-1-yl)thieno[3,2-*d*]pyrimidin-2-yl)amino)isoindolin-1-one,
(26) *N*-(1-(3-(dimethylamino)propyl)-1*H*-pyrazol-4-yl)-4-(5-phenyl-4,5-dihydro-1*H*-pyrazol-1-yl)thieno[3,2-*d*]pyrimidin-2-amine,
(27) *N*-(1-(2-(dimethylamino)ethyl)-1*H*-pyrazol-4-yl)-4-(5-phenyl-4,5-dihydro-1*H*-pyrazol-1-yl)thieno[3,2-*d*]pyrimidin-2-amine,
(28) 4-(5-phenyl-4,5-dihydro-1*H*-pyrazol-1-yl)-*N*-(1-(piperidin-4-yl)-1*H*-pyrazol-4-yl)thieno[3,2-*d*]pyrimidin-2-amine,
(29) *N*-(1-(1-methylpiperidin-4-yl)-1*H*-pyrazol-4-yl)-4-(5-phenyl-4,5-dihydro-1*H*-pyrazol-1-yl)thieno[3,2-*d*]pyrimidin-2-amine,
(30) 4-(5-cyclohexyl-4,5-dihydro-1*H*-pyrazol-1-yl)-*N*-(4-(4-methylpiperazin-1-yl)phenyl)thieno[3,2-*d*]pyrimidin-2-amine,
(31) *N*-(4-(4-methylpiperazin-1-yl)phenyl)-4-(5-(naphthalen-2-yl)-4,5-dihydro-1*H*-pyrazol-1-yl)thieno[3,2-*d*]pyrimidin-2-amine,
(32) 4-(5-(isoquinolin-3 -yl)-4,5-dihydro-1*H*-pyrazol-1-yl)-*N*(4-(4-methylpiperazin-1-yl)phenyl)thieno[3,2-*d*]pyrimidin-2-amine,
(33) *N*-(4-(4-methylpiperazin-1-yl)phenyl)-4-(5-(pyridin-2-yl)-4,5-dihydro-1*H*-pyrazol-1-yl)thieno[3,2-*d*]pyrimidin-2-amine,
(34) *N*-(4-(4-methylpiperazin-1-yl)phenyl)-4-(5-(pyridin-3-yl)-4,5-dihydro-1*H*-pyrazol-1-yl)thieno[3,2-*d*]pyrimidin-2-amine,
(35) *N*-(4-(4-methylpiperazin-1-yl)phenyl)-4-(5-(pyridin-4-yl)-4,5-dihydro-1*H*-pyrazol-1-yl)thieno[3,2-*d*]pyrimidin-2-amine,
(36) 4-(5-(3-fluorophenyl)-4,5 -dihydro-1*H*-pyrazol-1-yl)-*N*-(4-(4-methylpiperazin-1 - yl)phenyl)thieno[3,2-*d*]pyrimidin-2-amine,
(37) *N*-(4-(4-(4-ethylpiperazin-1-yl)piperidin-1-yl)phenyl)-4-(5-phenyl-4,5-dihydro-1*H*-pyrazol-1-yl)thieno[3,2-*d*]pyrimidin-2-amine,
(38) *N*-(4-(4-(dimethylamino)piperidin-1-yl)phenyl)-4-(5-phenyl-4,5-dihydro-1*H*-pyrazol-1-yl)thieno[3,2-*d*]pyrimidin-2-amine,
(39) *N*-(4-(4-ethylpiperazin-1-yl)phenyl)-4-(5-phenyl-4,5-dihydro-1*H*-pyrazol-1-yl)thieno[3,2-*d*]pyrimidin-2-amine,
(40) *N*-(3-methoxy-4-(4-methylpiperazin-1-yl)phenyl)-4-(-1-yl)thieno[3,2-*d*]pyrimidin-2-amine,
(41) *N*-(4-(4-ethylpiperazin-1-yl)-2-methoxyphenyl)-4-(5-phenyl-4,5-dihydro-1*H*-pyrazol-1-yl)thieno[3,2-*d*]pyrimidin-2-amine,
(42) *N*-(2-methoxy-4-(1-methyl-1*H*-pyrazol-4-yl)phenyl)-4-(5-phenyl-4,5-dihydro-1*H*-pyrazol-1-yl)thieno[3,2-*d*]pyrimidin-2-amine,
(43) *N*-(4-((*R*)-3-(dimethylamino)pyrrolidin-1-yl)phenyl)-4-(5 -phenyl -4,5 -dihydro-1*H*-pyrazol-1-yl)thieno[3,2-*d*]pyrimidin-2-amine,
(44) *N*-(4-((S)-3-(dimethylamino)pyrrolidin-1-yl)phenyl)-4-(5-phenyl-4,5-dihydro-1*H*-pyrazol-1-yl)thieno[3,2-*d*]pyrimidin-2-amine,
(45) *N*-(4-(2-(diethylamino)ethoxy)phenyl)-4-(5-phenyl-4,5-dihydro-1*H*-pyrazol-1-yl)thieno[3,2-*d*]pyrimidin-2-amine,
(46) *N*-(4-(2-(diethylamino)ethoxy)-3,5-dimethylphenyl)-4-(5-phenyl-4,5-dihydro-1*H*-pyrazol-1-yl)thieno[3,2-*d*]pyrimidin-2-amine,
(47) *N*-(4-(3-(diethylamino)propoxy)phenyl)-4-(5-phenyl-4,5-dihydro-1*H*-pyrazol-1-yl)thieno[3,2-*d*]pyrimidin-2-amine,
(48) *N*-(4-(3-(diethylamino)propoxy)-3,5-dimethylphenyl)-4-(5-phenyl-4,5-dihydro-1*H*-pyrazol-1-yl)thieno[3,2-*d]*pyrimidin-2-amine,
(49) *N*-(3,5-dimethyl-4-(3-(pyrrolidin-1-yl)propoxy)phenyl)-4-(5 -phenyl -4,5 -dihydro-1*H*-pyrazol-1-yl)thieno[3,2-*d*]pyrimidin-2-amine,
(50) *N*-(3,5-dimethyl-4-(3-(4-methylpiperazin-1-yl)propoxy)phenyl)-4-(5-phenyl-4,5-dihydro-1*H*-pyrazol-1-yl)thieno[3,2-*d*]pyrimidin-2-amine,
(51) 4-(5-phenyl-4,5-dihydro-1*H*-pyrazol-1-yl)-*N*-(3,4,5-trimethoxyphenyl)thieno[3,2-*d*]pyrimidin-2-amine,
(52) 2-methoxy-*N*-(1-methylpiperidin-4-yl)-4-((4-(5-phenyl-4,5-dihydro-1*H*-pyrazol-1-yl)thieno[3,2-*d*]pyrimidin-2-yl)amino)benzamide,
(53) *N*-(4-(4-methylpiperazin-1-yl)-3-(trifluoromethyl)phenyl)-4-(5-phenyl-4,5-dihydro-1*H*-pyrazol-1-yl)thieno[3,2-*d*]pyrimidin-2-amine,
(54) *N*-(4-((4-methylpiperazin-1-yl)methyl)-3-(trifluoromethyl)phenyl)-4-(5-phenyl-4,5-dihydro-1*H*-pyrazol-1-yl)thieno[3,2-*d*]pyrimidin-2-amine,
(55) *N*-(3,5-difluoro-4-(4-(4-methylpiperazin-1 -yl)piperidin-1-yl)phenyl)-4-(5 -phenyl-4,5 - dihydro-1*H*-pyrazol-1-yl)thieno[3,2-*d*]pyrimidin-2-amine,
(56) *N*-(4-(4-ethylpiperazin-1-yl)-3,5-difluorophenyl)-4-(5-phenyl-4,5-dihydro-1*H*-pyrazol-1-yl)thieno[3,2-*d*]pyrimidin-2-amine,
(57) (S)-*N*-(3-methoxy-4-(4-(4-methylpiperazin-1-yl)piperidin-1-yl)phenyl)-4-(3-phenylisoxazolidin-2-yl)thieno[3,2-*d*]pyrimidin-2-amine,
(58) (R)-*N*-(3-methoxy-4-(4-(4-methylpiperazin-1-yl)piperidin-1-yl)phenyl)-4-(3 - phenylisoxazolidin-2-yl)thieno[3,2-*d*]pyrimidin-2-amine,
(59) (S)-N-(4-(4-methylpiperazin-1-yl)phenyl)-4-(3-phenylisoxazolidin-2-yl)thieno[3,2-*d*]pyrimidin-2-amine,
(60) (R)-*N*-(4-(4-methylpiperazin-1-yl)phenyl)-4-(3-phenylisoxazolidin-2-yl)thieno[3,2-*d*]pyrimidin-2-amine,
(61) *N*-(3-((R)-3-(dimethylamino)pyrrolidin-1-yl)phenyl)-4-((S)-3-phenylisoxazolidin-2-yl)thieno[3,2-*d*]pyrimidin-2-amine,
(62) *N*-(3-((S)-3-(dimethylamino)pyrrolidin-1-yl)phenyl)-4-((S)-3-phenylisoxazolidin-2-yl)thieno[3,2-*d*]pyrimidin-2-amine,
(63) *N*-(3-((R)-3-(dimethylamino)pyrrolidin-1-yl)phenyl)-4-((R)-3-phenylisoxazolidin-2-yl)thieno[3,2-*d*]pyrimidin-2-amine,
(64) *N*-(3-((S)-3-(dimethylamino)pyrrolidin-1-yl)phenyl)-4-((R)-3-phenylisoxazolidin-2-yl)thieno[3,2-*d*]pyrimidin-2-amine,
(65) 2-((R)-3-(3-cyclopropyl-5-((4-((S)-3-phenylisoxazolidin-2-yl)thieno[3,2-*d*]pyrimidin-2-yl)amino)phenoxy)pyrrolidin-1-yl)ethan-1-ol,
(66) (S)-(4-(2-hydroxyethyl)piperazin-1-yl)(3-methoxy-5-((4-(3-phenylisoxazolidin-2-yl)thieno[3,2-*d*]pyrimidin-2-yl)amino)phenyl)methanone,

(67) (R)-(4-(2-hydroxyethyl)piperazin-1-yl)(3-methoxy-5-((4-(3-phenylisoxazolidin-2-yl)thieno[3,2-*d*]pyrimidin-2-yl)amino)phenyl)methanone,

(68) *N*-(4-(4-methylpiperazin-1-yl)phenyl)-4-(5-phenyl-4,5-dihydro-1*H*-pyrazol-1-yl)-7*H*-pyrrolo[2,3-*d*]pyrimidin-2-amine,

(69) *N*-(3-methoxy-4-(4-(4-methylpiperazin-1-yl)piperidin-1-yl)phenyl)-4-(5 -phenyl-4,5 - dihydro-1*H*-pyrazol-1-yl)-7*H*-pyrrolo[2,3-*d*]pyrimidin-2-amine,

(70) (4-(2-hydroxyethyl)piperazin-1-yl)(3-methoxy-5-((4-(5-phenyl-4,5-dihydro-1*H*-pyrazol-1-yl)-7*H*-pyrrolo[2,3-*d*]pyrimidin-2-yl)amino)phenyl)methanone,

(71) 2-((3R)-3-(3-cyclopropyl-5-((4-(5-phenyl-4,5-dihydro-1*H*-pyrazol-1-yl)-7*H*-pyrrolo[2,3-*d*]pyrimidin-2-yl)amino)phenoxy)pyrrolidin-1-yl)ethan-1-ol,

(72) *N*-(3-((R)-3-(dimethylamino)pyrrolidin-1-yl)phenyl)-4-(5-phenyl-4,5-dihydro-1*H*-pyrazol-1-yl)-7*H*-pyrrolo[2,3-*d*]pyrimidin-2-amine,

(73) *N*-(3-((S)-3-(dimethylamino)pyrrolidin-1-yl)phenyl)-4-(5-phenyl-4,5-dihydro-1*H*-pyrazol-1-yl)-7*H*-pyrrolo[2,3-*d*]pyrimidin-2-amine,

(74) (S)-*N*-(4-(4-methylpiperazin-1-yl)phenyl)-4-(3-phenylisoxazolidin-2-yl)-7*H*-pyrrolo[2,3-*d*]pyrimidin-2-amine,

(75) (*S*)-4-(3-(3-methoxyphenyl)isoxazolidin-2-yl)-*N*-(4-(4-methylpiperazin-1-yl)phenyl)-7*H*-pyrrolo[2,3-*d*]pyrimidin-2-amine,

(76) (*S*)-4-(3-(3-fluorophenyl)isoxazolidin-2-yl)-*N*-(4-(4-methylpiperazin-1-yl)phenyl)-7*H*-pyrrolo[2,3-*d*]pyrimidin-2-amine,

(77) (*S*)-*N*-(4-(4-methylpiperazin-1-yl)phenyl)-4-(3-(thiophen-2-yl)isoxazolidin-2-yl)-7*H*-pyrrolo[2,3-*d*]pyrimidin-2-amine,

(78) (*S*)-*N*-(4-(4-methylpiperazin-1-yl)phenyl)-4-(3-(pyridin-3-yl)isoxazolidin-2-yl)-7*H*-pyrrolo[2,3-*d*]pyrimidin-2-amine,

(79) (*S*)-*N*-(4-(4-ethylpiperazin-1-yl)phenyl)-4-(3-phenylisoxazolidin-2-yl)-7*H*-pyrrolo[2,3-*d*]pyrimidin-2-amine,

(80) (*S*)-*N*-(2-methoxy-4-(4-methylpiperazin-1-yl)phenyl)-4-(3-phenylisoxazolidin-2-yl)-7*H*-pyrrolo[2,3-*d*]pyrimidin-2-amine,

(81) (*S*)-2-(4-(4-((4-(3-phenylisoxazolidin-2-yl)-7*H*-pyrrolo[2,3-*d*]pyrimidin-2-yl)amino)phenyl)piperazin-1-yl)ethan-1-ol,

(82) (*S*)-2-(4-(2-methoxy-4-((4-(3-phenylisoxazolidin-2-yl)-7*H*-pyrrolo[2,3-*d*]pyrimidin-2-yl)amino)phenyl)piperazin-1-yl)ethan-1-ol,

(83) (*S*)-*N*-(4-(4-(dimethylamino)piperidin-1-yl)phenyl)-4-(3-phenylisoxazolidin-2-yl)-7*H*-pyrrolo[2,3-*d*]pyrimidin-2-amine,

(84) (*S*)-*N*-(4-(4-(4-methylpiperazin-1-yl)piperidin-1-yl)phenyl)-4-(3-phenylisoxazolidin-2-yl)-7*H*-pyrrolo[2,3-*d*]pyrimidin-2-amine,

(85) (*S*)-*N*-(4-(4-(4-ethylpiperazin-1-yl)piperidin-1-yl)phenyl)-4-(3-phenylisoxazolidin-2-yl)-7*H*-pyrrolo[2,3-*d*]pyrimidin-2-amine,

(86) (*S*)-*N*-(4-(4-(4-cyclopropylpiperazin-1-yl)piperidin-1-yl)phenyl)-4-(3-phenylisoxazolidin-2-yl)-7*H*-pyrrolo[2,3-*d*]pyrimidin-2-amine,

(87) (*S*)-*N*-(3-methoxy-4-(4-(4-methylpiperazin-1-yl)piperidin-1-yl)phenyl)-4-(3-phenylisoxazolidin-2-yl)-7*H*-pyrrolo[2,3-*d*]pyrimidin-2-amine,

(88) (*S*)-*N*-(2-methoxy-4-(4-(4-methylpiperazin-1-yl)piperidin-1-yl)phenyl)-4-(3-phenylisoxazolidin-2-yl)-7*H*-pyrrolo[2,3-*d*]pyrimidin-2-amine,

(89) (*S*)-2-(4-(1-(4-((4-(3-phenylisoxazolidin-2-yl)-7*H*-pyrrolo[2,3-*d*]pyrimidin-2-yl)amino)phenyl)piperidin-4-yl)piperazin-1-yl)ethan-1-ol,

(90) (*S*)-2-(4-(1-(2-methoxy-4-((4-(3-phenylisoxazolidin-2-yl)-7*H*-pyrrolo[2,3-*d*]pyrimidin-2-yl)amino)phenyl)piperidin-4-yl)piperazin-1-yl)ethan-1-ol,

(91) *N*-(3-((1*S*,4*S*)-5-methyl-2,5-diazabicyclo[2.2.1]heptan-2-yl)-5-(methylsulfonyl)phenyl)-4-((*S*)-3-phenylisoxazolidin-2-yl)-7*H*-pyrrolo[2,3-*d*]pyrimidin-2-amine,

(92) *N*-(3-((1*R*,4*R*)-5-methyl-2,5-diazabicyclo[2.2.1]heptan-2-yl)-5-(methylsulfonyl)phenyl)-4-((*S*)-3-phenylisoxazolidin-2-yl)-7*H*-pyrrolo[2,3-*d*]pyrimidin-2-amine,

(93) 2-((R)-3-(3-cyclopropyl-5-((4-((S)-3-phenylisoxazolidin-2-yl)-7*H*-pyrrolo[2,3-*d*]pyrimidin-2-yl)amino)phenoxy)pyrrolidin-1-yl)ethan-1-ol,

(94) (S)-(4-(2-hydroxyethyl)piperazin-1-yl)(3-methoxy-5-((4-(3-phenylisoxazolidin-2-yl)-7*H*-pyrrolo[2,3-*d*]pyrimidin-2-yl)amino)phenyl)methanone,

(95) (*S*)-*N*-(1-methylpiperidin-4-yl)-4-((4-(3-phenylisoxazolidin-2-yl)-7*H*-pyrrolo[2,3-*d*]pyrimidin-2-yl)amino)benzamide,

(96) (S)-(3-methoxy-4-((4-(3-phenylisoxazolidin-2-yl)-7*H*-pyrrolo[2,3-d]pyrimidin-2-yl)amino)phenyl)(morpholino)methanone,

(97) (S)-(3-methoxy-4-((4-(3-phenylisoxazolidin-2-yl)-7*H*-pyrrolo[2,3-d]pyrimidin-2-yl)amino)phenyl)(4-morpholinopiperidin-1-yl)methanone,

(98) (*S*)-(3-methoxy-4-((4-(3-phenylisoxazolidin-2-yl)-7*H*-pyrrolo[2,3-*d*]pyrimidin-2-yl)amino)phenyl)(4-(4-methylpiperazin-1-yl)piperidin-1-yl)methanone,

(99) *N*-(3-((S)-3-(dimethylamino)pyrrolidin-1-yl)phenyl)-4-((S)-3-phenylisoxazolidin-2-yl)-7*H*-pyrrolo[2,3-*d*]pyrimidin-2-amine,

(100) *N*-(3-((R)-3-(dimethylamino)pyrrolidin-1-yl)phenyl)-4-((S)-3-phenylisoxazolidin-2-yl)-7*H*-pyrrolo[2,3-*d*]pyrimidin-2-amine,

(101) (*S*)-*N*-(4-(3-(diethylamino)propoxy)-3,5 -dimethylphenyl)-4-(3-phenylisoxazolidin-2-yl)-7*H*-pyrrolo[2,3-*d*]pyrimidin-2-amine,

(102) (*S*)-*N*-(3,5-dimethyl-4-(3-(4-methylpiperazin-1-yl)propoxy)phenyl)-4-(3 - phenylisoxazolidin-2-yl)-7*H*-pyrrolo[2,3-d]pyrimidin-2-amine,

(103) (*S*)-*N,N*-dimethyl-2-(4-((4-(3-phenylisoxazolidin-2-yl)-7*H*-pyrrolo[2,3-*d*]pyrimidin-2-yl)amino)-1*H*-pyrazol-1-yl)acetamide,

(104) (*S*)-*N*-(4-(3-phenylisoxazolidin-2-yl)-7*H*-pyrrolo[2,3-*d*]pyrimidin-2-yl)-1,2,3,4-tetrahydroisoquinolin-7-amine,

(105) (*S*)-2-methyl-*N*-(4-(3-phenylisoxazolidin-2-yl)-7*H*-pyrrolo[2,3-*d*]pyrimidin-2-yl)-1,2,3,4-tetrahydroisoquinolin-7-amine,

(106) (*S*)-6-methoxy-2-methyl-*N*-(4-(3-phenylisoxazolidin-2-yl)-7*H*-pyrrolo[2,3-*d*]pyrimidin-2-yl)-1,2,3,4-tetrahydroisoquinolin-7-amine,

(107) (*S*)-6-methoxy-*N*-(4-(3-phenylisoxazolidin-2-yl)-7*H*-pyrrolo[2,3-*d*]pyrimidin-2-yl)-1,2,3,4-tetrahydroisoquinolin-7-amine,

(108) (*S*)-6-methoxy-7-((4-(3-phenylisoxazolidin-2-yl)-7*H*-pyrrolo[2,3-*d*]pyrimidin-2-yl)amino)-3,4-dihydroisoquinolin-1(2*H*)-one,

(109) (*S*)-2-methyl-*N*-(4-(3-phenylisoxazolidin-2-yl)-7*H*-pyrrolo[2,3-*d*]pyrimidin-2-yl)-1,2,3,4-tetrahydroisoquinolin-6-amine,

(110) (*S*)-*N*-(4-(3-phenylisoxazolidin-2-yl)-7*H*-pyrrolo[2,3-*d*]pyrimidin-2-yl)-1,2,3,4-tetrahydroisoquinolin-6-amine,

(111) (*S*)-2-methyl-1-(6-((4-(3-phenylisoxazolidin-2-yl)-7*H*-pyrrolo[2,3-*d*]pyrimidin-2-yl)amino)-3,4-dihydroisoquinolin-2(1*H*)-yl)propan-2-ol,

(112) (*S*)-1-(6-((4-(3-phenylisoxazolidin-2-yl)-7*H*-pyrrolo[2,3-*d*]pyrimidin-2-yl)amino)-3,4-dihydroisoquinolin-2(1*H*)-yl)ethan-1-one,

(113) (*S*)-6-methoxy-*N*-(4-(3-phenylisoxazolidin-2-yl)-7*H*-pyrrolo[2,3-*d*]pyrimidin-2-yl)-1,2,3,4-tetrahydroisoquinolin-5-amine,

(114) (*S*)-6-methoxy-2-methyl-*N*-(-4-(3-phenylisoxazolidin-2-yl)-7*H*-pyrrolo[2,3-*d*]pyrimidin 2-yl)-1,2,3,4-tetrahydroisoquinolin-5-amine,

(115) (*S*)-3,3-dimethyl-5-((4-(3-phenylisoxazolidin-2-yl)-7*H*-pyrrolo[2,3-*d*]pyrimidin-2-yl)amino)isobenzofuran-1(3*H*)-one,

(116) (*S*)-5-chloro-*N*-(4-(4-methylpiperazin-1-yl)phenyl)-4-(3-phenylisoxazolidin-2-yl)-7*H*-pyrrolo[2,3-*d*]pyrimidin-2-amine,

(117) (*S*)-*N*-(5-chloro-4-(3-phenylisoxazolidin-2-yl)-7*H*-pyrrolo[2,3-*d*]pyrimidin-2-yl)-1,2,3,4-tetrahydroisoquinolin-6-amine,

(118) (*S*)-2-((4-(4-methylpiperazin-1-yl)phenyl)amino)-4-(3-phenylisoxazolidin-2-yl)-7*H*-pyrrolo[2,3-*d*]pyrimidine-5-carbonitrile,

(119) (*S*)-2-((4-(4-(4-methylpiperazin-1-yl)piperidin-1-yl)phenyl)amino)-4-(3 - phenylisoxazolidin-2-yl)-7*H*-pyrrolo[2,3-*d*]pyrimidine-5-carbonitrile,

(120) (*S*)-4-(3-phenylisoxazolidin-2-yl)-2-((1,2,3,4-tetrahydroisoquinolin-6-yl)amino)-7*H*-pyrrolo[2,3-*d*]pyrimidine-5-carbonitrile,

(121) (*S*)-2-((2-methoxy-4-(4-(4-methylpiperazin-1-yl)piperidin-1-yl)phenyl)amino)-4-(3 - phenylisoxazolidin-2-yl)-7*H*-pyrrolo[2,3-*d*]pyrimidine-5-carbonitrile,

(122) (*S*)-2-((2-cyclopropyl-6-methoxy-1,2,3,4-tetrahydroisoquinolin-7-yl)amino)-4-(3-phenylisoxazolidin-2-yl)-7H-pyrrolo[2,3-*d*]pyrimidine-5-carbonitrile,

(123) (*S*)-*N*-(4-(4-methylpiperazin-1-yl)phenyl)-4-(3-phenylisoxazolidin-2-yl)-5-(trifluoromethyl)-7*H*-pyrrolo[2,3-*d*]pyrimidin-2-amine,

(124) (*S*)-*N*-(4-(3-phenylisoxazolidin-2-yl)-5-(trifluoromethyl)-7*H*-pyrrolo[2,3-*d*]pyrimidin-2-yl)-1,2,3,4-tetrahydroisoquinolin-6-amine,

(125) *N*-(3-((R)-3-(dimethylamino)pyrrolidin-1-yl)phenyl)-4-((R)-3-phenylisoxazolidin-2-yl)-7*H*-pyrrolo[2,3-*d*]pyrimidin-2-amine,

(126) *N*-(3-((S)-3-(dimethylamino)pyrrolidin-1-yl)phenyl)-4-((R)-3-phenylisoxazolidin-2-yl)-7*H*-pyrrolo[2,3-*d*]pyrimidin-2-amine,

(127) (R)-(4-(2-hydroxyethyl)piperazin-1-yl)(3 -methoxy-5-((4-(3-phenylisoxazolidin-2-yl)-7*H*-pyrrolo[2,3-*d*]pyrimidin-2-yl)amino)phenyl)methanone,

(128) (R)-*N*-(4-(4-methylpiperazin-1-yl)phenyl)-4-(3-phenylisoxazolidin-2-yl)-7*H*-pyrrolo[2,3-*d*]pyrimidin-2-amine, and

(129) (R)-*N*-(3-methoxy-4-(4-(4-methylpiperazin-1-yl)piperidin-1 -yl)phenyl)-4-(3 - phenylisoxazolidin-2-yl)-7*H*-pyrrolo[2,3-*d*]pyrimidin-2-amine.

**12.** A method for preparing the compound represented by Formula 1, or an isomer thereof, a solvate thereof, a hydrate thereof or a pharmaceutical thereof, comprising:

a step of reacting a compound represented by Formula 2 below with a compound represented by Formula 3 below to prepare a compound represented by Formula 4 below (STEP 1); and
a step of reacting the compound represented by Formula 4 obtained from the previous step with a compound represented by Formula 5 below (STEP 2):

[Scheme A]

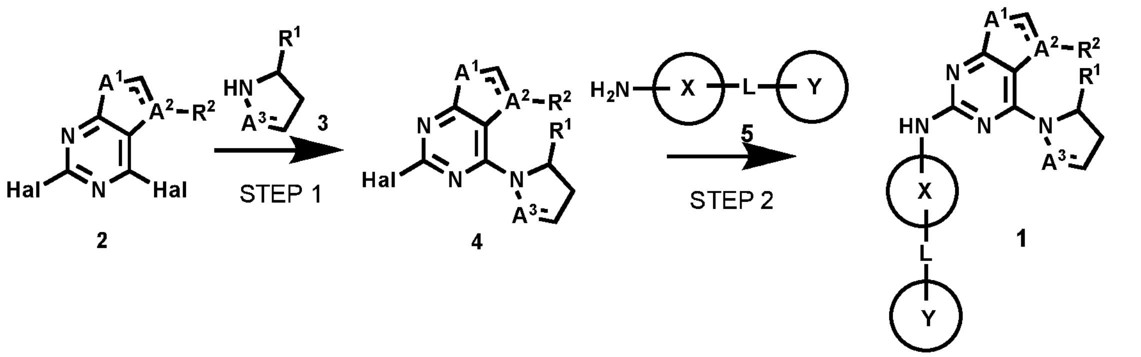


wherein,

Hal is halogen;
$A^1$ is CH or NH;
$A^2$ is CH or S;
$A^3$ is N or O;

------ is a single bond or a double bond, which is determined according to the valence of atoms located at both ends thereof;
$R^1$ is phenyl, thiophenyl, pyridinyl, $(C_{3-7})$cycloalkyl, naphthalenyl, or isoquinolinyl, where the phenyl may be substituted with $(C_{1-3})$alkoxy or halogen;
$R^2$ is absent, hydrogen, haloalkyl, cyano, or halogen;
ring X is benzene, pyridine, pyrazole, isoindoline, tetrahydroisoquinoline, dihydroisoquinoline, or isobenzofuran, where the benzene, pyridine, pyrazole, isoindoline, tetrahydroisoquinoline, dihydroisoquinoline, or isobenzofuran may be substituted with one or more non-hydrogen substituents selected from the group consisting of $(C_{1-5})$alkyl, $(C_{1-5})$alkoxy, (di$(C_{1-3})$alkylamino$(C_{1-5})$alkyl)$(C_{1-3})$alkylamino, di$(C_{1-3})$alkylamino$(C_{1-5})$alkyl, (di$(C_{1-3})$alkylamino)$(C_{1-5})$alkoxy, di$(C_{1-3})$alkylaminocarbonyl$(C_{1-3})$alkyl, $(C_{3-7})$cycloalkyl, oxo('=O'), $(C_{1-3})$alkylsulfonyl, haloalkyl, $(C_{1-3})$alkylcarbonyl, hydroxy$(C_{1-5})$alkyl, and halogen;
L and ring Y are absent, or
when L and ring Y are present, L is a direct bond, -$(C_{1-5})$alkylene-, -O-, -(C=O)-, -O-$(C_{1-5})$alkylene-, -(C=O)-NH-, or piperidine, where ring Y is a heterocyclic group selected from the group consisting of piperazine, pyrrolidine, morpholino, piperidine, imidazole, diazabicyclo[2,2,1]heptane, and pyrazole, where the heterocyclic group may be substituted with one or more non-hydrogen substituents selected from the group consisting of $(C_{1-5})$alkyl, di$(C_{1-3})$alkylamino, hydroxy$(C_{0-5})$alkyl, and $(C_{3-7})$cycloalkyl;
the case where both of L and ring Y are piperidine is excluded; and
the case where both of $A^1$ and $A^2$ are CH or are not CH is excluded.

**13.** A pharmaceutical composition for preventing or treating: hematologic malignancies including acute myeloid leukemia, myelodysplastic syndrome, acute lymphocytic leukemia, and chronic myeloid leukemia; cancer diseases including bladder cancer, brain cancer, breast cancer, cervical cancer, colon cancer, rectal cancer, colorectal cancer, stomach cancer, esophageal cancer, endometrial cancer, liver cancer, laryngeal cancer, lung cancer, osteosarcoma, ovarian cancer, pancreatic cancer, prostate cancer, kidney cancer, thyroid cancer, neuroblastoma, rhabdomyosarcoma, nephroblastoma, glioblastoma, medulloblastoma and Ewing's sarcoma; autoimmune diseases including multiple sclerosis, psoriasis, inflammatory small intestine disease, ulcerative colitis, Crohn's disease, rheumatoid arthritis and polyarthritis, local and systemic scleroderma, systemic lupus erythematosus, discoid lupus erythematosus, cutaneous lupus, dermatomyositis, polymyositis, Sjogren's syndrome, nodular panarteritis, autoimmune enteritis, atopic dermatitis and proliferative glomerulonephritis; allergic diseases including asthma, allergic rhinitis, allergic rhinosinusitis, anaphylaxis, urticaria, angioedema, atopic dermatitis, allergic contact dermatitis, erythema nodosum, erythema multiforme, cortical phlebitis and insect bite dermatitis or blood-sucking parasite infection; or neurological diseases including Huntington's disease, schizophrenia, Parkinson's disease, and Alzheimer's disease, comprising, as an active ingredient, the compound of any one of claims 1 to 11, or a stereoisomer thereof, a hydrate thereof or a pharmaceutically acceptable salt thereof.

**14.** The pharmaceutical composition according to claim 13, wherein the pharmaceutical composition inhibits activities of one or more protein kinases of FLT3, CDK4, CDK6, and HPK1.

## INTERNATIONAL SEARCH REPORT

International application No.
PCT/KR2020/006496

**A. CLASSIFICATION OF SUBJECT MATTER**

*C07D 495/04(2006.01)i, C07D 487/04(2006.01)i, A61K 31/519(2006.01)i, A61P 35/00(2006.01)i, A61P 35/02(2006.01)i*

According to International Patent Classification (IPC) or to both national classification and IPC

**B. FIELDS SEARCHED**

Minimum documentation searched (classification system followed by classification symbols)
C07D 495/04; A61K 31/417; A61K 31/4184; A61K 31/519; C07D 239/48; C07D 401/04; C07D 401/14; C07D 487/04; C07D 491/107; A61P 35/00; A61P 35/02

Documentation searched other than minimum documentation to the extent that such documents are included in the fields searched
Korean utility models and applications for utility models: IPC as above
Japanese utility models and applications for utility models: IPC as above

Electronic data base consulted during the international search (name of data base and, where practicable, search terms used)
eKOMPASS (KIPO internal), STN (Registry, Caplus) & Keywords: cancer, autoimmune, fused pyrimidine

**C. DOCUMENTS CONSIDERED TO BE RELEVANT**

| Category* | Citation of document, with indication, where appropriate, of the relevant passages | Relevant to claim No. |
|---|---|---|
| X | WO 2009-131687 A2 (PORTOLA PHARMACEUTICALS, INC.) 29 October 2009<br>See claims 1, 89-92, 100-101; pages 111-112. | 1-14 |
| X | KR 10-2010-0015353 A (PALAU PHARMA, S.A.) 12 February 2010<br>See claims 1, 37-40. | 1-14 |
| A | US 2017-0355708 A1 (CADENT THERAPEUTICS, INC.) 14 December 2017<br>See the entire document. | 1-14 |
| A | WO 2018-232094 A1 (BIOCRYST PHARMACEUTICALS, INC.) 20 December 2018<br>See the entire document. | 1-14 |
| A | KR 10-2019-0003242 A (ONCOBIX CO., LTD. et al.) 09 January 2019<br>See the entire document. | 1-14 |

☐ Further documents are listed in the continuation of Box C.  ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 14 AUGUST 2020 (14.08.2020) | **14 AUGUST 2020 (14.08.2020)** |

| Name and mailing address of the ISA/KR | Authorized officer |
|---|---|
| Korean Intellectual Property Office<br>Government Complex Daejeon Building 4, 189, Cheongsa-ro, Seo-gu, Daejeon, 35208, Republic of Korea<br>Facsimile No. +82-42-481-8578 | Telephone No. |

Form PCT/ISA/210 (second sheet) (January 2015)

**INTERNATIONAL SEARCH REPORT**
Information on patent family members

International application No.
**PCT/KR2020/006496**

| Patent document cited in search report | Publication date | Patent family member | Publication date |
|---|---|---|---|
| WO 2009-131687 A2 | 29/10/2009 | AU 2009-238590 A1 | 29/10/2009 |
| | | BR PI0910668 A2 | 24/09/2019 |
| | | CA 2723185 A1 | 29/10/2009 |
| | | CN 102066338 A | 18/05/2011 |
| | | CN 103224497 A | 31/07/2013 |
| | | EP 2271631 A2 | 12/01/2011 |
| | | EP 2271631 B1 | 04/07/2018 |
| | | IL 208719 A | 30/12/2010 |
| | | JP 2011-518219 A | 23/06/2011 |
| | | NZ 588830 A | 30/11/2012 |
| | | US 2009-0298823 A1 | 03/12/2009 |
| | | US 2013-0029944 A1 | 31/01/2013 |
| | | US 8258144 B2 | 04/09/2012 |
| | | US 9139581 B2 | 22/09/2015 |
| | | WO 2009-131687 A3 | 07/01/2010 |
| KR 10-2010-0015353 A | 12/02/2010 | AR 065901 A1 | 08/07/2009 |
| | | AU 2008-234822 A1 | 09/10/2008 |
| | | BR PI0809992 A2 | 14/10/2014 |
| | | CA 2682646 A1 | 09/10/2008 |
| | | CL 2008000946 A1 | 10/10/2008 |
| | | CN 101679440 A | 24/03/2010 |
| | | EP 2142550 A1 | 13/01/2010 |
| | | IL 201073 A | 17/05/2010 |
| | | JP 2010-523522 A | 15/07/2010 |
| | | MX 2009010595 A | 22/10/2009 |
| | | RU 2009140319 A | 10/05/2011 |
| | | TW 200904442 A | 01/02/2009 |
| | | US 2010-0113420 A1 | 06/05/2010 |
| | | US 2011-0160185 A9 | 30/06/2011 |
| | | WO 2008-119792 A1 | 09/10/2008 |
| US 2017-0355708 A1 | 14/12/2017 | None | |
| WO 2018-232094 A1 | 20/12/2018 | AR 112027 A1 | 11/09/2019 |
| | | AU 2018-283053 A1 | 16/01/2020 |
| | | CA 3066164 A1 | 20/12/2018 |
| | | CL 2019003669 A1 | 22/05/2020 |
| | | CN 110913855 A | 24/03/2020 |
| | | CO 2020000194 A2 | 24/04/2020 |
| | | EA 202090052 A1 | 22/05/2020 |
| | | EP 3638229 A1 | 22/04/2020 |
| | | KR 10-2020-0028939 A | 17/03/2020 |
| | | TW 201904958 A | 01/02/2019 |
| KR 10-2019-0003242 A | 09/01/2019 | None | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **HANNUM et al.** *Nature,* 1994, vol. 368, 643-648
- **ROSNET et al.** *Genomics,* 1991, vol. 9, 380-385
- **NAKAO et al.** *Leukemia,* 1996, vol. 12, 1911-1918
- **BARTOLO et al.** *JEM,* 2007, vol. 204, 681-691
- **LASSERRE et al.** *J Cell Biol,* 2011, vol. 195 (5), 839-853
- **SHUI, JW et al.** *Nat Immunol,* 2007, vol. 8 (1), 84-91
- **WANG, X. et al.** *J Biol Chem,* 2012, vol. 287 (14), 11037-48